# EUROPEAN PATENT SPECIFICATION

(11) **EP 0 585 371 B1**
(45) Date of publication and mention of the grant of the patent: **17.04.2002**
(21) Application number: 92912715.7
(22) Date of filing: 20.05.1992
(51) Int. Cl.: A61K 31/775, A61K 31/765, A61K 31/19, A61K 31/225, A61K 31/74

(54) **AROMATIC OLIGOMERIC COMPOUNDS USEFUL AS MIMICS OF BIOACTIVE MACROMOLECULES**
AROMATISCHE OLIGOMERE VERBINDUNGEN ALS IMITATOREN BIOAKTIVES MAKROMOLEKÜLE
COMPOSES OLIGOMERES AROMATIQUES UTILISES COMME IMITATEURS DE MACROMOLECULES BIOACTIVES

(30) Priority: 20.05.1991 US 703061
(43) Date of publication of application: 09.03.1994
(73) Proprietor: RHONE-POULENC RORER INTERNATIONAL (HOLDINGS) INC., Greenville, Delaware 19807 (US)
(72) Inventor: REGAN, John, R., Princeton, NJ 08540 (US); MCGARRY, Daniel, G., King of Prussia, PA 19406 (US); CHANG, Michael, N., Newtown, PA 18940 (US); BARTON, Jeffrey, N., Philadelphia, PA 19100 (US); NEWMAN, Jack, Warrington, PA 18976 (US); BEN-SASSON, Schmuel, Jerusalem (IL)
(74) Representative: Le Pennec, Magali
(86) International application number: US9204274
(87) International publication number: WO9220350

(56) References cited:
- WO-A-91/03226
- WO-A-91/07183
- WO-A-92/13542
- US-A- 2 323 481
- US-A- 2 910 484
- US-A- 4 604 404
- HELVETICA CHIMICA ACTA vol. 64, no. 59 , 1981 pages 599 - 609 G.H. HAKIMELAHI ET AL. 'THE SYNTHESIS OF POLYFUNCTIONAL AROMATIC RING SYSTEMS.'
- RENDICONTO DELL'ACCADEMIA DELLE SCIENZE FISICHE E MATEMATICHE vol. 36 , 1969 pages 108 - 125 G. CIAMPA ET AL. 'POLIMERI DI INTERESSE FARMACOLOGICO INDAGINE MEDIANTE TITRIMETRIA NON ACQUOSA'
- Tetrahedron 46(10), 3613-20, 1990, A. Arduini et al.

## Description

### BACKGROUND OF THE INVENTION

### Field of the Invention

The present invention relates to: (A) methods of treatment involving the use of pharmaceutical compositions containing aromatic oligomers which mimic the action of bioactive naturally ocurring polymers including glycosaminoglycans, peptides and polynucleic acids; (B) pharmaceutical compositions containing such oligomers; and (C) the oligomers for use in such compositions and treatments.

Glycosaminoglycans (GAG) are linear polysaccharides formed by characteristic repeating disaccharide units usually composed of a uronic acid and a hexosamine. The term "acid mucopolysaccharides" was used originally to designate hexosamine-rich acid polysaccharides extracted from connective tissue. In recent years, the term "glycosaminoglycans" has gained greater acceptance and is now used in place of mucopolysaccharides. The hexosamine can be glucosamine or galactosamine, and the uronic acid can be glucuronic or iduronic acid. Sulphate groups are found on all glycosaminoglycans apart from hyaluronic acid, and all of the sulphated glycosaminoglycans are covalently linked to protein forming different classes of proteoglycans. However, it would be an oversimplification to consider glycosaminoglycans to be simple repeat-unit polysaccharides, since considerable chemical and configurational variability can be superimposed upon the component sugars.

Among other functions it has been shown that the glycosaminoglycans serve as a support which binds various bioactive peptides. This association is based on a non-covalent interaction since the bound protein can be readily released upon the addition of free glycosaminoglycans. Well known examples of such bound proteins include enzymes such as lipoprotein lipase (LPL) or growth-regulating peptides such as fibroblast growth factor (FGF). Another example of GAG-protein interaction is that of the enzyme heparanase which participates in cell-invasion processes. It has been demonstrated also that the commercially available glycosaminoglycan, heparin, inhibits the growth of vascular smooth muscle cells, which is involved in arteriosclerosis and the proliferation of kidney mesangial cells which plays a role in glomerulosclerosis. Heparin is known also to be involved in the release of lipoprotein lipase, the inhibition of heparanase and the release of fibroblast growth factor. The most common application of heparin is as an anticoagulant where heparin interacts with proteins which play a key role in hemostasis.

Glycosaminoglycans such as heparin are a major constituent participating in the composition of various biological structures such as basement membranes, connective tissues, cartilage and cell-surface glycocalyx. Connective tissues are responsible for providing and maintaining form in the body. Functioning in a mechanical role, they provide a matrix that serves to connect and bind the cells and organs and ultimately give support to the body. Unlike the other tissue types (epithelium, muscle and nerve) formed mainly by cells, the major constituent of connective tissue is its extracellular matrix, composed of protein fibers, an amorphous ground substance, and tissue fluid, the latter consisting primarily of bound water of solvation. Embedded within the extracellular matrix are the connective tissue cells.

In terms of structural composition, connective tissue can be subdivided into three classes of components: cells, fibers and ground substance. The wide variety of connective tissue types in the body represents modulations in the degree of expression of these three components.

The amorphous intercellular ground substance fills the space between cells and fibers of the connective tissue; it is viscous and acts as a lubricant and also as a barrier to the penetration of the tissues by foreign particles. Glycosaminoglycans and structural glycoproteins are the two principal classes of components comprising the ground substance.

Various disease states are characterized by the pathological hydrolysis of structural glycoproteins such as collagen, fibronectin and elastin. This hydrolysis can be mediated by the enzyme, elastase, which is possibly the most destructive enzyme in the body. The elastase produced by human neutrophil leukocytes (otherwise known as PMN or HNE or HLE) is believed to be involved in various diseases characterized by the destruction of structural proteins comprising the ground substance, including pulmonary emphysema, chronic bronchitis, cystic fibrosis, bronchiectasis, adult respiratory distress syndrome, atherosclerosis, arthritis, psoriasis, vasculitis, glomerulonephritis and consumption coagulopathies associated with gram-negative sepsis or leukemias.

Cell proliferation and directed cell movement are key events in several normal processes including embryogenesis and development, responses to injury such as wound repair, and in those tissues that maintain themselves by continual or intermittent cell turnover, such as the epithelia that line all body surfaces and cavities, and the cells of the hematopoietic system. Such proliferation is related to the response of cells to various growth factors which may act singly or in concert to stimulate the proliferation of cells. The most ubiquitous of these growth factors is platelet derived growth factor (PGDF).

Abnormal smooth muscle cell proliferation is associated with a number of conditions including atherosclerosis, lung fibrosis, arthritis, and arterial restenosis, for example, following angioplasty or other invasive procedures which distend or injure the walls of arteries. Angioplasty is a surgical procedure wherein an artery, for example a coronary, femoral, or carotid artery, which is stenosed, i.e., blocked or constricted, by atherosclerotic plaque is dilated by the passage of an inflatable catheter through the vessel to the area of disease where inflation of the catheter compresses the plaque against the vessel wall, thereby relieving the constriction. While this procedure may be successful intially, 30-60% of patients undergoing angioplasty experience a restenosis of the artery within six months to one year. This restenosis is due predominantly to the increased proliferation of smooth muscle cells at the site of injury.

The present invention is based on the discovery of a class of compounds exhibiting properties which mimic the action of glycosaminoglycans and which are capable of modulating biological systems containing complexes between bioactive peptides and/or proteins and glycosaminoglycans by competing with the binding interactions of glycosaminoglycans.

### Reported Developments

Heparin has been used for decades as an anticoagulant in situations where intravenous administration thereof can be monitored and controlled. More recently, purified fractions of heparin have been used as a more effective substitute. Nevertheless, both complete or fractionated heparin require intravenous administration.

PCT International Publication Number WO 91/03226, published March 21, 1991, and PCT International Publication Number WO 91/07183, published May 30, 1991, hereby incorporated by reference, disclose pharmaceutical compositions comprising, in admixture with a pharmaceutically acceptable carrier, therapeutically effective amounts of aromatic ring-containing polymeric compounds, substantially free of monomer, and having properties which mimic the pharmacological activity of glycosaminoglycans and which are capable of competing with the binding thereof to bioactive peptides and/or proteins. Examples of such polymeric compounds include those having a molecular weight of about 2,000 to about 20,000 Daltons and in which each monomeric unit of the polyaromatic compound includes from 1 to about 10 aromatic rings, which may be substituted by electronegative substituents and/or negatively charged residues.

International Publication Number WO 91/03226 discloses the preferred compounds for use in the pharmaceutical compositions are those in which each monomeric unit contains between 3 and 10 aromatic rings, and particularly those in which the aromatic rings contain at least one substituent on at least two of the rings. The preferred molecular weight of these polymeric compounds are described as being about 2000 to about 20,000, the most preferred molecular weight being about 2000 to about 4000 Daltons, as measured by gel permeation chromatography. Aromatic polymers described in WO 91/03226 exhibit anticoagulant properties, are capable of being administered orally and are capable of being absorbed into the bloodstream from the gastrointestinal tract. Another aspect of the invention described and claimed in International Publication Number WO 91/03226 includes use of the aforementioned pharmaceutical compositions to treat humans or other animals for cardiovascular disorders, metabolic disorders of bone tissue, and neuronal disorders.

International Publication Number WO 91/07183 discloses a biologically active polymeric compound having an alkylaryl backbone, including particularly those polymeric compounds which have about 5 to about 50 repeating monomeric or dimeric aromatic ring-containing units. More specifically, there is disclosed a biologically active polymeric compound having an alkylaryl backbone and from about 5 to about 50 repeating monomeric or dimeric aromatic ring-containing units and which, according to the computer program marketed as SYBYL version 5.2 running on a DEC VAX 11/750 computer, is capable of forming a linear backbone having a helical secondary structure, and wherein the maximum diameter of the helical structure, as measured by the alkylaryl backbone, is less than 3 times greater than the maximum diameter of the aryl group of the alkylaryl backbone. In preferred form, the polymeric compound is substantially linear and includes polymeric compounds in which the alkylaryl group is polysubstituted.

International Publication Number WO 91/07183 discloses a class of polymeric compounds including as a repeating unit in the polymeric chain a single mononuclear aromatic ring or a single polynuclear aromatic ring, which is prepared by polymerizing a monomer comprising a mononuclear aromatic ring, for example, phenylenes such as hydroxybenzoic acid, or by polymerizing a monomer comprising a polynuclear aromatic ring, for example, naphthalenes such as hydroxynaphthoic acid. Another class of polymeric compounds of the aforementioned type, that is, those which have the computer-predicted helical secondary structure as referred to above, comprise polymeric compounds which include as a repeating unit in the polymeric chain two substituted aromatic ring-containing units, each of the aromatic ring-containing units being substituted with the same group(s) and being bonded together by an alkyl bridge. Preferably, the positions of the corresponding substituents of each ring have the same orientation (for example, ortho-, meta- or para-) with regard to the position of the alkyl bridge. The most preferred compounds are those which comprise as the repeating unit in the polymeric chain two identically-substituted phenylene groups and wherein the alkyl bridging groups are attached to each phenylene in an orientation meta- to each other. Such polymeric compounds can be prepared by forming first the dimer of the monomeric form of the compound comprising the aromatic ring and then polymerizing the dimer.

Although considerable efforts have been directed to the isolation, from the aforesaid polymeric reaction mixtures, of a narrow band molecular weight fraction of the resulting polymeric material, the fractionated materials obtained nevertheless comprise mixtures of more than one polymeric species differing primarily in the number of monomeric units making up each polymeric component of the fractionated mixture. The mixture nature of the polymeric product is of concern in connection with various governmental health authority requirements regulating the approval for commercial use of pharmaceutical products. Such requirements mandate the precise reproducibility of the process used to produce the drug submitted for approval. Since the mixture of bioactive aromatic polymers discussed hereinabove is produced from a polymerization reaction, the reproducibility of the product obtained from the polymeric mixture and subsequent fractionation, in terms of all chemical physical and biological properties, would be expected to come under intense governmental scrutiny. Consequently, continuing work has focused on the precise manufacture of the bioactive aromatic polymer, and, in particular, has focused on a process for the preparation of oligomeric compounds, that is compounds having a precisely defined molecular weight and monomer number content yet retaining the chemical, physical and biological characteristics of the polymeric materials described hereinabove.

The literature discloses oligomeric compounds characterized by extended π chains, such as disclosed in Schenck, et al., *J. Am. Chem. Soc.* **113** (7), 2634-2647 (1991), ollgo(phenylene-vinylenes), and oligo(hydroxyphenylmethylenes) utilized as intermediates for the preparation of calixarenes, which are cyclic oligomers comprising [1ₙ] metacyclophanes made up of para-substituted phenolic units and methylene groups such as disclosed, for example, in Gutsche, *Top. Curr. Chem.* **123**, 1-47 (1984); Bohmer, et al., *J. Org. Chem.* **52** (15) 3200-3205 (1987); Royer, et al., *Tetrahedron Lett.* **28** (52), 6595-6596 (1987). The aforesaid Gutsche reference discloses that certain of the calixarenes as well as linear tetramers made up of p-halophenol units exhibit activity against various pathogenic organisms. Similarly, smaller oligomer compounds, such as the trimer, 3,5-bis-(2,4-dihydroxy-6-methoxy-5-methyl-3-propanoylbenzyl)-2,4,6-trihydroxybutyrophenone (i.e. trisaspidinol), synthesized as an analogue of terramycin, have been reported to possess antibacterial activity, Hakimelahi, et al., *Helv. Chim. Acta* Vol. 64, Fasc. 2, 599-609 (1981).

### SUMMARY OF THE INVENTION

As is described in more detail below the present invention relates to pharmaceutical compositions comprising, in admixture with a pharmaceutically acceptable carrier, aromatic oligomeric compounds, comprising a single molecular species having an ascertainable molecular weight and between 4 to about 50 linked structural units, and capable of mimicking the action of bioactive naturally ocurring polymers including glycosaminoglycans, peptides and polynucleic acids.

The present invention relates specifically to pharmaceutical compositions comprising, in admixture with a pharmaceutically acceptable carrier, an aromatic oligomeric compound comprising a single polymeric species of the formula I

Mᵢ-(Mₙ)ₙ-Mₜ I

wherein
n represents a sequence of whole numbers beginning at 2 and having an upper limit of 50;
Mᵢ is 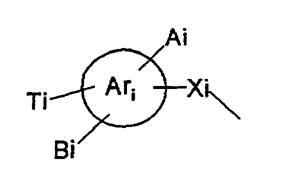
Mₙ is 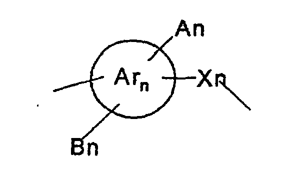
Mₜ is 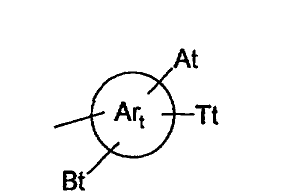 where 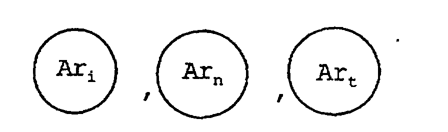 are independently benzene or naphthalene,
   where 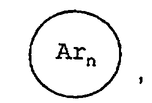 Aₙ, Bₙ, and Xₙ may be the same or different for each of the (Mₙ) groups in the n sequence; and
Aⱼ, Bⱼ, Aₙ, Bₙ, Aₜ, Bₜ, Tⱼ and Tₜ are independently hydrogen or a substituent group;
Xⱼ and Xₙ are independently a bond, alkylene unsaturated alkylene, or heteroatom-containing linking group;
or a pharmaceutically acceptable salt thereof.

A further aspect of the present invention relates to oligomeric compounds having at least one substituent group or pro-substituent group capable of participating in electrostatic interactions with bioactive molecules at biological pH according to Formula 1 above, and wherein n represents a sequence of whole numbers beginning at 3 and terminating at less than or equal to 50.

Still another aspect of the present invention relates to pharmacological methods comprising the administration of an effective amount of said pharmaceutical composition to a human or other animal patient in need of cardiovascular therapy such as anticoagulant and/or antithrombotic therapy and/or bone metabolic therapy and/or antihypolipaemia therapy and/or therapy for the treatment of neuronal disorders and/or gastrointestinal disorders and/or disorders which may be treated by agents effective in binding DNA.

A further aspect of the present invention is based on the surprising and unexpected discovery that a preferred class of oligomers according to the present invention are highly selective and potent inhibitors of the enzyme, elatase, which is produced by human polymorphonuclear neutrophils (PMN). Accordingly, another aspect of the present invention relates to the use of a pharmaceutical composition comprising an effective elastase-inhibiting amount of said oligomer for the preparation of a medicament able to be administered to a human or other animal patient suffering from an elastase-mediated connective tissue degradation disorder.

It has also been found that a further class of oligomers according to the present invention inhibit PDGF stimulated smooth muscle cell proliferation. Accordingly, another aspect of the present invention relates to pharmacological methods comprising the administration of a pharmaceutical composition comprising an effective smooth muscle cell proliferation inhibiting amount of a said oligomer to a human or other animal patient in need of inhibiting abnormal smooth muscle cell proliferation, underlying disorders including arterial restenosis, atherosclerosis, lung fibrosis, and arthritis.

Advantages which flow from the practice of the present invention include extended duration of bioactivity in-vivo, as compared to naturally occurring compounds such as heparin, and the availability of oral administration. Heparin, for example, cannot be administered orally, as it is degraded in the digestive system before being absorbed into the bloodstream. In practice, however, it is anticipated that for some therapies the preferred mode of administration of the compositions and compounds of the present invention will be parenteral, for example, intravenous injection.

### DETAILED DESCRIPTION OF THE INVENTION

As used above, and throughout the description of this invention, the following terms, unless otherwise indicated, shall be understood to have the following meanings:
"Aromatic carbocyclic ring" means an aromatic hydrocarbon ring system. Preferred aromatic carbocyclic rings include benzene and naphthalene.
"Aromatic heterocyclic ring" means about a 5- to about a 15- membered monocyclic or multicyclic aromatic ring system in which one or more of the atoms in the ring or rings is an element other than carbon, for example nitrogen, oxygen or sulfur. Preferred aromatic heterocyclic rings include pyridine, quinoline, and isoquinoline.
"Alkyl" means a saturated aliphatic hydrocarbon group which may be straight or branched and having about 1 to about 20 carbon atoms in the chain. Branched means that a lower alkyl group such as methyl, ethyl or propyl is attached to a linear alkyl chain. Preferred alkyl groups are the "lower alkyl" groups which are those alkyl groups having from 1 to about 6 carbons.
"Aryl" means phenyl or naphthyl, or phenyl or naphthyl substituted with one or more aryl group substituents which may be the same or different, where "aryl group substituent" includes alkyl, alkenyl, alkynyl, aryl, aralkyl, hydroxy, alkoxy, aryloxy, aralkoxy, hydroxyalkyl, acyl, formyl, carboxy, alkenoyl, aroyl, halo, nitro, trihalomethyl, cyano, alkoxycarbonyl, aryloxycarbonyl, aralkoxycarbonyl, amino, alkylamino, and dialkylamino.
"Aralkyl" means an alkyl group substituted by an aryl radical. Exemplary aralkyl groups include benzyl and phenethyl.
"Halogen (halo)" means fluorine (fluoro), chlorine (chloro), bromine (bromo) or iodine (iodo).
"Alkenyl" means an alkyl group containing a carbon-carbon double bond. Exemplary groups include allyl and vinyl.
"Alkoxy" means an alkyl-O- group. Lower alkoxy groups are preferred. Exemplary groups include methoxy, ethoxy, n-propoxy, i-propoxy and n-butoxy.
"Aralkoxy" means an aralkyl-O- group. Exemplary groups include benzyloxy and phenethyloxy.
"Aryloxy" means an aryl-O- group. Exemplary groups include phenoxy and 2-naphthyloxy.
"Acyl" means an 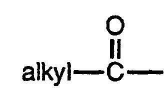 group. Preferred acyl groups are those in which the alkyl group is lower alkyl.
"Alkoxycarbonyl" means an 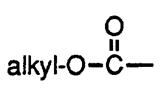 group. Preferred groups include methoxycarbonyl and ethoxycarbonyl.
"Aralkoxycarbonyl" means an 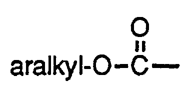 group. A preferred group is benzyloxycarbonyl.
"Aryloxycarbonyl" means an 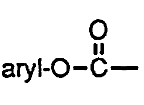 group. A preferred group is phenoxycarbonyl.
"Biological pH" refers to that pH of blood, plasma or serum in the body between about 7.2 and about 7.5 and which does not interfere with normal degradation of materials present therein. The normal pH of blood, plasma or serum values is about 7.35-7.45 and is preferably about pH 7.39-7.41.

The oligomers of the present invention are described by Formula I wherein the number of individual units making up the oligomer are provided for by the use of sequence notation. More particularly, the formula is dependent on the variable "n" which is defined in the Summary of Invention section hereinabove as a sequence of whole numbers beginning at 2 and having an upper limit of about 50. For n=2, the oligomer according to Formula I is a tetramer of the formula Mᵢ-M₁-M₂-Mₜ; for n=4, the oligomer is a hexamer of the formula Mᵢ-M₁-M₂-M₃-M₄-Mₜ; and for n=6, the oligomer is an octomer of the formula Mᵢ-M₁-M₂-M₃-M₄-M₅-M₆-Mₜ. The variable "n" serves to identify not only the individual oligomeric units in the sequence of units making up the oligomer, but also serves to differentiate the various Arₙ units with their associated substituent and linking groups, Aₙ, Bₙ, and Xₙ. For example, M₄ is equivalent to the group Ar₄ with substituent groups, A₄ and B₄, and linking group X₄.

This means of defining the oligomers according to the present invention highlights the variability in oligomer sequence order and chemical makeup that is enabled by the oligomer preparation processes disclosed herein. By virtue of the processes disclosed herein, the preparation of oligomers characterized by specific molecular weight, differing oligomeric unit content and specific oligomeric unit sequence, specifically chosen and located linking groups is possible. This ability to design a discrete oligomeric molecule of specific chain length and characterized by a selected functionalization and/or elimination of substituent groups on specific oligomeric units enables the fine tuning and focusing of the potency, pharmacological specificity and oral bioavailability of the oligomers of the present invention. Consequently, a further aspect of this invention relates to processes for the preparation of the oligomers of the present invention. The particular pharmacological properties and the assays used to determine the pharmacological profiles of the present oligomers are discussed in more detail below.

The pharmaceutical compositions according to the invention comprise oligomeric compounds according to Formula I, wherein the substituent and linking groups are defined as follows:
Aᵢ, Bᵢ, Aₙ, Bₙ, Aₜ and Bₜ are independently hydrogen, (C₁-C₂₀)alkyl, benzyl, phenethyl, cyano, (C₁-C₄)alkoxy (C₁-C₄)alkoxy, or -(CH₂)ₐ-W-(CH₂)_{b}-Rₛ, where Rₛ is NRR', COOR, CONRR', NR(COR'), PO(OR)₂, COR, SO₂OR, OSO₂OR, halogen, OR, SO₂R, SOR, SR or CHO, and where a and b are independently 0 to 4, (a+b) is less than 5, W is -O-, -S- , -SO-, -SO₂-, -NR(COR')-, -NR-, or a bond, and R and R' are independently hydrogen, (C₁-C₂₀)alkyl, benzyl, or phenethyl,
Tᵢ and Tₜ are independently hydrogen, (C₁-C₂₀)alkyl, allyl, vinyl, halo, cyano, (C₁-C₄)alkoxy(C₁-C₄)alkoxy, halo(C₁-C₂₀)alkyl, hydroxy, *tert*-butyldimethylsilyloxy(C₁-C₂₀)alkyl, hydroxy (C₁-C₂₀)alkyl, (C₁-C₄)alkoxy, benzyloxy, phenethyloxy, phenoxy, 2-napthyloxy, (C₁-C₄) alkoxy (C₁-C₂₀)alkyl, benzyloxy(C₁-C₂₀)alkyl, phenethyloxy (C₁-C₂₀)alkyl, phenoxy (C₁-C₂₀)alkyl, 2-napthyloxy (C₁-C₂₀) alkyl, mercapto, mercapto (C₁-C₂₀)alkyl, (C₁-C₂₀)alkylthio, benzylthio, phenethylthio, (C₁-C₂₀) alkylthio (C₁-C₂₀)alkyl, benzylthio(C₁-C₂₀)alkyl, phenethylthio (C₁-C₂₀)alkyl, amino, (C₁-C₂₀)alkylamino, amino (C₁-C₂₀)alkyl, (C₁-C₂₀)alkylamino (C₁-C₂₀)alkyl, acylamino, acylamino(C₁-C₂₀)alkyl, carboxy, carboxy(C₁-C₂₀) alkyl, (C₁-C₄)alkoxycarbonyl, (C₁-C₄) alkoxycarbony(C₁-C₂₀) lalkyl, benzyloxycarbonyl, phenethyloxycarbonyl, benzyloxycarbonyl (C₁-C₂₀)alkyl, phenethyloxycarbonyl(C₁-C₂₀)alkyl, benzyloxycarbonyloxy, phenethyloxycarbonyloxy, formyl, formyl (C₁-C₂₀)alkyl, acyl, acyloxy, and acyl (C₁-C₂₀)alkyl,
Xᵢ and Xₙ are independently -(CR₁R₂)ₘ-Y-(CR₃R₄)ₚ-where R₁, R₂, R₃ and R₄ are independently hydrogen or (C₁-C₂₀)alkyl, m and p are independently 0 to 5, provided that (m+p) is 0 to 5, and Y is a bond, -O-, -S-, cis or trans -CR₆=CR₇-, -CR₆=CR₇-CR₈=CHR₉- where each of the double bonds may independently be cis or trans, -N(R₅)-, -N(R₅)CO-, -CONR₅-, carbonyl, carbonyloxy, or oxycarbonyl where R₅, R₆, R₇, R₈ and R₉ are independently hydrogen or (C₁-C₂₀)alkyl;
or a pharmaceutically acceptable salt thereof,
provided that when
n is 2;
Tᵢ and Tₜ are hydrogen;
Xᵢ and Xₙ are both carbonyl or methylene; 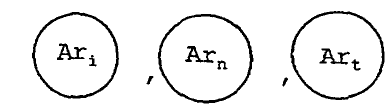 are all phenyl;
then Aᵢ, Aₙ and Aₜ are not all OH or OCOCH₃ and Bᵢ, Bₙ and Bₜ are not F nor Cl, or Aᵢ, Aₙ and Aₜ are not F nor Cl and Bᵢ, Bₙ and Bₜ are not all OH nor OCOCH₃; and provided that the oligomeric compound is not:
the tetrameric (di[5-methoxycarbonylethyl-3-(5-[2-methoxycarbonylethyl]-2-hydroxybenzyl)-2-hydroxyphenyl] methane) and di(5-hydroxycarbonyl-ethyl-3-[5-(2-hydroxycarbonylethyl)-2-hydroxybenzyl]-2-hydroxyphenyl) methane, provided further that at least one of Aᵢ, Bᵢ, Bₙ, Aₜ, Bₜ, Tᵢ and Tₜ is a substituent group chosen from the following list: amines, carboxyl, phosphate, phosphonate ester, sulfate, sulfonate, sulfate esters, sulfonate ester and thiol or a pro-substituent group chosen from the following list: amides, esters, alkylcarbonyl, aldehyde and alkylthiol.

A more particularly preferred class of compounds useful for the present invention is described by formula I, wherein
Mᵢ is 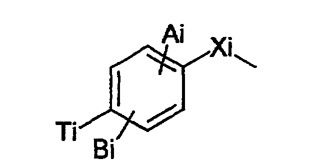
Mₙ is 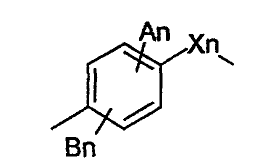
and Mₜ is

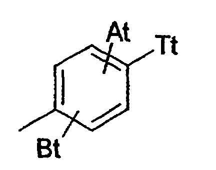

Another preferred class of compounds within the scope of the present invention is described by Formula I, wherein
Mᵢ is 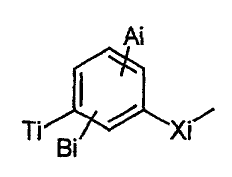
Mₙ is 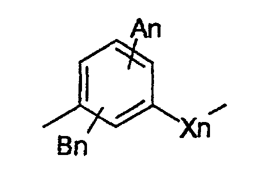
and Mₜ is

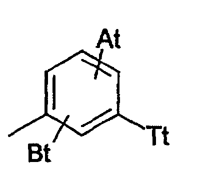

A more preferred class of compounds is described by Formula I, wherein n is a sequence of whole numbers beginning at 2 and having an upper limit of 18 and wherein (Mₙ)ₙ represents from 2 to 18 monomeric units.

Another more preferred class of compounds is described by Formula 1, wherein n is a sequence of whole numbers beginning at 4 and having an upper limit of 14 and wherein (Mₙ)ₙ represents from 4 to 14 monomeric units.

Another more preferred class of compounds is described by Formula 1, wherein n is a sequence of whole numbers beginning at 6 and having an upper limit of 18 and wherein (Mₙ)ₙ represents from 6 to 18 monomeric units.

The compound aspect of the present invention as defined above comprises oligomers having substituent groups (or pro-substituent groups thereof) capable of interacting electrostatically with bioactive molecules such as proteins. For the purposes of this invention, it is understood that substituent groups capable of electrostatic interaction are amines, carboxyl, phosphate, phosphate ester, sulfate, sulfonate, sulfate esters, sulfonate esters, and thiol. It is further understood that the present oligomers can be formulated more conveniently for pharmaceutical administration in a masked or prodrug form utilizing pro-substituent groups which are capable of being metabolized in the body to groups capable of electrostatic interaction. These pro-substituent groups are not limited to amides, esters, alkylcarbonyl, aldehyde and alkyl thiol.

A special embodiment of the present invention is described by Formula II below: 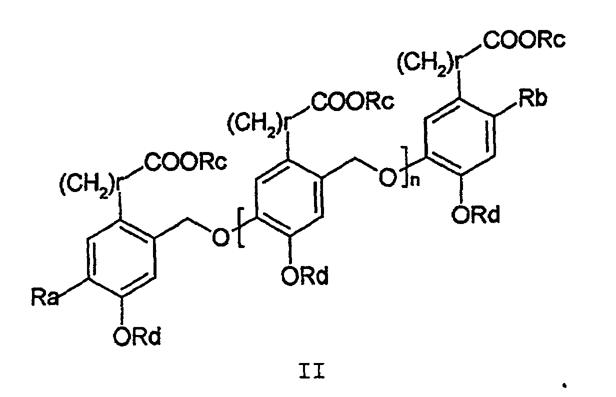 wherein
Ra is hydrogen, hydroxy or acyloxy
Rb is hydrogen, (C₁-C₂₀)alkyl or hydroxy(C₁-C₂₀)alkyl;
Rc is hydrogen or (C₁-C₂₀)alkyl;
Rd is hydrogen or (C₁-C₂₀)alkyl;
r is 1 to 4 and
n is 2 to 30;
or a pharmaceutically acceptable salt thereof.

Another special embodiment of the present invention is described by Formula III below, 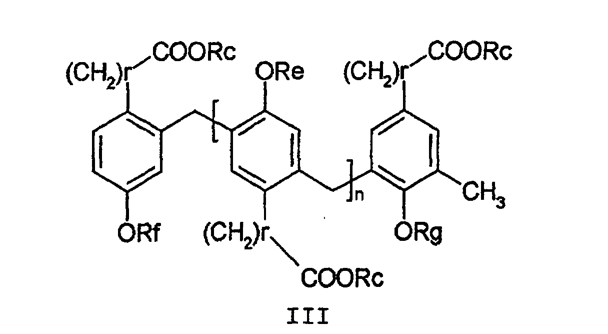 wherein
R_{c}, Rₑ, R_{f} and R_{g} are independently hydrogen or (C₁-C₂₀)alkyl;
r is 1 to 4 and
n is 1 to 30;
or a pharmaceutically acceptable salt thereof.

Another special embodiment of the present invention is described by preceding formulas, wherein n is a sequence of whole numbers having an upper limit of from 4 to 8.

Representative compounds of the present invention include:
- 2-[4-[4-[4-[[5-(2-carboxyethyl)-2-methoxy] phenoxymethyl]-[5-(2-carboxyethyl)-2-methoxy] phenoxymethyl]-[5-(2-carboxy-ethyl)-2-methoxy]phenoxymethyl]-[5-(2-carboxyethyl)-2-methoxy]phenoxymethyl]-4-methoxyhydro-cinnamic acid,
- 1,2-bis-[4-[4-[4-[[2-(2-carboxyethyl)-5-hydroxy] benzyl]-[2-(2-carboxyethyl-5-hydroxy]benzyl]-[2-(2-carboxyethyl)-5-hydroxy]benzyl]-[2-(2-carboxyethyl)-5-hydroxy]phenyl] ethane
- bis-[5-(2-carboxyethyl)-3-[5-(2-carboxyethyl)-2-hydroxybenzyl]-2-hydroxyphenyl],
- methyl-5-acetoxy-2-[4-[4-[[5-(2-methoxycarbonylethyl)-4-hydroxymethyl-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-4-methoxyhydrocinnamate,
- 1,2-bis-[2-(2-carboxyethyl)-4-[2-(2-carboxyethyl)-5-hydroxybenzyl]-5-hydroxyphenyl],
- 5-[4-[4-[4-[4-[4-[4-[[2-(2-carboxyethyl)-5-hydroxy] benzyl]-[2-(2-carboxyethyl)-5-hydroxy]benzyl]-[2- (2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl) -5-hydroxy]benzyl]-4-hydroxy-2- methylhydrocinnamic acid,
- 2-(2-methoxycarbonylethyl)-4-[4-[4-[4-[4-[4-[4-[[2-(2-methoxycarbonylethyl)-5-trimethylacetyloxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-5-methoxybenzyloxy-tert-butyldimethylsilane,
- 1,2-bis-[4-[4-[4-[[2-(2-methoxycarbonylethyl)-5-trimethylacetyloxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]phenyl]ethane,
- 5-[4-[4-[4-[4-[4-[4-[[2-(2-carboxyethyl)-5-hydroxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-4-methoxy-2-methylhydrocinnamic acid,
- 5-[4-[4-[4-[4-[4-[4-[4-[4-[4-[4-[4-[4-[4-[4-[[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-4-hydroxy-2-methylhydrocinnamic acid,
- 1,2-bis-[4-[4-[4-[4-[4-[4-[4-[[2-(2-carboxyethyl)-5-hydroxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]phenyl]ethane,
- 5-[4-[4-[4-[4-[4-[4-[4-[4-[4-[4-[4-[4-[4-[4-[[2-(2-carboxyethyl)-5-hydroxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-4-hydroxy-2-methylhydrocinnamic acid,
- methyl-5-acetoxy-2-[4-[4-[4-[4-[4-[4-[[5-(2-methoxycarbonylethyl)-4-hydroxymethyl-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-4-methoxyhydrocinnamate,
- methyl-5-acetoxy-2-[4-[4-[4-[4-[4-[4-[4-[4-[4-[4-[4-[4-[4-[4-[[5-(2-methoxycarbonylethyl)-4-hydroxymethyl-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-(5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-4-methoxyhydrocinnamate,
- 1,2-bis-[4-[4-[4-[[2-(2-carboxyethyl)-5-hydroxy] benzyl]-[2-(2-carboxyethyl)-5-methoxy]benzyl]-[2-(2-carboxyethyl)-5-methoxy]benzyl]-[2-(2-carboxyethyl)-5-methoxy]phenyl]ethane.
- methyl-5-acetoxy-2-[4-[4-[4-[4-[4-[4-[4-[4-[4-[4-[4-[4-[4-[4-[[5-(2-methoxycarbonylethyl)-4-hydroxymethyl-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-4-methoxyhydrocinnamate,
- 1,2-bis-[4-[4-[4-[[2-(2-carboxyethyl)-5-hydroxy]benzyl]
- [2-(2-carboxyethyl)-5-methoxy]benzyl]-[2-(2-carboxyethyl)-5-methoxy]benzyl]-[2-(2-carboxyethyl)-5-methoxy]phenyl]ethane.

Another object of the present invention is the use of a composition according to the instant invention for the manufacture of a medicament for treating an elastase-mediated connective tissue degradation and for the manufacture of a medicament for treating thrombosis, inhibiting smooth muscle cell proliferation, or use as anticoagulant.

Another object of the present invention is an oligomeric compound comprising a single polymeric species of the formula I

Mᵢ-(Mₙ)ₙ-Mₜ I

wherein
n represents a sequence of whole numbers beginning at 2 and having an upper limit of 50;
Mᵢ is 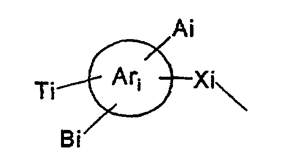
Mₙ is 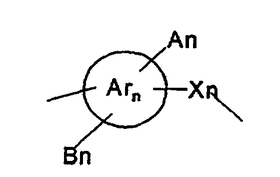
Mₜ is 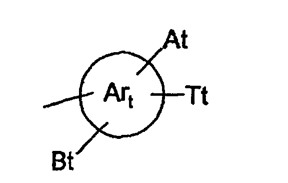 where 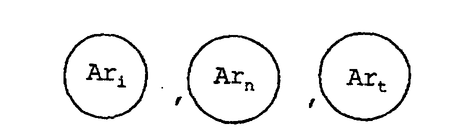 are independently benzene or naphthalene,
   where 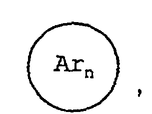 Aₙ , Bₙ, and Xₙ
   may be the same or different for each of the (Mₙ) groups in the n sequence; and
Aᵢ, Bᵢ, Aₙ, Bₙ, Aₜ and Bₜ are independently hydrogen, (C₁-C₂₀)alkyl, benzyl, phenethyl, cyano, (C₁-C₄)alkoxy(C₁-C₄)alkoxy, or -(CH₂)ₐ-W-(CH₂)_{b}-Rₛ, where Rₛ is NRR', COOR, CONRR', NR(COR'), PO(OR)₂, COR, SO₂OR, OSO₂OR, halogen, OR, SO₂R, SOR, SR or CHO, and where a and b are independently 0 to 4, (a+b) is less than 5, W is -O-, -S-, -SO-, -SO₂-, -NR(COR')-, -NR-, or a bond, and R and R' are independently hydrogen, (C₁-C₂₀)alkyl, benzyl, or phenethyl,
Tᵢ and Tₜ are independently hydrogen, (C₁-C₂₀)alkyl, allyl, vinyl, halo, cyano, (C₁-C₄)alkoxy(C₁-C₄)alkoxy, halo(C₁-C₂₀)alkyl, hydroxy, *tert*-butyldimethylsilyloxy(C₁-C₂₀)alkyl, hydroxy(C₁-C₂₀)alkyl, (C₁-C₄)alkoxy, benzyloxy, phenethyloxy, phenoxy, 2-napthyloxy, (C₁-C₄) alkoxy(C₁-C₂₀)alkyl, benzyloxy(C₁-C₂₀)alkyl, phenethyloxy (C₁-C₂₀)alkyl, phenoxy(C₁-C₂₀)alkyl, 2-napthyloxy(C₁-C₂₀)alkyl, mercapto, mercapto (C₁-C₂₀)alkyl, (C₁-C₂₀)alkylthio, benzylthio, phenethylthio, (C₁-C₂₀)alkylthio (C₁-C₂₀)alkyl, benzylthio (C₁-C₂₀)alkyl, phenethylthio (C₁-C₂₀)alkyl, amino, (C₁-C₂₀)alkylamino, amino (C₁-C₂₀)alkyl, (C₁-C₂₀)alkylamino (C₁-C₂₀)alkyl, acylamino, acylamino(C₁-C₂₀)alkyl, carboxy, carboxy(C₁-C₂₀)alkyl, (C₁-C₄)alkoxycarbonyl, (C₁-C₄)alkoxycarbony(C₁-C₂₀)lalkyl, benzyloxycarbonyl, phenethyloxycarbonyl, benzyloxycarbonyl(C₁-C₂₀)alkyl, phenethyloxycarbonyl (C₁-C₂₀)alkyl, benzyloxycarbonyloxy, phenethyloxycarbonyloxy, formyl, formyl(C₁-C₂₀)alkyl, acyl, acyloxy, and acyl(C₁-C₂₀)alkyl,
Xᵢ and Xₙ are independently -(CR₁R₂)ₘ-Y-(CR₃R₄)ₚ-where R₁, R₂, R₃ and R₄ are independently hydrogen or (C₁-C₂₀)alkyl, m and p are independently 0 to 5, provided that (m+p) is 0 to 5, and Y is a bond, -O-, -S-, cis or trans -CR₆=CR₇-, -CR₆=CR₇-CR₈=CHR₉- where each of the double bonds may independently be cis or trans, -N(R₅)-, -N(R₅)CO-, -CONR₅-, carbonyl, carbonyloxy, or oxycarbonyl where R₅, R₆, R₇, R₈ and R₉ are independently hydrogen or (C₁-C₂₀)alkyl;
or a pharmaceutically acceptable salt thereof,
provided that when
n is 2;
Tᵢ and Tₜ are hydrogen;
Xᵢ and Xₙ are both carbonyl or methylene; 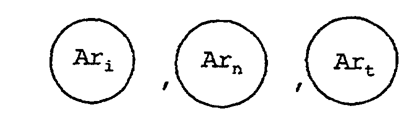 are all phenyl;
then Aᵢ, Aₙ and Aₜ are not all OH or OCOCH₃ and Bᵢ, Bₙ and Bₜ are not F nor Cl, or Aᵢ, Aₙ and Aₜ are not F nor Cl and Bᵢ, Bₙ and Bₜ are not all OH nor OCOCH₃;
and
provided that the oligomeric compound is not:
the tetrameric (di[5-methoxycarbonylethyl-3-(5-[2-methoxycarbonylethyl]-2-hydroxybenzyl)-2-hydroxyphenyl] methane) and di(5-hydroxycarbonyl-ethyl-3-[5-(2-hydroxycarbonylethyl)-2-hydroxybenzyl]-2-hydroxyphenyl) methane, provided further that at least one of Aᵢ, Bᵢ, Bₙ, Aₜ, Bₜ, Tᵢ and Tₜ is a substituent group chosen from the following list: amines, carboxyl, phosphate, phosphonate ester, sulfate, sulfonate, sulfate esters, sulfonate ester and thiol or a pro-substituent group chosen from the following list: amides, esters, alkylcarbonyl, aldehyde and alkylthiol.

Compounds of the present invention may contain asymmetric centers. These asymmetric centers may independently be in either the R or S configuration. The present invention comprises the individual stereoisomers and mixtures thereof.

The compounds of the present invention may be useful in the form of the free base or acid or in the form of a pharmaceutically acceptable salt thereof. All forms are within the scope of the invention.

Where the compound of the present invention is substituted with one or more basic moieties, acid addition salts may be formed and are simply a more convenient form for use; and in practice, use of the salt form inherently amounts to use of the free base form. The acids which can be used to prepare the acid addition salts include preferably those which produce, when combined with the free base, pharmaceutically acceptable salts, that is, salts whose anions are non-toxic to the animal organism in pharmaceutical doses of the salts, so that the beneficial properties inherent in the free base are not vitiated by side effects ascribable to the anions. Although pharmaceutically acceptable salts of said basic compounds are preferred, all acid addition salts are useful as sources of the free base form even if the particular salt, per se, is desired only as an intermediate product as for example, when the salt is formed only for purposes of purification, and identification, or when it is used as intermediate in preparing a pharmaceutically acceptable salt by ion exchange procedures. Pharmaceutically acceptable salts within the scope of the invention are those derived from the following acids: mineral acids such as hydrochloric add, sulfuric acid, phosphoric acid and sulfamic acid; and organic acids such as acetic acid, citric acid, lactic acid, tartaric acid, malonic acid, methanesufonic acid, ethanesulfonic acid, benzenesulfonic acid, p-toluenesulfonic acid, cyclohexylsulfamic acid, quinic acid, and the like. The corresponding acid addition salts comprise the following: hydrochloride, sulfate, phosphate, sulfamate, acetate, citrate, lactate, tartarate, malonate, methanesulfonate, ethanesulfonate, benzenesulfonate, p-toluenesulfonate, cyclohexylsulfamate and quinate, respectively.

The acid addition salts of the compounds of this invention are prepared either by dissolving the free base in aqueous or aqueous-alcohol solution or other suitable solvents containing the appropriate acid and isolating the salt by evaporating the solution, or by reacting the free base and acid in an organic solvent, in which case the salt separates directly or can be obtained by concentration of the solution.

Where the compound of the invention is substituted by one or more acidic moieties, base addition salts may be formed and are simply a more convenient form for use; and in practice, use of the salt form inherently amounts to use of the free acid form. The bases which can be used to prepare the base addition salts include preferably those which produce, when combined with the free acid, pharmaceutically acceptable salts, that is, salts whose cations are non-toxic to the animal organism in pharmaceutical doses of the salts, so that the beneficial properties inherent in the free acid are not vitiated by side effects ascribable to the cations. Pharmaceutically acceptable salts within the scope of the invention are those derived from the following bases: sodium hydroxide, potassium hydroxide, calcium hydroxide, aluminum hydroxide, lithium hydroxide, magnesium hydroxide, zinc hydroxide, ammonia, ethylenediamine, N-methyl-glucamine, lysine, arginine, ornithine, choline, N,N'-dibenzylethylenediamine, chloroprocaine, diethanolamine, procaine, N-benzylphenethylamine, diethylamine, piperazine, tris(hydroxymethyl)aminomethane, tetramethylammonium hydroxide, and the like.

Metal salts of compounds of the present invention may be obtained by contacting a hydroxide, carbonate or similar reactive compound of the chosen metal in an aqueous solvent with the free acid form of the compound. The aqueous solvent employed may be water or it may be a mixture of water with an organic solvent, preferably an alcohol such as methanol or ethanol, a ketone such as acetone, an aliphatic ether such as tetrahydrofuran, or an ester such as ethyl acetate. Such reactions are normally conducted at ambient temperature but they may, if desired, be conducted with heating.

Amine salts of compounds of the present invention may be obtained by contacting an amine in an aqueous solvent with the free acid form of the compound. Suitable aqueous solvents include water and mixtures of water with alcohols such as methanol or ethanol, ethers such as tetrahydrofuran, nitriles such as acetonitrile, or ketones such as acetone. Amino acid salts may be similarly prepared.

Compounds of this invention may be prepared in accordance with the reaction sequences described below, and certain of the compounds can be prepared by methods known in the art. The starting materials used in the preparation of compounds of this invention are known or are commercially available, or can be prepared by known methods or by specific reaction schemes described herein.

The oligomeric compounds of the present invention are generally available by a series of reactions whereby monomeric or oligomeric precursors are sequentially coupled together and/or modified to prepare the desired compound.

If it is necessary or desirable to prevent cross-reaction between chemically active substituents on the monomeric or oligomeric precursor compounds, either during coupling reactions or at other points in the reaction sequence, the substituents may be protected by standard blocking groups which may be retained or subsequently removed, as required, by known methods to afford the desired product or precursor (see, for example, Green, "Protective Groups in Organic Synthesis", Wiley, New York, 1981).

A preferred method of preparation of compounds of the present invention is shown in Scheme I below.

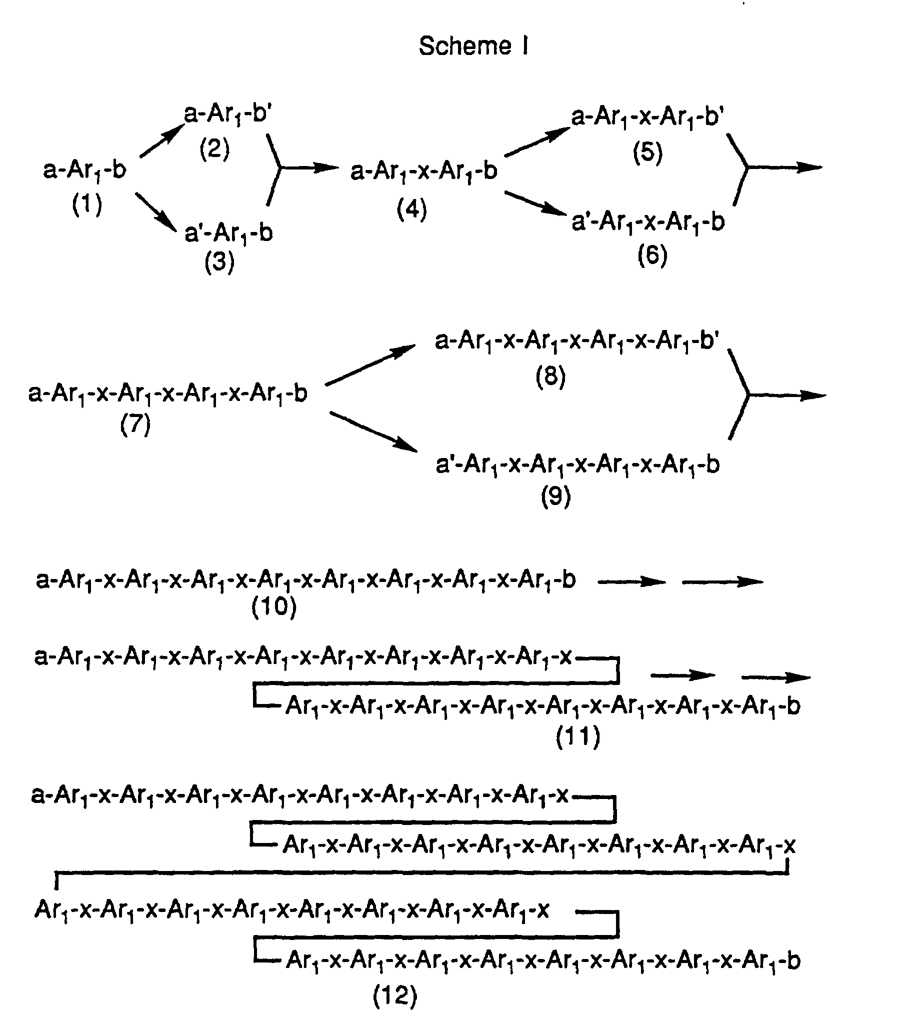

Ar₁ represents an aromatic carbocyclic or aromatic heterocyclic ring containing the substituent groups desired in the final oligomeric product, or protected derivatives thereof, or precursor moieties thereto. Ar₁ also contains the substituent groups a and b, which groups may be selectively and independently converted to groups a' and b', which groups a' and b' are such so as to form a linking group x between the aromatic groups when subjected to the appropriate reaction conditions.

In Scheme I, the monomeric precursor (1) is converted to two separate monomeric precursors (2) and (3) by chemical modification of the substitutent groups b and a, respectively. Precursor (2) has a substituent moiety b' which is chemically reactive toward a substituent moiety a' on precursor (3). (2) and (3) are then reacted together to give the dimer (4), the two monomeric residues being connected by the linking group x.

The dimeric precursor (4) is likewise selectively converted to the two separate dimeric precursors (5) and (6) which contain the substituent groups b' and a', respectively. (5) and (6) are then reacted together to form the tetramer (7). This sequence is repeated to prepare the octomer (10), hexadecamer (11), and the 32-membered oligomer (12).

This method of preparation is also used to prepare compounds of the present invention containing non-identical monomeric residues. An example of this is shown in Scheme II below.

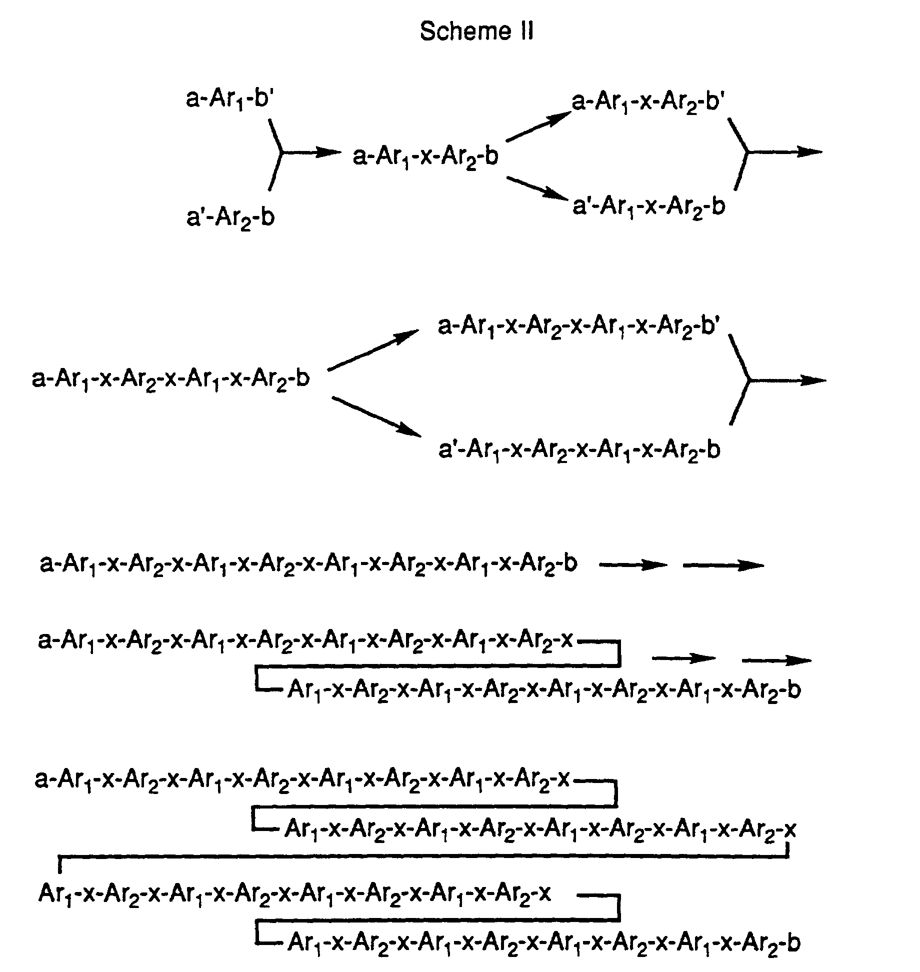

In Scheme II, two different monomeric precursors are coupled as above and the resulting dimer carried on as in Scheme I to give oligomers having repeating units of the dimer.

It should be apparent that different monomeric or oligomeric precursors (i.e. monomeric or oligomeric precursors containing different aromatic rings or containing different substituent groups or protected derivatives or precursor moieties thereto, or oligomeric precursors containing varying numbers of monomeric units) may be introduced at any stage in the above Schemes to effect preparation of oligomers containing any desired number of monomeric residues, each having any desired substituent groups. It should also be apparent that the nature of the linking group x at a given point in the oligomer may be varied by varying the nature of the groups a' and b'.

An exemplary preparation scheme is shown in Scheme III below.

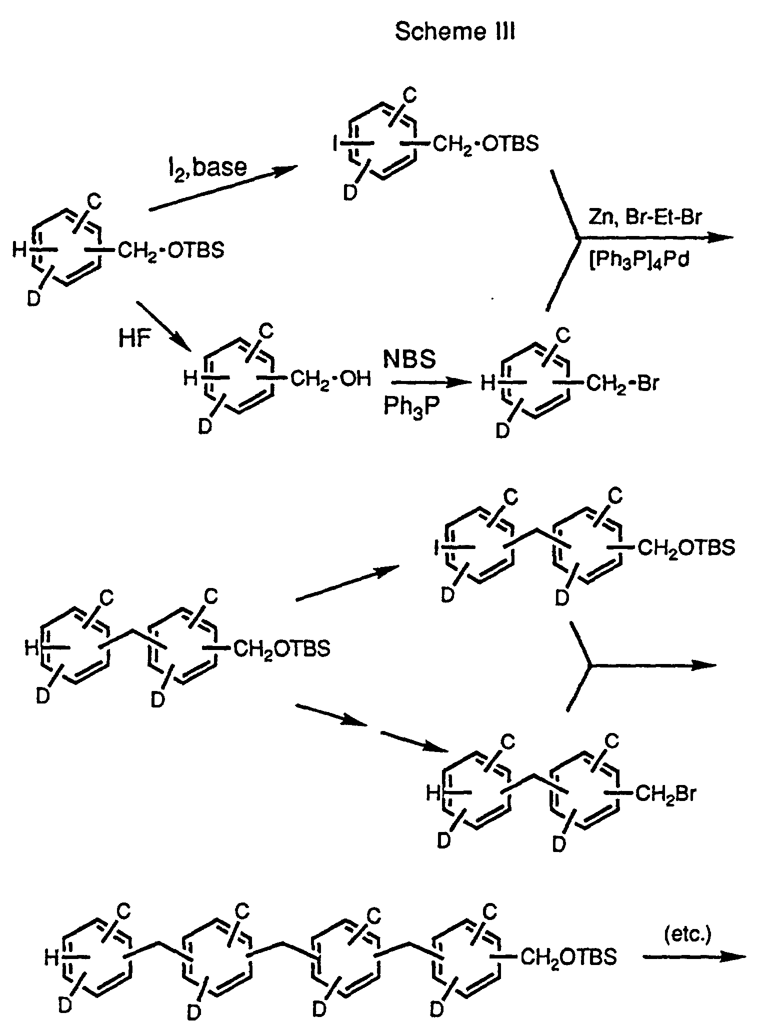

In Scheme III a monomeric precursor benzyl alcohol, protected as the tert-butyldimethylsilyl ether, and substituted with substituent groups C and D, which may be the substituent groups desired in the final product or may be protected derivatives thereof or precursor substituents thereto, is treated with iodine in the presence of an organic base, for example morpholine, in an organic solvent, for example methylene chloride, to give the aryl iodide.

The monomeric precursor silyl ether, in a separate step, is deprotected by treatment with 48% hydrofluoric acid in an aprotic organic solvent, for example acetonitrile, to prepare the benzyl alcohol. The benzyl bromide is then prepared by treatment of the benzyl alcohol with N-bromosuccinimide in the presence of triphenylphosphine in an aprotic organic solvent, for example tetrahydrofuran.

The resulting aryl iodide and benzyl bromide then may be coupled, for example by treatment with zinc and 1,2-dibromoethane, in the presence of tetrakis(triphenylphosphine)palladium, in an aprotic organic solvent, for example tetrahydrofuran, to form the dimeric product. In this example the linking group so formed is the methylene group.

The resulting dimeric silyl ether may then be sequentially converted to the analgous aryl iodide and benzyl bromide and these dimeric products coupled, as above, to prepare the analogous tetramer. This sequence may be repeated, as required, to prepare the oligomer of desired length. The substituent groups C and D may then be deprotected or modified, if necessary or desired, to prepare the compound of the present invention.

Another exemplary preparation is shown in Scheme IV below.

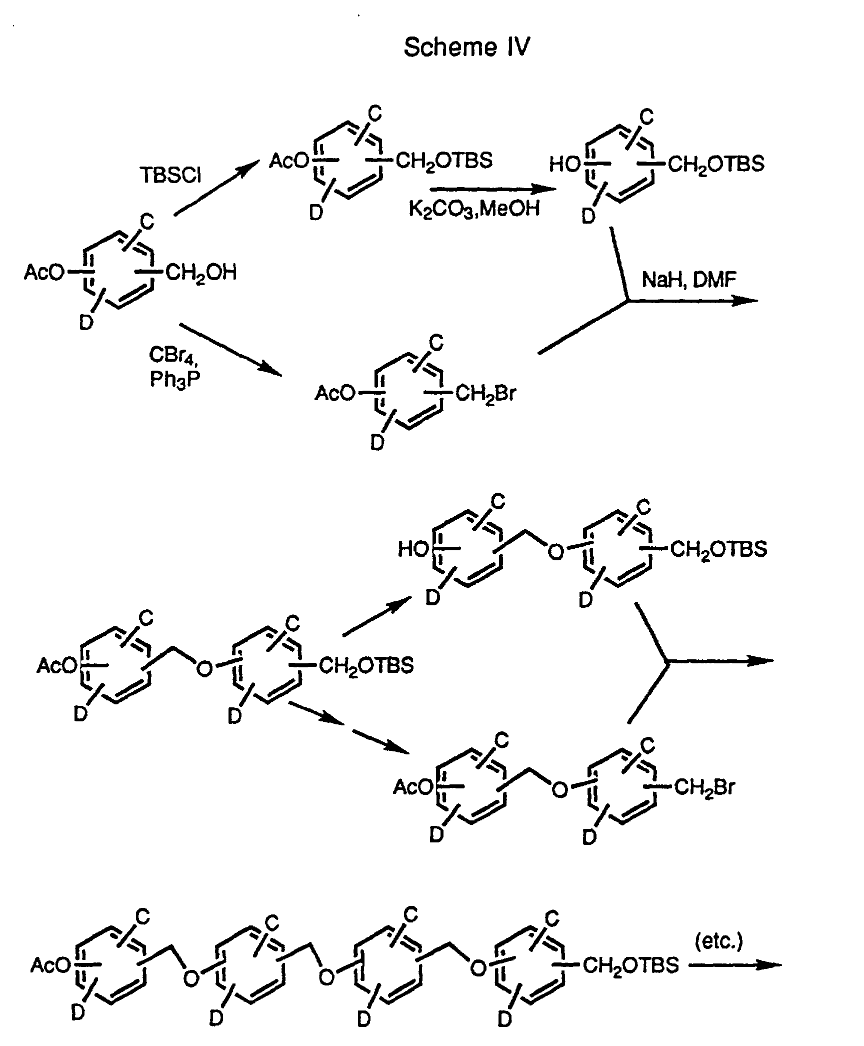

The appropriately substituted acetoxy benzyl alcohol is converted to the tert-butyldimethylsilyl ether by treatment with tert-butylchlorodimethylsilane in an aprotic organic solvent, for example methylene chloride. The acetyl group is then removed by treatment with potassium carbonate in a polar organic solvent such as methanol to prepare the corresponding phenol.

The acetoxy benzyl alcohol is separately converted to the benzyl bromide by treatment with carbon tetrabromide in the presence of triphenylphosphine in an aprotic organic solvent, for example tetrahydrofuran.

The resulting benzyl bromide and phenol are coupled by treatment with a strong base, for example sodium hydride, in a polar aprotic organic solvent, for example dimethylformamide to prepare the dimeric precursor. In this example the linking group so formed is the oxymethyl group.

The resulting dimeric silyl ether may then be sequentially converted to the analgous phenol and benzyl bromide and these dimeric products coupled, as above, to prepare the analogous tetramer. This sequence may be repeated, as required, to prepare the oligomer of desired length. The substituent groups C and D may then be deprotected or modified, if necessary or desired, to prepare the compound of the present invention.

The methods of preparation of compounds of the present invention discussed above allow the formation of essentially pure oligomeric intermediates or final products, each having specific composition and length, in high yield. A limited number of compounds of the present invention, in particular the tetramers, may also be prepared treating monomeric precursors under conditions, known in the art, which cause the precursors to condense or couple with each other to form a mixture of oligomers which may then be separated by standard methods to give the desired products. An exemplary preparation of this kind is shown in Scheme V below.

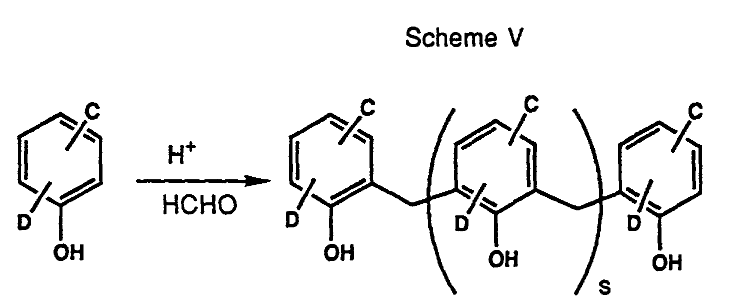

An appropriately substituted phenol is treated with formalin in the presence of a mineral acid, for example sulfuric acid, in methanol to give a mixture of products which includes oligomers of the desired composition, being phenolic monomers linked by methylene groups, and containing the desired number, s+2, of monomeric units. The individual components of the mixture are then isolated by standard methods, for example flash chromatography or HPLC.

The present invention is further explained by the following illustrative examples.

### Example 1

### Preparation of bis-[5-(2-carboxyethyl)-3-[5-(2-carboxyethyl)-2-hydroxybenzyl]2-hydroxyphenyl]methane

### Step 1: Preparation of methyl 3-(4-hydroxyphenyl) propionate

A solution of p-hydroxycinnamic acid (32.8g) in methanol (200 ml) is hydrogenated over 5% palladium on carbon (0.5g) until hydrogen uptake ceases. The catalyst is removed by filtration and sulfuric acid (1 ml) added to the filtrate and the resulting solution stirred at reflux for about 18 hours. The solution is concentrated in vacuo, the residue dissolved in ether and the solution washed with water, saturated sodium bicarbonate solution, and brine. The organic solution is concentrated in vacuo to give the desired product.

### Step 2: Preparation of bis-[5-(2-methoxycarbonylethyl)-3-[5-(2-methoxycarbonylethyl)-2-hydroxybenzyl]2-hydroxyphenyl]methane

A solution of methyl 3-(4-hydroxyphenyl) propionate (4.68g) in methanol (8 ml) is cooled to 0°C and sulfuric acid (16 ml) is cautiously added. The temperature is allowed to return to 0°C and a solution of 37% aqueous formaldehyde (0.8 ml) in methanol (1 ml) is added dropwise over about 4 hours. The mixture is stirred at room temperature for about 18 hours, poured over ice, this mixture is extracted with ethyl acetate, and the ethyl acetate solution concentrated in vacuo. The crude product is flash chromatographed over silica gel, eluting with 50% ethyl acetate in hexane. The mixture of oligomers so obtained is purified by preparative HPLC to give the desired product.

### Step 3: Preparation of bis-[5-(2-carboxyethyl)-3-[5-(2-carboxyethyl)-2-hydroxybenzyl]2-hydroxyphenyl]methane

A solution of bis-[5-(2-methoxycarbonylethyl)-3-[5-(2-methoxycarbonylethyl)-2-hydroxybenzyl]2-hydroxyphenyl]methane (0.1g) in methanol (2 ml) is stirred with 10% aqueous sodium hydroxide (NaOH) (1 ml) at room temperature. The mixture is quenched with dilute aqueous hydrochloric acid (HCl) and the organic soluble material retreated as above. The mixture is quenched as above and the organic soluble material flash chromatographed, eluting with 10% methanol in chloroform. The product so obtained is treated with ammonium hydroxide solution and lyophilized to give the desired product as the ammonium salt, m.p. 150°C (dec.).

### Example 2

Preparation of 5-[4-[4-[4-[4-[4-[4-[4-[4-[4-[4-[4-[4-[4-[4-[[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-4-hydroxy-2-methylhydrocinnamic acid

### Step 1: Preparation of 2-bromo-5-trimethylacetyloxybenzaldehyde

To a suspension of 3-hydroxybenzaldehyde (36g) in chloroform (800 ml) is added a solution of bromine (15.8 ml) in chloroform (60 ml) over a period of about 2.5 minutes. The mixture is stirred for 5 minutes, washed with dilute sodium bicarbonate solution, then water, and the organic solution is dried over magnesium sulfate, filtered and concentrated in vacuo. Dry toluene is azeotroped from the residue and this residue is dissolved in methylene chloride (700 ml). To this solution is added triethylamine (55 ml), trimethylacetic anhydride (70 ml), and 4-dimethylaminopyridine (DMAP) (2g). The solution is stirred for 14 hours, diluted with ether, and washed with water, then brine. The organic solution is dried over magnesium sulfate, concentrated in vacuo and the residue purified by flash chromatography, eluting with 5% ether in hexanes, to give the desired product.

### Step 2: Preparation of 2-tert-butyldimethylsilyloxymethyl-4-trimethylacetyloxybromobenzene

A solution of 2-bromo-5-trimethylacetyloxybenzaldehyde (52g) is tetrahydrofuran (THF) (400 ml) is cooled to -78°C and 0.5M sodium borohydride in diglyme (182 ml) is added. The solution is stirred at room temperature for 20 minutes, quenched with 1 M HCl (200 ml), and diluted with ether. The ether solution is washed with six portions of water, dried over magnesium sulfate, filtered, and concentrated in vacuo. Dry toluene is azeotroped from the residue and this residue is dissolved in anhydrous dimethylformamide (DMF) (400 ml) and imidazole (15 g), tert-butyldimethylchlorosilane (30 g) and DMAP (1.26g) are added. The solution is stirred for 30 minutes, diluted with ether and the ether solution washed with water, dried over magnesium sulfate, filtered and concentrated in vacuo. The residue is purified by flash chromatography, eluting with 2% ether in hexanes, to give the desired product.

### Step 3: Preparation of methyl 2-tert-butyldimethylsilyloxymethyl-4-trimethylacetyloxycinnamate

To a solution of 2-tert-butyldimethylsilyloxymethyl-4-trimethylacetyloxybromobenzene (48g) in dry dimethylformamide (460 ml) is added bis(triphenylphosphine)palladium dichloride (2.5g). The resulting solution is degassed and dry triethylamine (67 ml) added, followed by methyl acrylate (42.6ml). The resulting solution is stirred at 135°C for about 1 hour, cooled, diluted with ether, washed with water, dried over magnesium sulfate, filtered, then concentrated under reduced pressure. The residue is purified by flash chromatography, eluting with 10% ether in hexanes, to give the desired product.

### Step 4: Preparation of methyl 2-tert-butyldimethylsilyloxymethyl-4-trimethylacetyloxyhydrocinnamate

A solution of methyl 2-tert-butyldimethylsilyloxymethyl-4-trimethylacetyloxycinnamate (40.8g) and tris(triphenylphosphine)rhodium chloride (2.5g) in dry benzene (500 ml) is degassed, then stirred under an atmosphere of hydrogen at 40°C for 48 hours. The hydrogen atmosphere is replaced by nitrogen and the solution concentrated in vacuo. The residue is diluted with ether and the mixture filtered, and the filtrate concentrated in vacuo to give the desired product.

### Step 5: Preparation of methyl 2-tert-butyldimethylsilyloxymethyl-4-hydroxy-5-iodohydrocinnamate

A solution of methyl 2-tert-butyldimethylsilyloxymethyl-4-trimethylacetyloxy hydrocinnamate (8.16g) and 25% sodium methoxide in methanol (3.9 ml) in methanol (40 ml) is stirred at room temperature for 30 minutes and diluted with ether. The ether solution is washed with 1M hydrochloric acid, brine, dried over magnesium sulfate, concentrated in vacuo and the residue dissolved in methylene chloride (145 ml). To this solution is added morpholine (4.25g), then iodine (6.09g) and the resulting suspension stirred at room temperature for 2 hours and diluted with ether. The ether solution is washed with 1 M HCI (25 ml), then brine, dried over magnesium sulfate, filtered, and concentrated in vacuo. The residue is purified by flash chromatography, eluting with 15% ether in hexanes, to give the desired product.

### Step 6: Preparation of methyl 2-tert-butyldimethylsilyloxymethyl-4-methoxy-5-iodohydrocinnamate

A suspension of 60% sodium hydride in mineral (1.2g) in THF (56 ml) is cooled to -20°C and of solution of methyl 2-tert-butyidimethylsilyloxymethyl-4-hydroxy-5-iodohydrocinnamate (12.6g), iodomethane (5 ml) and 1,3-dimethyl-3,4,5,6-tetrahydro-2(1H)-pyrimidinone (DMPU) (8 ml) in THF (20 ml) is added. The cold bath is removed, the mixture stirred for about 2 hours, and diluted with ether. The ether solution is washed with 0.1 M HCI, brine, dried over magnesium sulfate, filtered and concentrated in vacuo. The residue is purified by flash chromatography, eluting with 10% ether in hexanes, to give the desired product.

### Step 7: Preparation of methyl 2-hydroxymethyl-4-trimethylacetyloxy hydrocinnamate

To a solution of methyl 2-tert-butyldimethylsilyloxymethyl-4-trimethylacetyloxyhydrocinnamate (32.6g) in acetonitrile (350 ml) is added 48% hydrofluoric acid (4.6 ml) and the solution stirred at room temperature for 40 minutes, the diluted with ether. The ether solution is washed with water, dilute sodium bicarbonate solution, brine, dried over magnesium sulfate, and concentrated in vacuo. To the residue is added triethylamine (5 ml) and this purified by flash chromatography, eluting with 60% ether in hexanes, to give the desired product.

### Step 8: Preparation of methyl 2-bromomethyl-4-trimethylacetyloxyhydrocinnamate

To a solution of 2-hydroxymethyl-4-trimethylacetyloxy hydrocinnamate (22.6g) in THF (800 ml) is added triphenylphosphine (31.4g), then recrystallized N-bromosuccinimide (20g). The mixture is stirred for about 15 minutes, concentrated in vacuo, and the residue purified by flash chromatography, eluting with 30% ether in hexanes, to give the desired product.

### Step 9: Preparation of methyl 2-tert-butyldimethylsilyloxymethyl-4-methoxy-5-[2-(2-methoxycarbonylethyl)-5-trimethylacetyloxy]benzyl hydrocinnamate

To a suspension of zinc dust (3.64g) in THF (4 ml) is added freshly distilled 1,2-dibromoethane and the mixture is warmed to reflux, stirred for about 1 minute, then cooled to -5°C. A solution of 2-bromomethyl-4-trimethylacetyloxyhydrocinnamate (8.28g) in THF (20 ml) is added over about 1.3 hours, and the mixture is stirred for and additional 30 minutes. Stirring is stopped and the solid allowed to settle. The supernatent liquid is transferred via cannula to a solution of methyl 2-tert-butyldimethylsilyloxymethyl-4-methoxy-5-iodo hydrocinnamate (9.26g) and
tetrakis(triphenylphosphine)palladium (1.17g) in THF (40 ml). The resulting solution is stirred at 60°C for about 2 hours, diluted with ether, washed with 5% aqueous ammonia, the brine. The ether solution is dried over magnesium sulfate, concentrated in vacuo, and the residue purified by flash chromatography, eluting with a gradient of 20% to 30% ether in hexanes to give the desired product.

### Step 10: Preparation of methyl 2-tert-butyldimethylsilyloxymethyl-4-methoxy-5-[2-(2-methoxycarbonylethyl)-5-hydroxy-4-iodo]benzyl hydrocinnamate

Using essentially the procedure of Example 2, Step 5, and purifying the crude product by flash chromatography, eluting with a gradient of 20% to 25% ether in hexanes, the desired product is prepared from methyl 2-tert-butyldimethylsilyloxymethyl-4-methoxy-5-[2-(2-methoxycarbonylethyl)-5-trimethylacetyloxy]benzyl hydrocinnamate.

### Step 11: Preparation of methyl 2-tert-butyldimethylsilyloxymethyl-4-methoxy-5-(2-(2-methoxycarbonylethyl)-5-methoxy-4-iodo]benzyl hydrocinnamate

Using essentially the procedure of Example 2, Step 6, and purifying the crude product by flash chromatography, eluting with a gradient of 20% to 25% ether in hexanes, the desired product is prepared from methyl 2-tert-butyldimethylsilyloxymethyl-4-methoxy-5-[2-(2-methoxycarbonylethyl)-5-hydroxy-4-iodo]benzyl hydrocinnamate.

### Step 12: Preparation of methyl 2-hydroxymethyl-4-methoxy-5-[2-(2-methoxycarbonylethyl)-5-trimethylacetyloxy)benzylhydrocinnamate

Using essentially the procedure of Example 2, Step 7, and purifying the crude product by flash chromatography, eluting with a gradient of 60% to 80% ether in hexanes, the desired product is prepared from methyl 2-tert-butyldimethylsilyloxymethyl-4-methoxy-5-[2-(2-methoxycarbonylethyl)-5-trimethylacetyloxy]benzyl hydrocinnamate.

### Step 13: Preparation of methyl 2-bromomethyl-4-methoxy-5-[2-(2-methoxycarbonylethyl)-5-trimethylacetyloxy]benzylhydrocinnamate

Using essentially the procefure of Example 2, Step 8, and purifying the crude product by flash chromatography, eluting with 40% ether in hexanes, the desired product is prepared from methyl 2-hydroxymethyl-4-methoxy-5-[2-(2-methoxycarbonylethyl)-5-trimethylacetyloxy]benzyl hydrocinnamate.

### Step 14: Preparation of methyl 5-[4-[4-[[2-(2-methoxycarbonylethyl)-5-trimethylacetyloxy]benzyl]-[2-(2-methoxycarbonylethyl)-5-methoxy]benzyl]-[2-(2-methoxycarbonylethyl)-5-methoxy]benzyl]-4-methoxy-2-tert-butyldimethylsilyloxymethylhydrocinnamate

Using essentially the procedure of Example 2, Step 9, and purifying the crude product by flash chromatography, eluting with ether/methylene chloride/hexane (4:1:5), the desired product is prepared from 2-bromomethyl-4-methoxy-5-[2-(2-methoxycarbonylethyl)-5-trimethylacetyloxy]benzyl hydrocinnamate and methyl 2-tert-butyldimethylsilyloxymethyl-4-methoxy-5-[2-(2-methoxycarbonylethyl)-5-methoxy-4-iodo]benzyl hydrocinnamate.

A second product, 1,2-bis-[2-(2-methoxycarbonylethyl)-4-[2-(2-methoxycarbonylethyl)-5-trimethylacetyloxybenzyl]-5-methoxyphenyl]ethane, is also isolated.

### Step 15: Preparation of methyl 5-[4-[4-[[2-(2-methoxycarbonylethyl)-5-trimethylacetyloxy]benzyl]-[2-(2-methoxycarbonylethyl)-5-methoxy]benzyl]-[2-(2-methoxycarbonylethyl)-5-methoxy]benzyl]-4-methoxy-2-hydroxymethylhydrocinnamate

Using essentially the procedure of Example 2, Step 7, and purifying the crude product by flash chromatography, eluting with 90% ether in methylene chloride, the desired product is prepared from methyl 5-[4-[4-[[2-(2-methoxycarbonylethyl)-5-trimethylacetyloxy]benzyl]-[2-(2-methoxycarbonylethyl)-5-methoxy]benzyl]-[2-(2-methoxycarbonylethyl)-5-methoxy]benzyl]-4-methoxy-2-tert-butyldimethylsilyloxymethylhydrocinnamate.

### Step 16: Preparation of methyl 5-[4-[4-[[2-(2-methoxycarbonylethyl)-5-trimethylacetyloxy]benzyl]-[2-(2-methoxycarbonylethyl)-5-methoxy]benzyl]-[2-(2-methoxycarbonylethyl)-5-methoxy]benzyl]-4-methoxy-2-bromomethylhydrocinnamate

Using essentially the procedure of Example 2, Step 8, and purifying the crude product by flash chromatography, eluting with ether/methylene choride/hexanes (4:1:5), the desired product is prepared from methyl 5-[4-[4-[[2-(2-methoxycarbonylethyl)-5-trimethylacetyloxy]benzyl]-[2-(2-methoxycarbonylethyl)-5-methoxy]benzyl]-[2-(2-methoxycarbonylethyl)-5-methoxy]benzyl]-4-methoxy-2-hydroxymethylhydrocinnamate.

### Step 17: Preparation of methyl 5-[4-[4-[[2-(2-methoxycarbonylethyl)-5-hydroxy-4-iodo]benzyl]-[2-(2-methoxycarbonylethyl)-5-methoxy]benzyl]-[2-(2-methoxycarbonylethyl)-5-methoxy]benzyl]-4-methoxy-2-tert-butyldimethylsilyloxymethylhydrocinnamate

Using essentially the procedure of Example 2, Step 5, and purifying the crude product by flash chromatography, eluting with ether/methylene chloride/hexanes (3:1:6), the desired product is prepared from methyl 5-[4-[4-[[2-(2-methoxycarbonylethyl)-5-trimethylacetyloxy]benzyl]-[2-(2-methoxycarbonylethyl)-5-methoxy]benzyl]-[2-(2-methoxycarbonylethyl)-5-methoxy]benzyl]-4-methoxy-2-tert-butyldimethylsilyloxymethylhydrocinnamate

### Step 18: Preparation of methyl 5-[4-[4-[[2-(2-methoxycarbonylethyl)-5-methoxy-4-iodo]benzyl]-[2-(2-methoxycarbonylethyl)-5-methoxy]benzyl]-[2-(2-methoxycarbonylethyl)-5-methoxy]benzyl]-4-methoxy-2-tert-butyldimethylsilyloxymethylhydrocinnamate

Using essentially the procedure of Example 2, Step 6, and purifying the crude product by flash chromatography, eluting with ether/methylene chloride/hexanes (3:1:6), the desired product is prepared from methyl 5-[4-[4-[[2-(2-methoxycarbonylethyl)-5-hydroxy-4-iodo]benzyl]-[2-(2-methoxycarbonylethyl)-5-methoxy]benzyl]-[2-(2-methoxycarbonylethyl)-5-methoxy]benzyl]-4-methoxy-2-tert-butyldimethylsilyloxymethylhydrocinnamate.

### Step 19: Preparation of 2-(2-methoxycarbonylethyl)-4-[4-[4-[4-[4-[4-[4-[[2-(2-methoxycarbonylethyl)-5-trimethylacetyl]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-5-methoxybenzyloxy-2-tert-butyldimethylsilane

Using essentially the same procedure of Example 2, Step 9, and purifying the crude product by flash chromatography, eluting with ether/methylene chloride/hexanes (2:1:2), the desired product is prepared from 5-[4-[4-[[2-(2-methoxycarbonylethyl)-5-methoxy-4-iodo]benzyl]-[2-(2-methoxycarbonylethyl)-5-methoxy]benzyl]-[2-(2-methoxycarbonylethyl)-5-methoxy]benzyl]-4-methoxy-2-tert-butyldimethylsilyloxymethylhydrocinnamate and methyl 5-[4-[4-[[2-(2-methoxycarbonylethyl)-5-trimethylacetyloxy]benzyl]-[2-(2-methoxycarbonylethyl)-5-methoxy]benzyl]-[2-(2-methoxycarbonylethyl)-5-methoxy]benzyl]-4-methoxy-2-bromomethylhydrocinnamate.

A second product, 1,2-bis-[4-[4-[4-[[2-(2-methoxycarbonylethyl)-5-trimethylacetyloxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]phenyl]ethane, is also isolated.

### Step 20: Preparation of methyl 5-[4-[4-[4-[4-[4-[4-[[2-(2-methoxycarbonylethyl)-5-trimethylacetyloxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-4-methoxy-2-hydroxymethylhydrocinnamate

Using essentially the procedure of Example 2, Step 7, purifying the crude product by flash chromatography, eluting with ether/methylene chloride/hexanes (3:1:1) then 80% ether in methylene chloride, the desired product is prepared from 2-(2-methoxycarbonylethyl)-4-[4-[4-[4-[4-[4-[4-[[2-(2-methoxycarbonylethyl)-5-trimethylacetyl]benzyl]-[2-(2-methoxycarbonylethyl)-5-methoxy]benzyl]-(2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-5-methoxybenzyloxy-tert-butyldimethylsilane.

### Step 21: Preparation of methyl 5-[4-[4-[4-[4-[4-[4-[[2-(2-methoxycarbonylethyl)-5-trimethylacetyloxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-4-methoxy-2-bromomethylhydrocinnamate

Using essentially the procedure of Example 2, Step 8, and purifying the crude product by flash chromatography, eluting with ether, methylene chloride, hexanes (5:2:3) the desired product is prepared from 5-[4-[4-[4-[4-[4-[4-[[2-(2-methoxycarbonylethyl)-5-trimethylacetyloxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-4-methoxy-2-hydroxymethylhydrocinnamate.

### Step 22: Preparation of methyl 5-[4-[4-[4-[4-[4-[4-[[2-(2-methoxycarbonylethyl)-5-hydroxy-4-iodo]benzyl]-[2-(2-methoxycarbonylethyl)-5-methoxy]benzyl]-[2-(2-methoxycarbonylethyl)-5-melhoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-4-methoxy-2-tert-butyldimethylsilyloxymelhylhydrocinnamale

Using essentially the procedure of Example 2, Step 5, and purifying the crude product by flash chromatography, eluting with ether/methylene chloride/hexanes (6:1:3), the desired product is prepared from 2-(2-methoxycarbonylethyl)-4-[4-[4-[4-[4-[4-[4-[[2-(2-methoxycarbonylethyl)-5-trimethylacetyl]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-5-methoxybenzyloxy-tert-butyldimethylsilane.

### Step 23: Preparation of methyl 5-[4-[4-[4-[4-[4-[4-[[2-(2-methoxycarbonylethyl)-5-methoxy-4-iodo]benzyl]-[2-(2-methoxycarbonylethyl)-5-methoxy]benzyl]-[2 (2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-4-methoxy-2-tert-butyldimethylsilyloxymethylhydrocinnamate

Using essentially the procedure of Example 2, Step 6, and purifying the crude product by flash chromatography, eluting with ether/methylene chloride/hexanes (5:2:3), the desired product is prepared from methyl 5-[4-[4-[4-[4-[4-[4-[[2-(2-methoxycarbonylethyl)-5-hydroxy-4-iodo]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-4-methoxy-2-tert-butyldimethylsilyloxymethylhydrocinnamate.

### Step 24: Preparation of methyl 5-[4-[4-[4-[4-[4-[4-[4-[4-[4-[4-[4-[4-[4-[4-[[2-(2-methoxycarbonylethyl)-5-trimethylacetyloxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-4-methoxy-2-tert-butyldimethylsilyloxymethylhydrocinnamate

Using essentially the procedure of Example 2, Step 9, and purifying the crude product by flash chromatography, eluting with ethyl acetate/methylene chloride/hexanes (3:2:5), and further purifying the product so obtained by flash chromatography, eluting with ethyl acetate/methylene chloride/hexanes (3:3:4), the desired product is obtained from methyl 5-[4-[4-[4-[4-[4-[4-[[2-(2-methoxycarbonylethyl)-5-methoxy-4-iodo]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-4-methoxy-2-tert-butyldimethylsilyloxymethylhydrocinnamate and methyl 5-[4-[4-[4-[4-[4-[4-[[2-(2-methoxycarbonylethyl)-5-trimethylacetyloxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-4-methoxy-2-bromomethylhydrocinnamate.

A second product, 1,2-bis-[4-[4-[4-[4-[4-[4-[4-[[2-(2-methoxycarbonylethyl)-5-trimethylacetyloxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]phenyl]ethane is also isolated.

### Step 25: Preparation of methyl 5-[4-[4-[4-[4-[4-[4-[4-[4-[4-[4-[4-[4-[4-[4-[[2-(2-methoxycarbonylethyl)-5-trimethylacetyloxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-4-methoxy-2-methylhydrocinnamate

To a solution of the silylether product from Example 2, Step 24 (0.253g) and acetic acid (1 ml) in THF (4 ml) is added palladium hydroxide (0.15g) and the resulting suspension stirred under an atmosphere of hydrogen at 40°C for about 48 hours. The hydrogen atmosphere is replaced by nitrogen and the mixture diluted with methylene chloride. The catalyst is removed by filtration and the filtrate concentrated in vacuo to give the desired product.

### Step 26: Preparation of 5-[4-[4-[4-[4-[4-[4-[4-[4-[4-[4-[4-[4-[4-[4-[[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-4-hydroxy-2-methylhydrocinnamic acid

To a solution of the product from Example 2, Step 25 (0.236g) in methylene chloride (0.5 ml) is added 1M boron tribromide in methylene chloride (4 ml). The solution is stirred at room temperature for about 60 hours, methanol (1 ml) is added dropwise, and the resulting solution poured into 10% methanol in ethyl acetate (50 ml). The solution is washed with water, brine, dried over magnesium sulfate, filtered, and concentrated in vacuo. Toluene is azeotroped from the residue and this residue is taken up in THF (1 ml) and methanol (1 ml). To this solution is added 30% aqueous sodium hydroxide (1 ml) and the solution stirred at room temperature for about 48 hours. The mixture is adjusted to pH 1 with 2N HCl, then extracted with 10% methanol in ethyl acetate. The organic layer is washed with brine, dried over magnesium sulfate, concentrated in vacuo, and the residue dissolved in concentrated aqueous ammonia solution. The solution is lyophilized to give the desired product as the ammonium salt.

### Example 3

### Preparation of 1,2-bis-[2-(2-carboxyethyl)-4-[2-(2-carboxyethyl)-5-hydroxybenzyl]-5-hydroxyphenyl]ethane

Using essentially the procedure of Example 2, Step 26, the desired product is prepared from 1,2-bis-[2-(2-methoxycarbonylethyl)-4-[2-(2-methoxycarbonylethyl)-5-trimethylacetyloxybenzyl]-5-methoxyphenyl]ethane, and isolated as the ammonium salt, m.p. 211-214°C.

### Example 4

### Preparation of 5-[4-[4-[4-[4-[4-[4-[[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-4-hydroxy-2-methylhydrocinnamic acid

Using essentially the procedures of Example 2, Steps 25 and 26, the desired product is prepared from 2-(2-methoxycarbonylethyl)-4-[4-[4-[4-[4-[4-[4-[[2-(2-methoxycarbonylethyl)-5-trimethylacetyl]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-5-methoxybenzyloxy-tert-butyldimethylsilane, and isolated as the ammonium salt.

### Example 5

### Preparation of 5-[4-[4-[4-[4-[4-[4-[[2-(2-carboxyethyl)-5-hydroxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-4-methoxy-2-methylhydrocinnamic acid

### Step 1: Preparation of 5-[4-[4-[4-[4-[4-[4-[[2-(2-carboxyethyl)-5-trimethylacetyloxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-4-methoxy-2-methylhydrocinnamic acid

Using essentially the procedure of Example 2, Step 25, the desired product is prepared from 2-(2-methoxycarbonylethyl)-4-[4-[4-[4-[4-[4-[4-[[2-(2-methoxycarbonylethyl)-5-trimethylacetyl]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-5-methoxybenzyloxy-tert-butyldimethylsilane.

### Step 2: Preparation of 5-[4-[4-[4-[4-[4-[4-[[2-(2-carboxyethyl)-5-hydroxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-4-methoxy-2-methylhydrocinnamic acid

To a solution of the product from Example 5, Step 1 (0.18g) in THF (1 ml) and methanol (1 ml) is added 30% aqueous sodium hydroxide (1 ml) and the resulting is stirred at room temperature for about 40 hours. The mixture is adjusted to pH 1 with 2N HCI and the solid collected by centrifugation, then washed repeatedly with water until the pH is >5.5. The solid is then dissolved in 30% aqueous ammonia and concentrated in vacuo at about 45°C to give the desired product as the ammonium salt.

### Example 6

### Preparation of 1,2-bis-[4-[4-[4-[[2-(2-carboxyethyl)-5-hydroxy]benzyl]-[2-(2-carboxyethyl)-5-hydroxy]benzyl]-(2-(2-carboxyethyl)-5-hydroxy]benzyl]-[2-(2-carboxyethyl)-5-hydroxy]phenyl]ethane

Using essentially the procedure of Example 2, Step 26, the desired product is prepared from 1,2-bis-[4-[4-[4-[[2-(2-methoxycarbonylethyl)-5-trimethylacetyloxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]phenyl]ethane.

### Example 7

### Preparation of 1,2-bis-[4-[4-[4-[[2-(2-carboxyethyl)-5-hydroxy]benzyl]-[2-(2-carboxyethyl)-5-methoxy]benzyl]-[2-(2-carboxyethyl)-5-methoxy]benzyl]-[2-(2-carboxyethyl)-5-methoxy]phenyl]ethane

Using essentially the procedure of Example 5, Step 2, the desired product is prepared, as the ammonium salt, from 1,2-bis-[4-[4-[4-[[2-(2-methoxycarbonylethyl)-5-trimethylacetyloxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]phenyl]ethane.

### Example 8

### Preparation of 5-[4-[4-[4-[4-[4-[4-[4-[4-[4-[4-[4-[4-[4-[4-[[2-(2-carboxyethyl)-5-hydroxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-4-hydroxy-2-methylhydrocinnamic acid

Using essentially the procedure of Example 5, Step 2, the desired product is prepared, and isolated as the ammonium salt, from the ester product of Example 2, Step 25.

### Example 9

### Preparation of 1,2-bis-[4-[4-[4-[4-[4-[4-[4-[[2-(2-carboxyethyl)-5-hydroxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]phenyl]ethane

Using essentially the procedure of Example 5, Step 2, the desired product is prepared, and isolated as the ammonium salt, from 1,2-bis-[4-[4-[4-[4-[4-[4-[4-[[2-(2-methoxycarbonylethyl)-5-trimethylacetyloxy]benzyl]-[2-(2-methoxycarbonylethyl)-5-methoxylbenzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]phenyl]ethane.

### Example 10

The following abbreviation is used in this example:
MMPM represents [5-(2-methoxycarbonylethyl)-2-methoxy]phenoxymethyl] Preparation of methyl-5-acetoxy-2-[4-[4-[4-[4-[4-[4-[4-[4-[4-[4-[4-[4-[4-[4-[[5-(2-methoxycarbonylethyl)-4-hydroxymethyl-2-methoxy]phenoxymethyl]-MMPM-MMPM-MMPM-MMPM-MMPM-MMPM-MMPM-MMPM-MMPM-MMPM-MMPM-MMPM-MMPM-MMPM-4-methoxyhydrocinnamate

### Step 1: Preparation of 4-acetoxy-3-methoxybenzaldehyde

A solution of vanillin (60.8g), triethylamine (60 ml), acetic anhydride (44 ml), and 4-dimethylaminopyridine (1g) in methylene chloride (800 ml) is stirred at room temperature for about 30 minues, and the volume then reduced by 50% in vacuo. The residue is diluted with ether and the solution washed with water, brine, dried over magnesium sulfate, filtered and concentrated in vacuo to give the desired product.

### Step 2: Preparation of 4-acetoxy-6-bromo-3-methoxybenzaldehyde

To a mixture of 4-acetoxy-3-methoxybenzaldehyde (75.7g), anhydrous sodium acetate (76g) and iodine (0.8g) in acetic acid (300 ml) is added bromine (21 ml). The mixture is warmed to 45°C for about 1 hour, then stirred stirred at room temperature for 3 days, poured into water (700 ml) and filtered. The solid is dissolved in toluene and the solution washed with brine, dried over magnesium sulfate, filtered and concentrated in vacuo. The residue is recrystallized twice from hexanes to give the desired product.

### Step 3: Preparation of methyl 5-acetoxy-2-formyl-4-methoxycinnamate

To a degassed solution of 4-acetoxy-6-bromo-3-methoxybenzaldehyde (15.4g), and bis(triphenylphosphine)palladium (II) (1.2g) in DMF (150 ml) is added triethylamine (31.9 ml) and methyl acrylate (19.6g). The mixture is heated 135°C for about 1.5 hours, cooled to room temperature and diluted with ether and water. The organic layer is washed with water, brine, dried over magnesium sulfate, filtered and concentrated in vacuo. The residue is purified by HPLC, eluting with 33% ethyl acetate in hexanes, then methylene chloride, to give the desired product.

### Step 4: Preparation of methyl 5-acetoxy-2-fomyl-4-methoxyhydrocinnamate

A mixture of methyl 5-acetoxy-2-formyl-4-methoxycinnamate (10.8g) and 10% palladium on carbon (3g) in ethyl acetate (100 ml) is hydrogenated at 45 psi for about 18 hours and filtered. The filtrate is concentrated in vacuo and the residue purified by HPLC, eluting 50% ethyl acetate in hexanes, to give the desired product.

### Step 5: Preparation of methyl 5-acetoxy-2-tert-butyldimethylsilyloxymethyl-4-methoxyhydrocinnamate

To a solution of methyl 5-acetoxy-2-formyl-4-methoxyhydrocinnamate (3.31g) and imidazole (2.42g) in methylene chloride (25 ml) is added a solution of tert-butylchlorodimethylsilane (2.73g) in methylene chloride (25 ml). The mixture is stirred at room temperature for about 60 minutes, diluted with ether and water, and the organic layer washed with water, brine, dried over magnesium sulfate, filtered and concentrated in vacuo to give the desired product.

### Step 6: Preparation of methyl 5-hydroxy-2-tert-butyldimethylsilyloxymethyl-4-methoxyhydrocinnamate

A mixture of methyl 5-acetoxy-2-tert-butyldimethylsilyloxymethyl-4-methoxyhydrocinnamate (4.64g) and potassium carbonate (0.16g) in anhydrous methanol (50 ml) is stirred at 0-5°C for about 18 hours, diluted with water, and the pH adjusted to 5 with dilute HCI. The mixture is extracted with ether and the ether solution is washed with brine, dried over magnesium sulfate, filtered and concentrated in vacuo. The residue is purified by HPLC, eluting with 20% ethyl acetate in hexanes to give the desired product.

### Step 7: Preparation of methyl 5-acetoxy-2-bromomethyl-4-methoxyhydrocinnamate

To a solution of methyl 5-hydroxy-2-tert-butyldimethylsilyloxymethyl-4-methoxyhydrocinnamate (5.21g) in carbon tetrabromide (12.35g) in anhydrous ether (75 ml) and anhydrous THF (50 ml) is added portionwise triphenylphosphine (9.78g) and the mixture stirred at room temperature for 60 minutes, diluted with ether, then filtered. The filtrate is concentrated in vacuo and the residue purified by filtration through silica gel in 50% ethyl acetate in hexanes followed by HPLC, eluting with 25% ethyl acetate in hexanes, to give the desired product.

### Step 8: Preparation of methyl 5-acetoxy-2-[4-tert-butyldimethylsilyloxymethyl-2-methoxy-5-(2-methoxycarbonylethyl)]phenoxymethyl-4-methoxyhydrocinnamate

To a suspension of pentane-washed sodium hydride (60% in mineral oil, 0.575g) in anhydrous DMF (5 ml) is added dropwise a solution of methyl 5-hydroxy-2-tert-butyldimethylsilyloxymethyl-4-methoxyhydrocinnamate (4.84g) in DMF (20 ml). The solution is stirred at room temperature for 15 minutes and a solution of methyl 5-acetoxy-2-bromomethyl-4-methoxyhydrocinnamate (5.20g) in DMF (25 ml) is added. After stirring at room temperature for 45 minutes the mixtured is quenched with water and ether. The organic layer is washed with water, brine, dried over magnesium sulfate, filtered and concentrated in vacuo. The residue is purified by HPLC, eluting with 25% ethyl acetate in hexanes, to give the desired product.

### Step 9: Preparation of methyl 5-acetoxy-2-[4-hydroxymethyl-2-methoxy-5-(2-methoxycarbonylethyl)]phenoxymethyl-4-methoxyhydrocinnamate

To a solution of methyl 5-acetoxy-2-[4-tert-butyldimethylsilyloxymethyl-2-methoxy-5-(2-methoxycarbonylethyl)]phenoxymethyl-4-methoxyhydrocinnamate (2.51g) and acetic acid (0.7 ml) in anhydrous THF (20 ml) is added tetrabutylammonium fluoride (6.1 ml of a 1N solution in THF). The solution is stirred at room temperature for about 60 minutes, diluted with ether and the organic solution washed with water, brine, dried over magnesium sulfate, filtered and concentrated in vacuo. The residue is purified by HPLC, eluting with 40% hexanes in ethyl acetate.

### Step 10: Preparation of methyl 5-acetoxy-2-[4-bromomethyl-2-methoxy-5-(2-methoxycarbonylethyl)]phenoxymethyl-4-methoxyhydrocinnamate

Using essentially the procedure of Example 10, Step 7, and purifying the crude product by flash chromatography, eluting with 33% ethyl acetate in hexanes, the desired is prepared from methyl 5-acetoxy-2-[4-hydroxymethyl-2-methoxy-5-(2-methoxycarbonylethyl)]phenoxymethyl-4-methoxyhydrocinnamate.

### Step 11: Preparation of methyl 5-hydroxy-2-[4-tert-butyldimethylsilyloxymethyl-2-methoxy-5-(2-methoxycarbonylethyl)]phenoxymethyl-4-methoxyhydrocinnamate

A solution of methyl 5-acetoxy-2-[4-tert-butyldimethylsilyloxymethyl-2-methoxy-5-(2-methoxycarbonylethyl)]phenoxymethyl-4-methoxyhydrocinnamate (2.57g) and sodium methoxide (4.2 ml of a 1N solution in methanol) in THF (25 ml) is kept at -78°C for about 18 hours, then quenched with acetic acid (0.50 ml) and diluted with ethyl acetate. The solution is washed with water, brine, dried over magnesium sulfate, filtered and concentrated in vacuo. The residue is purified by flash chromatography, eluting with 30% ethyl acetate in hexane, to give the desired product.

### Step 12: Preparation of methyl-5-acetoxy-2-[4-[4-[[5-(2-methoxycarbonylethyl)-4-tert-butyldimethylsilyloxymethyl-2-methoxy]phenoxymethyl]-MMPM-MMPM-4-methoxyhydrocinnamate

A mixture of sodium hydride (0.6g of a 60% suspension in mineral oil) in DMF (2 ml) is cooled to -15°C and a solution of methyl 5-hydroxy-2-[4-tert-butyldimethylsilyloxymethyl-2-methoxy-5-(2-methoxycarbonylethyl)]phenoxymethyl-4-methoxyhydrocinnamate (0.803g) in DMF (8 ml) is added. The mixture is stirred for about 30 minutes and a solution of methyl 5-acetoxy-2-[4-bromomethyl-2-methoxy-5-(2-methoxycarbonylethyl)]phenoxymethyl-4-methoxyhydrocinnamate (0.79g) in DMF (10 ml) is added. The mixture is warmed slowly to room temperature, stirred for 2 hours, and diluted with ether and water. The aqueous layer is extracted with ether and ethyl acetate and the combined organic layers washed with water, brine, dried over magnesium sulfate, filtered and concentrated in vacuo. The residue was purified by flash chromatography, eluting with 40% ethyl acetate in hexanes, to give the desired product.

### Step 13: Preparation of methyl-5-acetoxy-2-[4-[4-[[5-(2-methoxycarbonylethyl)-4-hydroxymethyl-2-methoxy]phenoxymethyl]-MMPM-MMPM-4-methoxyhydrocinnamate

Using essentially the procedure of Example 10, Step 9, the desired product, m.p. 126-127°C, is prepared from methyl-5-acetoxy-2-[4-[4-[[5-(2-methoxycarbonylethyl)-4-tert-butyldimethylsilyloxymethyl-2-methoxy]phenoxymethyl]-MMPM-MMPM-4-methoxyhydrocinnamate.

### Step 14: Preparation of methyl-5-acetoxy-2-[4-[4-[[5-(2-methoxycarbonylethyl)-4-bromomethyl-2-methoxy]phenoxymethyl]-MMPM-MMPM-4-methoxyhydrocinnamate

Using essentially the procedure of Example 10, Step 7, the desired product is prepared from methyl-5-acetoxy-2-[4-[4-[[5-(2-methoxycarbonylethyl)-4-hydroxymethyl-2-methoxy]phenoxymethyl]-MMPM-MMPM-4-methoxyhydrocinnamate.

### Step 15: Preparation of methyl-5-hydroxy-2-[4-[4-[[5-(2-methoxycarbonylethyl)-4-tert-butyldimethylsilyloxymethyl-2-methoxy]phenoxymethyl]-MMPM-MMPM-4-methoxyhydrocinnamate

Using essentially the procedure of Example, Step 11, the desired product is prepared from methyl-5-acetoxy-2-[4-[4-[[5-(2-methoxycarbonylethyl)-4-tert-butyldimethylsilyloxymethyl-2-methoxy]phenoxymethyl]-MMPM-MMPM-4-methoxyhydrocinnamate.

### Step 16: Preparation of methyl-5-acetoxy-2-[4-[4-[4-[4-[4-[4-[[5-(2-methoxycarbonylethyl)-4-tert-butyldimethylsilyloxymethyl-2-methoxy]phenoxymethyl]-MMPM-MMPM-MMPM-MMPM-MMPM-MMPM-4-methoxyhydrocinnamate

Using essentially the procedure of Example 10, Step 12, the desired is prepared from methyl-5-hydroxy-2-[4-[4-[[5-(2-methoxycarbonylethyl)-4-tert-butyldimethylsilyloxymethyl-2-methoxy]phenoxymethyl]-MMPM-MMPM-4-methoxyhydrocinnamate and methyl-5-acetoxy-2-[4-[4-[[5-(2-methoxycarbonylethyl)-4-bromomethyl-2-methoxy]phenoxymethyl]-MMPM-MMPM-4-methoxyhydrocinnamate.

### Step 17: Preparation of methyl-5-acetoxy-2-[4-[4-[4-[4-[4-[4-[[5-(2-methoxycarbonylethyl)-4-hydroxymethyl-2-methoxy]phenoxymethyl]-MMPM-MMPM-MMPM-MMPM-MMPM-MMPM-4-methoxyhydrocinnamate

Using essentially the procedure of Example 10, Step 9, The desired product is prepared from methyl-5-acetoxy-2-[4-[4-[4-[4-[4-[4-[[5-(2-methoxycarbonylethyl)-4-tert-butyldimethylsilyloxymethyl-2-methoxy]phenoxymethyl]-MMPM-MMPM-MMPM-MMPM-MMPM-MMPM-4-methoxyhydrocinnamate.

### Step 18: Preparation of methyl-5-acetoxy-2-[4-[4-[4-[4-[4-[4-[[5-(2-methoxycarbonylethyl)-4-bromomethyl-2-methoxy]phenoxymethyl]-MMPM-MMPM-MMPM-MMPM-MMPM-MMPM-4-methoxyhydrocinnamate

Using essentially the procedure of Example 10, Step 7, the desired product is prepared from methyl-5-acetoxy-2-[4-[4-[4-[4-[4-[4-[[5-(2-methoxycarbonylethyl)-4-hydroxymethyl-2-methoxy]phenoxymethyl]-MMPM-MMPM-MMPM-MMPM-MMPM-MMPM-4-methoxyhydrocinnamate.

### Step 19: Preparation of methyl-5-hydroxy-2-[4-[4-[4-[4-[4-[4-[[5-(2-methoxycarbonylethyl)-4-tert-butyldimethylsilyloxymethyl-2-methoxy]phenoxymethyl]-MMPM-MMPM-MMPM-MMPM-MMPM-MMPM-4-methoxyhydrocinnamate

Using essentially the procedure of Example 10, Step 11, the desired product is prepared from methyl-5-acetoxy-2-[4-[4-[4-[4-[4-[4-[[5-(2-methoxycarbonylethyl)-4-tert-butyldimethylsilyloxymethyl-2-methoxy]phenoxymethyl]-MMPM-MMPM-MMPM-MMPM-MMPM-MMPM-4-methoxyhydrocinnamate.

### Step 20: Preparation of methyl-5-acetoxy-2-[4-[4-[4-[4-[4-[4-[4-[4-[4-[4-[4-[4-[4-[4-[[5-(2-methoxycarbonylethyl)-4-tert-butyldimethylsilyloxymethyl-2-methoxy]phenoxymethyl]-MMPM-MMPM-MMPM-MMPM-MMPM-MMPM-MMPM-MMPM-MMPM-MMPM-MMPM-MMPM-MMPM-MMPM-4-methoxyhydrocinnamate

Using essentially the procedure of Example 10, Step 12, the desired compound is prepared from methyl-5-acetoxy-2-[4-[4-[4-[4-[4-[4-[[5-(2-methoxycarbonylethyl)-4-bromomethyl-2-methoxy]phenoxymethyl]-MMPM-MMPM-MMPM-MMPM-MMPM-MMPM-4-methoxyhydrocinnamate and methyl-5-acetoxy-2-[4-[4-[4-[4-[4-[4-[[5-(2-methoxycarbonylethyl)-4-tert-butyldimethylsilyloxymethyl-2-methoxy]phenoxymethyl]-MMPM-MMPM-MMPM-MMPM-MMPM-MMPM-4-methoxyhydrocinnamate.

### Step 21: Preparation of methyl-5-acetoxy-2-[4-[4-[4-[4-[4-[4-[4-[4-[4-[4-[4-[4-[4-[4-[[5-(2-methoxycarbonylethyl)-4-hydroxymethyl-2-methoxy]phenoxymethyl]-MMPM-MMPM-MMPM-MMPM-MMPM-MMPM-MMPM-MMPM-MMPM-MMPM-MMPM-MMPM-MMPM-MMPM-4-methoxyhydrocinnamate

Using essentially the procedure of Example 10, Step 9, the desired product is prepared from methyl-5-acetoxy-2-[4-[4-[4-[4-[4-[4-[4-[4-[4-[4-[4-[4-[4-[4-[[5-(2-methoxycarbonylethyl)-4-tert-butyldimethylsilyloxymethyl-2-methoxy]phenoxymethyl]-MMPM-MMPM-MMPM-MMPM-MMPM-MMPM-MMPM-MMPM-MMPM-MMPM-MMPM-MMPM-MMPM-MMPM-4-methoxyhydrocinnamate.

### Example 11

The following abbreviations are used in this example:
MMPM represents [5-(2-methoxycarbonylethyl)-2-methoxy]phenoxymethyl]
BIPM represents [2-benzyloxy-5-isobutyl]phenoxymethyl]

### Preparation of methyl-5-acetoxy-2-[4-[4-[4-[4-[4-[4-[4-[4-[4-[4-[4-[4-[4-[[5-(2-methoxycarbonylethyl)-4-hydroxymethyl-2-methoxy]phenoxymethyl]-BIPM-MMPM-MMPM-BIPM-MMPM-MMPM-BIPM-MMPM-MMPM-BIPM-MMPM-MMPM-BIPM-4-methoxyhydrocinnamate

### Step 1: Preparation of methyl-5-acetoxy-2-[4-[[4-tert-butyldimethylsilyloxymethyl-5-(2-methoxycarbonylethyl)-2-methoxy]phenoxymethyl]-BIPM-4-methoxyhydrocinnamate

Using essentially the procedure of Example 10, Step 8, the desired product is prepared from methyl 5-acetoxy-2-bromomethyl-4-methoxyhydrocinnamate and methyl 5-[(5-benzyloxy-2-isobutyl-4-hydroxy)benzyloxy]-2-tert-butyldimethylsiloxymethyl-4-methoxyhydrocinnamate.

### Step 2: Preparation of methyl-5-acetoxy-2-[4-[[4-bromomethyl-5-(2-methoxycarbonylethyl)-2-methoxy]phenoxymethyl]-BIPM-4-methoxyhydrocinnamate

Using essentially the procedures of Example 10, Steps 9 and 10, the desired product is prepared from methyl-5-acetoxy-2-[4-[[4-tert-butyldimethylsilyloxymethyl-5-(2-methoxycarbonylethyl)-2-methoxy]phenoxymethyl]-BIPM-4-methoxyhydrocinnamate.

### Step 3: Preparation of methyl-5-hydroxy-2-[4-[[4-tert-butyldimethylsilyloxymethyl-5-(2-methoxycarbonylethyl)-2-methoxy]phenoxymethyl]-BIPM-4-methoxyhydrocinnamate

Using essentially the procedure of Example 10, Step 11, the desired product is prepared from methyl-5-acetoxy-2-[4-[[4-tert-butyldimethylsilyloxymethyl-5-(2-methoxycarbonylethyl)-2-methoxy]phenoxymethyl]-BIPM-4-methoxyhydrocinnamate.

### Step 4: Preparation of methyl-5-acetoxy-2-[4-[4-[4-[4-[[4-tert-butyldimethylsilyloxymethyl-5-(2-methoxycarbonylethyl)-2-methoxy]phenoxymethyl]-BIPM-MMPM-MMPM-BIPM-4-methoxyhydrocinnamate

Using essentially the procedure of Example 10, Step 12, the desired product is prepared from methyl-5-acetoxy-2-[4-[[4-bromomethyl-5-(2-methoxycarbonylethyl)-2-methoxy]phenoxymethyl]-BIPM-4-methoxyhydrocinnamate and methyl-5-hydroxy-2-[4-[[4-tert-butyldimethylsilyloxymethyl-5-(2-methoxycarbonylethyl)-2-methoxy]phenoxymethyl]-BIPM-4-methoxyhydrocinnamate.

### Step 5: Preparation of methyl-5-acetoxy-2-[4-[4-[4-[4-[4-[4-[4-[4-[4-[4-[[4-tert-bulyldimethylsilyloxymethyl-5-(2-methoxycarbonylethyl)-2-methoxy]phenoxymethyl]-BIPM-MMPM-MMPM-BIPM-MMPM-MMPM-BIPM-MMPM-MMPM-BIPM-4-methoxyhydrocinnamate

Using essentially the procedures of Example 11, Steps 2, 3, 4, and 5, the desired product is prepared from methyl-5-acetoxy-2-[4-[4-[4-[4-[[4-tert-butyldimethylsilyloxymethyl-5-(2-methoxycarbonylethyl)-2-methoxy]phenoxymethyl]-BIPM-MMPM-MMPM-BIPM-4-methoxyhydrocinnamate.

### Step 6: Preparation of methyl-5-hydroxy-2-[4-[4-[4-[4-[4-[4-[4-[4-[4-[4-[[4-tert-butyldimethylsilyloxymethyl-5-(2-methoxycarbonylethyl)-2-methoxy]phenoxymethyl]-BIPM-MMPM-MMPM-BIPM-MMPM-MMPM-BIPM-MMPM-MMPM-BIPM-4-methoxyhydrocinnamate

Using essentially the procedure of Example 10, Step 11, the desired product is prepared from methyl-5-acetoxy-2-[4-[4-[4-[4-[4-(4-[4-[4-[4-[4-[(4-tert-butyldimethylsilyloxymethyl-5-(2-methoxycarbonylethyl)-2-methoxy]phenoxymethyl]-BIPM-MMPM-MMPM-BIPM-MMPM-MMPM-BIPM-MMPM-MMPM-BIPM-4-methoxyhydrocinnamate.

### Step 6: Preparation of methyl-5-acetoxy-2-[4-[4-[4-[4-[4-[4-[4-[4-[4-[4-[4-[4-[4-[[5-(2-methoxycarbonylethyl)-4-tert-butyldimethylsilyloxymethyl-2-methoxy]phenoxymethyl]-BIPM-MMPM-MMPM-BIPM-MMPM-MMPM-BIPM-MMPM-MMPM-BIPM-MMPM-MMPM-BIPM-4-methoxyhydrocinnamate

Using essentially the procedure of Example 10, Step 12, the desired product is prepared from methyl-5-hydroxy-2-[4-[4-[4-[4-[4-[4-[4-[4-[4-[4-[[4-tert-butyldimethylsilyloxymethyl-5-(2-methoxycarbonylethyl)-2-methoxy]phenoxymethyl]-BIPM-MMPM-MMPM-BIPM-MMPM-MMPM-BIPM-MMPM-MMPM-BIPM-4-methoxyhydrocinnamate and methyl-5-acetoxy-2-[4-[[4-bromomethyl-5-(2-methoxycarbonylethyl)-2-methoxy]phenoxymethyl]-BIPM-4-methoxyhydrocinnamate.

### Step 7: Preparation of methyl-5-acetoxy-2-[4-[4-[4-[4-[4-[4-[4-[4-[4-[4-[4-[4-[4-[[5-(2-methoxycarbonylethyl)-4-hydroxymethyl-2-methoxy]phenoxymethyl]-BIPM-MMPM-MMPM-BIPM-MMPM-MMPM-BIPM-MMPM-MMPM-BIPM-MMPM-MMPM-BIPM-4-methoxyhydrocinnamate

Using essentially the procedure of Example 10, Step 9, the desired product is prepared from methyl-5-acetoxy-2-[4-[4-[4-[4-[4-[4-[4-[4-[4-[4-[4-[4-[4-[[5-(2-methoxycarbonylethyl)-4-tert-butyldimethylsilyloxymethyl-2-methoxy]phenoxymethyl]-BIPM-MMPM-MMPM-BIPM-MMPM-MMPM-BIPM-MMPM-MMPM-BIPM-MMPM-MMPM-BIPM-4-methoxyhydrocinnamate.

### Example 12

### Preparation of 2-[4-[4-[4-[[5-(2-carboxyethyl)-2-methoxy]phenoxymethyl]-[5-(2-carboxyethyl)-2-methoxy]phenoxymethyl]-[5-(2-carboxyethyl)-2-methoxy]phenoxymethyl]-[5-(2-carboxyethyl)-2-methoxy]phenoxymethyl]-4-methoxyhydrocinnamic acid

### Step 1: Preparation of 2-bromo-5-methoxybenzaldehyde

To a solution of m-anisaldehyde (25.2g) and anhydrous sodium acetate (37.6g) in acetic acid (150 ml) is added bromine (30.8g) and the resulting solution stirred 45°C overnight, then at room temperature for about 4 hours. The reaction mixture is poured into water (300 ml), stirred for 15 minutes, then filtered and the solid residue dissolved in toluene. The toluene solution is washed with brine, dried over magnesium sulfate, filtered and concentrated in vacuo. The residue is recrystallized from hexanes to give the desired product.

### Step 2: Preparation of methyl 2-formyl-4-methoxycinnamate

Using essentially the procedure of Example 10, Step 3, and purifying the crude product by recrystallization from hexanes, the desired product is prepared from 2-bromo-5-methoxybenzaldehyde.

### Step 3: Preparation of methyl 2-hydroxymethyl-4-methoxyhydrocinnamate

A mixture of methyl 2-formyl-4-methoxycinnamate (15.9g) and 10% palladium on carbon (2g) in ethyl acetate (200 ml) is shaken under a hydrogen pressure of 45 psi for about 18 hours. The mixture is filtered, concentrated in vacuo, and the residue purified by HPLC, eluting with hexanes/ethyl acetate (3:2), to give the desired product.

### Step 4: Preparation of methyl 2-bromomethyl-4-methoxyhydrocinnamate

Using essentially the procedure of Example 10, Step 7, and purifying the crude product by HPLC, eluting with 10% ethyl acetate in hexanes, the desired product is prepared from methyl 2-hydroxymethyl-4-methoxyhydrocinnamate.

### Step 5: Preparation of methyl-2-[4-[[5-(2-methoxycarbonylethyl)-4-tert-butyldimethylsilyloxymethyl-2-methoxy]phenoxymethyl]-[5-(2-methoxycarbonylethyl)-2-methoxy]phenoxymethyl]-4-methoxyhydrocinnamate

Using essentially the procedure of Example 10, Step 8, and purifying the crude product by flash chromatography, eluting with hexanes/ethyl acetate (2:1), the desired product is prepared from methyl 5-hydroxy-2-[4-tert-butyldimethylsilyloxymethyl-2-methoxy-5-(2-methoxycarbonylethyl)]phenoxymethyl-4-methoxyhydrocinnamate and methyl 2-bromomethyl-4-methoxyhydrocinnamate.

### Step 6: Preparation of methyl-2-[4-[[5-(2-methoxycarbonylethyl)-4-hydroxymethyl-2-methoxy]phenoxymethyl]-(5-(2-methoxycarbonylethyl)-2-methoxy]phenoxymethyl]-4-methoxyhydrocinnamate

Using essentially the procedure of Example 10, Step 9, the desired product is prepared from methyl-2-[4-[[5-(2-methoxycarbonylethyl)-4-tert-butyldimethylsilyloxymethyl-2-methoxy]phenoxymethyl]-[5-(2-methoxycarbonylethyl)-2-methoxy]phenoxymethyl]-4-methoxyhydrocinnamate.

### Step 7: Preparation of methyl-2-bromomethyl-4-methoxy-5-[2-(2-methoxycarbonylethyl)-5-methoxybenzyloxy]hydrocinnamate

To a solution of methyl-2-[4-[[5-(2-methoxycarbonylethyl)-4-hydroxymethyl-2-methoxy]phenoxymethyl]-[5-(2-methoxycarbonylethyl)-2-methoxy]phenoxymethyl]-4-methoxyhydrocinnamate (0.282g) in chloroform (5 ml) is added dropwise bromotrimethylsilane (0.167 ml) and the solution stirred at room temperature for 5 minutes, then diluted with ethyl acetate and saturated sodium bicarbonate solution. The organic layer is washed with water, brine, dried over magnesium sulfate, filtered and concentrated in vacuo. The residue is purified by flash chromatography, eluting with hexanes/ethyl acetate (3:1) to give the desired product.

### Step 8: Preparation of methyl-5-acetoxy-2-[4-[[5-(2-methoxycarbonylethyl)-2-methoxy]phenoxymethyl]-[5-(2-methoxycarbonylethyl)-2-methoxy]phenoxymethyl]-4-methoxyhydrocinnamate

Using essentially the procedure of Example 10, Step 8, and purifying the crude product by flash chromatography, eluting with hexanes/ethyl acetate (1:1), the desired product is prepared from methyl 5-acetoxy-2-[4-bromomethyl-2-methoxy-5-(2-methoxycarbonylethyl)]phenoxymethyl-4-methoxyhydrocinnamate and methyl-3-hydroxy-4-methoxyhydroxycinnamate.

### Step 9: Preparation of methyl-5-hydroxy-2-[4-[[5-(2-methoxycarbonylethyl)-2-methoxy]phenoxymethyl]-[5-(2-methoxycarbonylethyl)-2-methoxy]phenoxymethyl]-4-methoxyhydrocinnamate

A solution of methyl-5-acetoxy-2-[4-[[5-(2-methoxycarbonylethyl)-2-methoxy]phenoxymethyl]-[5-(2-methoxycarbonylethyl)-2-methoxy]phenoxymethyl]-4-methoxyhydrocinnamate (0.197g) in THF (3 ml) is cooled to -78°C, and a 1M solution of sodium methoxide in methanol (0.34 ml) is added dropwise. The resulting solution is stirred at -78°C for about 20 hours, then quenched with acetic acid (0.2 ml), and diluted with water and ethyl acetate. The organic layer is washed with water, brine, dried over magnesium sulfate, filtered and concentrated in vacuo to give the desired product.

### Step 10: Preparation of methyl 2-[4-[4-[4-[[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-4-methoxyhydrocinnamate

Using essentially the procedure of Example 10, Step 8, and purifying the crude by flash chromatography, eluting with hexanes/ethyl acetate (3:2), the desired product is prepared from methyl-5-hydroxy-2-[4-[[5-(2-methoxycarbonylethyl)-2-methoxy]phenoxymethyl]-[5-(2-methoxycarbonylethyl)-2-methoxy]phenoxymethyl]-4-methoxyhydrocinnamate and methyl-2-bromomethyl-4-methoxy-5-[2-(2-methoxycarbonylethyl)-5-methoxybenzyloxy]hydrocinnamate.

### Step 11: Preparation of 2-[4-[4-[4-[[5-(2-carboxyethyl)-2-methoxy]phenoxymethyl]-[5-(2-carboxyethyl)-2-methoxy]phenoxymethyl]-[5-(2-carboxyethyl)-2-methoxy]phenoxymethyl]-[5-(2-carboxyethyl)-2-methoxy]phenoxymethyl]-4-methoxyhydrocinnamic acid

To a solution of methyl 2-[4-[4-[4-[[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-4-methoxyhydrocinnamate (0.145g) in methanol (1.5 ml) is added 1N sodium hydroxide solution (1.5 ml). The mixture is stirred at room temperature for about 2 hours and THF (2 ml) is added. This mixture is stirred at room temperature for about 18 hours and concentrated in vacuo to about one-half volume. This mixture is cooled to 0°C and the pH adjusted to 4 with 10% HCI, then filtered. The solid residue is washed with water, dried, and dissolved in 10% aqueous ammonia and this solution concentrated in vacuo to give the desired product as the ammonium salt, m.p. 199-201 °C.

### Example 13

### Preparation of polymer of 3,4-dihydroxy-6-methylphenylacetic acid and formaldehyde

A solution of 3,4-dihydroxy-6-methylphenylacetic acid (1.72g) and s-trioxane (0.25g) in acetic acid (5 ml) is heated to 90°C and acetic acid/concentrated sulfuric acid (4:1) (0.25 ml) is added. The mixture is heated at 90°C for four hours, then poured into ice water. The mixture is filtered, the solid washed with water, then dissolved in dilute aqueous ammonia. The solution is concentrated in vacuo at 40-45°C to give the desired product as the ammonium salt.

### Example 14

### Preparation of 5-[3-[3-[[5-carbamoylmethyloxy-2-carboxymethyloxy]benzyl]-[5-carboxymethyloxy-2-methoxy]benzyl]-[5-carbamoylmethyloxy-2-methoxy]benzyl]-3-methyl-4-methoxyphenoxyacetic acid

### Step 1: Preparation of 5-benzoyloxy-3-bromo-2-hydroxybenzaldehyde

A mixture of 5-benzoyloxy-2-hydroxybenzaldehyde (32g) and sodium acetate (12.2g) is acetic acid (165 ml) is cooled to 15°C and bromine (25 ml) is added dropwise. The mixture is stirred at 15°C for 15 minutes and at room temperature for about 2 hours. The mixture is diluted with water and filtered. The residue is washed with water, dried, then dissolved in toluene and azeotroped twice with toluene to give the desired product.

### Step 2: Preparation of 5-benzoyloxy-3-bromo-2-methoxybenzaldehyde

5-Benzoyloxy-3-bromo-2-hydroxybenzaldehyde (43.6g) and potassium carbonate (19.3g) are combined in acetonitrile (300 ml) and methyl iodide (40 ml) is added. The mixture is stirred at 45-50°C for about 24 hours, and the excess methyl iodide removed in vacuo. The mixture is diluted with ether, washed with water, brine, and the organic layer dried over magnesium sulfate. The organic is filtered, and concentrated in vacuo to give the desired product.

### Step 3: Preparation of 3-bromo-5-hydroxy-2-methoxy benzaldehyde

5-benzoyloxy-3-bromo-2-methoxybenzaldehyde (41.9g) in THF (350 ml) is cooled to -40°C and sodium methoxide in methanol (25%, 27 ml) is added. The mixture is stirred for about 35 minutes, quenched with 1M HCl (140 ml), diluted with ether, and washed with water, then brine. The organic solution is dried over magnesium sulfate, filtered, concentrated in vacuo, and the residue azeotroped with toluene to give the desired product, which was used, without further treatment, for the next step.

### Step 4: Preparation of 5-benzyloxy-3-bromo-2-methoxybenzaldehyde

3-bromo-5-hydroxy-2-methoxy benzaldehyde (26.8g) and potassium carbonate (17.6g) are combined in acetonitrile (400 ml) and benzyl bromide (16.5 ml) are added. The mixture is stirred at 81°C for about 2.5 hours, cooled to room temperature, and diluted with ether. The mixture is washed with water, brine, dried over magnesium sulfate, filtered, and concentrated in vacuo. The crude product is purified by flash chromatography, eluting with ether/methylene chloride/hexane (1:1:8), to give the desired product.

### Step 5: Preparation of 5-benzyloxy-1-bromo-3-hydroxymethyl-2-methoxybenzene

A solution of 5-benzyloxy-3-bromo-2-methoxybenzaldehyde (42g) in THF (400 ml) is cooled to -60°C and a 0.5M solution of sodium borohydride in diglyme (130 ml) is added dropwise. The mixture is stirred about 40 minutes, then 2M HCI (130 ml) is added. The mixture is diluted with ether and the organic layer washed with water, brine, dried over magnesium sulfate, filtered and concentrated in vacuo. The crude product is purified by flash chromatography, eluting with 50% ether in hexane, to give the desired product.

### Step 6: Preparation of 5-benzyloxy-1-bromo-3-tert-butyldiphenylsilyloxymethyl-2-methoxybenzene

To a solution of 5-benzyloxy-1-bromo-3-hydroxymethyl-2-methoxybenzene (41 g) in methylene chloride (350 ml) is added imidazole (10.6g), and tert-butyl diphenylsilyl chloride (34 ml) followed by DMAP (0.5g). The mixture is stirred for about 45 minutes, diluted with ether, washed with water, brine, and the organic solution dried over magnesium sulfate, filtered, and concentrated in vacuo. The crude product is purified by flash chromatography, eluting with 10% ether in hexane, to give the desired product.

### Step 7: Preparation of 2-(2-methoxy)ethoxymethoxy-5-benzoyloxybenzaldehyde

To a suspension of sodium hydride (60% dispersion, 0.84g) in anhydrous THF (15 ml) which had been cooled to -20°C is added, over 5 minutes, a mixture of 5-benzoyloxy-2-hydroxybenzaldehyde (4.84g), 2-methoxyethoxymethyl chloride (2.51 ml), and DPMU (5 ml). The mixture is then stirred at room temperature for about 4.5 hours, 1N HCl (5 ml) is added, and the mixture diluted with ether. The mixture is washed with water, brine, and the organic layer dried over magnesium sulfate, filtered, and concentrated in vacuo. The crude product is purifed by flash chromatography, eluting with ethyl acetate/methylene chloride/hexane (3:1:6), to give the desired product.

### Step 8: Preparation of 2-(2-methoxy)ethoxymethoxy-5-benzoyloxybenzyl alcohol

A solution of 2-(2-methoxy)ethoxymethoxy-5-benzoyloxybenzaldehyde (5.25g) in THF (35 ml) is cooled to -78°C and a 0.5M solution of sodium borohydride in diglyme (15.9 ml) is added. The mixture is stirred at -78°C for about 5 minutes, then at -45°C for about 3 hours. The mixture is diluted with ether and 1N HCl (16 ml) is added. The layers are separated and the organic layer washed with water, dried over magnesium sulfate, filtered, and concentrated in vacuo. The crude product is purified by flash chromatography, eluting with 35% ethyl acetate/5% methanol in hexane, to give the desired product.

### Step 9: Preparation of 2-(2-methoxy)ethoxymethoxy-5-benzoyloxybenzyl bromide

To a solution of 2-(2-methoxy)ethoxymethoxy-5-benzoyloxybenzyl alcohol (5.25g) is THF (90 ml) is added triphenylphosphine (4.98g) and N-bromosuccinimide (3.38g). The mixture is stirred at 20°C for about 30 minutes, filtered, and concentrated in vacuo. The crude product is purified by flash chromatography, eluting with 25% ethyl acetate in hexane, to give the desired product.

### Step 10: Preparation of 5-benzyloxy-1-[5-benzoyloxy-2-(2-methoxy)ethoxymethoxy]benzyl-3-tert-butyldiphenylsilyloxymethyl-2-methoxybenzene

To a suspension of zinc (41.5g) in THF (20 ml) is added 1,2-dibromoethane (1.35 ml) and the mixture is stirred vigorously at reflux for about 1.5 minutes. The mixture is cooled to -3°C and solution of 2-(2-methoxy)ethoxymethoxy-5-benzoyloxybenzyl bromide (30.7g) in THF (90 ml) is added over a period of about 3 hours. The mixture is stirred for an additional 15 minutes after the addition, then allowed to settle. About one-half of the supernatent solution is then added to a solution of 5-benzyloxy-1-bromo-3-tert-butyldiphenylsilyloxymethyl-2-methoxybenzene (38.2g) and tetrakis(triphenylphosphine)palladium (2.77g) in THF (205 ml). The mixture is stirred at 70°C for about 1.5 hours, then the remainder of the benzylic zinc solution is added and stirring continued at 70°C for about 15 hours. The mixture is cooled, diluted with ether, washed with aqueous ammonium chloride solution, brine, and the organic layer dried over magnesium sulfate, filtered, and concentrated in vacuo. The crude product is purified by flash chromatography, eluting with 40% ether in hexane, to give the desired product.

### Step 11: Preparation of 5-benzyloxy-1-[5-benzoyloxy-2-(2-methoxy)ethoxymethoxy]benzyl-3-hydroxymethyl-2-methoxybenzene

To a solution of 5-benzyloxy-1-[5-benzoyloxy-2-(2-methoxy)ethoxymethoxy]benzyl-3-tert-butyldiphenylsilyloxymethyl-2-methoxybenzene (28.3g) in THF (75 ml) is added a 2M solution of acetic acid in THF (25 ml), then a solution of 1 M tetrabutylammonium fluoride in THF (50 ml). The mixture is stirred at room temperature for about 3 hours, diluted with ether, washed with water, brine, and the organic solution dried over magnesium sulfate, filtered, and concentrated in vacuo. The crude product is purified by flash chromatography, eluting with a gradient of 80% to 100% ether in hexane, to give the desired product.

### Step 12: Preparation of 5-benzyloxy-1-[5-benzoyloxy-2-(2-methoxy)ethoxymethoxy]benzyl-3-bromomethyl-2-methoxybenzene

Using essentially the procedure of Example 14, Step 9, and purifying the crude product by flash chromatography, eluting with ether/methylene chloride/hexane (5:1:4), the desired is prepared from 5-benzyloxy-1-[5-benzoyloxy-2-(2-methoxy)ethoxymethoxy]benzyl-3-hydroxymethyl-2-methoxybenzene.

### Step 13: Preparation of 5-benzyloxy-1-(5-benzoyloxy-2-hydroxy)benzyl-3-tert-butyldiphenylsilyloxymethyl-2-methoxybenzene

To a solution of 5-benzyloxy-1-[5-benzoyloxy-2-(2-methoxy)ethoxymethoxy]benzyl-3-tert-butyldiphenylsilyloxymethyl-2-methoxybenzene (4.3g) in isopropanol (20 ml) and acetone (5 ml) is added pyridinium p-toluenesulfonate (1.35g) and the mixture heated at 68°C for about 34 hours. The mixture is cooled, diluted with ether, washed with water, brine, and the organic solution dried over magnesium sulfate, filtered, and concentrated in vacuo. The crude product purified by flash chromatography, eluting with 40% ether in hexane, to give the desired product.

### Step 14: Preparation of 5-benzyloxy-1-(5-benzoyloxy-2-hydroxy-3-iodo)benzyl-3-tert-butyldiphenylsilyloxymethyl-2-methoxybenzene

To a solution of 5-benzyloxy-1-(5-benzoyloxy-2-hydroxy)benzyl-3-tert-butyldiphenylsilyloxymethyl-2-methoxybenzene (3.34g) and morpholine (1 ml) in methylene chloride (20 ml) is added iodine (1.31g) and the mixture stirred for about 2.5 hours. To the mixture is added 1 M HCl (7 ml) and the mixture is washed with sodium bisulfite solution, brine, dried over magnesium sulfate, and concentrated in vacuo. The crude product is purified by flash chromatography, eluting with ether/methylene chloride/hexane (2:1:7), to give the desired product.

### Step 15: Preparation of 5-benzyloxy-1-(5-benzoyloxy-2-methoxy-3-iodo)benzyl-3-tert-butyidiphenylsilyloxymethyl-2-methoxybenzene

Using essentially the procedure of Example 2, Step 6, and purifying the crude product by flash chromatography, eluting with a gradient of 20% to 30% ether in hexane, the desired product is prepared from 5-benzyloxy-1-(5-benzoyloxy-2-hydroxy-3-iodo)benzyl-3-tert-butyldiphenylsilyloxymethyl-2-methoxybenzene.

### Step 16: Preparation of 1-[3-[3-[[5-benzoyloxy-2-(2-methoxy)ethoxymethoxy]benzyl]-[5-benzyloxy-2-methoxy]benzyl]-[5-benzoyloxy-2-methoxy]benzyl]-5-benzyloxy-3-tert-butyldiphenylsilyloxymethyl-2-methoxybenzene

Using essentially the procedure of Example 14, Step 10, and purifying the crude product by flash chromatography, eluting with 70% ether in hexane, the desired product is prepared from 5-benzyloxy-1-(5-benzoyloxy-2-methoxy-3-iodo)benzyl-3-tert-butyldiphenylsilyloxymethyl-2-methoxybenzene and 5-benzyloxy-1-[5-benzoyloxy-2-(2-methoxy)ethoxymethoxy]benzyl-3-bromomethyl-2-methoxybenzene.

### Step 17: Preparation of 1-[3-[3-[[5-hydroxy-2-(2-methoxy)ethoxymethoxy]benzyl]-[5-benzyloxy-2-methoxy]benzyl]-[5-hydroxy-2-methoxy]benzyl]-5-benzyloxy-3-tert-butyldiphenylsilyloxymethyl-2-methoxybenzene

A solution of 1-[3-[3-[[5-benzoyloxy-2-(2-methoxy)ethoxymethoxy]benzyl]-(5-benzyloxy-2-methoxy]benzyl]-[5-benzoyloxy-2-methoxy]benzyl]-5-benzyloxy-3-tert-butyldiphenylsilyloxymethyl-2-methoxybenzene (0.65g) in THF (2 ml) is cooled to -50°C and a solution of 25% sodium methoxide in methanol (0.23 ml) is added. The solution is stirred for about 35 minutes, quenched with 0.1 M HCI (10 ml), diluted with ether and the organic solution washed with water, dried over magnesium sulfate, filtered, and concentrated in vacuo. The crude product is purified by flash chromatography, eluting with ether/methylene chloride/hexane (1:2:7), to give the desired product.

### Step 18: Preparation of 1-[3-[3-[[5-carbamoylmethyloxy-2-(2-methoxy)ethoxymethoxy]benzyl]-[5-benzyloxy-2-methoxy]benzyl]-[5-carbamoylmethyloxy-2-methoxy]benzyl]-5-benzyloxy-3-tert-butyldiphenylsilyloxymethyl-2-methoxybenzene

To a mixture of 1-[3-[3-[[5-hydroxy-2-(2-methoxy)ethoxymethoxy]benzyl]-[5-benzyloxy-2-methoxy]benzyl]-[5-hydroxy-2-methoxy]benzyl]-5-benzyloxy-3-tert-butyldiphenylsilyloxymethyl-2-methoxybenzene (0.43g) and potassium carbonate (0.12g) in acetonitrile (2 ml) is added 2-bromoacetamide (0.12g) and the mixture is stirred at 40°C for about 14 hours. The mixture is cooled, diluted with ethyl acetae, washed with 0.1 M HCI (4 ml), brine, the dried over magnesium sulfate, filtered, and concentrated in vacuo. The crude is purified by flash chromatography, eluting with ethyl acetate, to give the desired product.

### Step 19: Preparation of 1-[3-[3-[[5-carbamoylmethyloxy-2-(2-methoxy)ethoxymethoxy]benzyl]-[5-hydroxy-2-methoxy]benzyl]-[5-carbamoylmethyloxy-2-methoxy]benzyl]-5-hydroxy-3-methyl-2-methoxybenzene

The product from Example 14, Step 18 is dissolved in a solution of THF/acetic acid (1:1), 5% palladium on carbon (100mg) is added and the mixture stirred under hydrogen at atmospheric pressure for about 7 hours. This was filtered, concentrated and the residue dissolved in ethanol, 200 mg 5% palladium on carbon added, and stirred under hydrogen for about 24 hours. The mixture is filtered, concentrated, to give the desired product which is used, without further treatment, for the next step.

### Step 20: Preparation of 1-[3-[3-[[5-carbamoylmethyloxy-2-hydroxy]benzyl]-[5-hydroxy-2-methoxy]benzyl]-[5-carbamoylmethyloxy-2-methoxy]benzyl]-5-hydroxy-3-methyl-2-methoxybenzene

Using essentially the procedure of Example 14, Step 13, the desired product is prepared from 1-[3-[3-[[5-carbamoylmethyloxy-2-(2-methoxy)ethoxymethoxy]benzyl]-[5-hydroxy-2-methoxy]benzyl]-[5-carbamoylmethyloxy-2-methoxy]benzyl]-5-hydroxy-3-methyl-2-methoxybenzene.

### Step 21: Preparation of tert-butyl-5-[3-[3-[[5-carbamoylmethyloxy-2-tert-butyloxycarbonylmethyloxy]benzyl]-[5-tert-butyloxycarbonyl-2-methoxy]benzyl]-[5-carbamoylmethyloxy-2-methoxy]benzyl]-3-methyl-4-methoxyphenoxyacetate

To a mixture of 1-[3-[3-[[5-carbamoylmethyloxy-2-hydroxy]benzyl]-[5-hydroxy-2-methoxy]benzyl]-[5-carbamoylmethyloxy-2-methoxy]benzyl]-5-hydroxy-3-methyl-2-methoxybenzene (0.35 mmol) and potassium carbonate (0.2g) in acetonitrile (2 ml) is added tert-butyl-2-bromoacetate (0.22 ml) and the mixture stirred at 40°C for 18 hours. The mixture is diluted with ethyl acetate, washed with 1M HCI, brine, dried over magnesium sulfate, filtered, and concentrated in vacuo. The crude product is purified by flash chromatography, eluting with ethyl acetate, to give the desired product.

### Step 22: Preparation of 5-[3-[3-[[5-carbamoylmethyloxy-2-carboxymethyloxy]benzyl]-[5-carboxymethyloxy-2-methoxy]benzyl]-[5-carbamoylmethyloxy-2-methoxy]benzyl]-3-methyl-4-methoxyphenoxyacetic acid

A solution of tert-butyl-5-[3-[3-[[5-carbamoylmethyloxy-2-tert-butyloxycarbonylmethyloxy]benzyl]-[5-tert-butyloxycarbonyl-2-methoxy]benzyl]-[5-carbamoylmethyloxy-2-methoxy]benzyl]-3-methyl-4-methoxyphenoxyacetate (0.3 mmol) in methylene chloride/trifluoroacetic acid (4:1) (3 ml) is stirred at room temperature for about 3.5 hours. The solution is diluted with toluene, concentrated in vacuo, and the residue azeotroped with toluene to give the desired product.

### Example 15

### Preparation of 2-[5-[3-[3-[3-[3-[3-[3-[[5-(2-hydroxyethoxy)-2-methoxy]benzyl]-[5-(2-hydroxyethoxy)-2-methoxy]benzyl]-[5-(2-hydroxyethoxy)-2-methoxy]benzyl]-[5-(2-hydroxyethoxy)-2-methoxy]benzyl]-[5-(2-hydroxyethoxy)-2-methoxy]benzyl]-[5-(2-hydroxyethoxy)-2-methoxy]benzyl]-[5-(2-hydroxyethoxy)-2-methoxy]benzyl]-3-ethyl-4-methoxyphenoxy]ethanol

### Step 1: Preparation of tert-butyl-5-[3-[3-[3-[3-[3-[3-[[5-tert-butyloxycarbonyloxy-2-methoxy]benzyl]-[5-tert-butyloxycarbonyloxy-2-methoxy]benzyl]-[5-tert-butyloxycarbonyloxy-2-methoxy]benzyl]-[5-tert-butyloxycarbonyloxy-2-methoxy]benzyl]-[5-tert-butyloxycarbonyloxy-2-methoxy]benzyl]-[5-tert-butyloxycarbonyloxy-2-methoxy]benzyl]-[5-tert-butyloxycarbonyloxy-2-methoxy]benzyl]-3-ethyl-4-methoxyphenoxyacetate

Using essentially the procedures described above, the desired product is prepared.

### Step 2: Preparation of Preparation of 2-[5-[3-[3-[3-[3-[3-[3-[[5-(2-hydroxyethoxy)-2-methoxy]benzyl]-[5-(2-hydroxyethoxy)-2-methoxy]benzyl]-[5-(2-hydroxyethoxy)-2-methoxy]benzyl]-[5-(2-hydroxyethoxy)-2-methoxy]benzyl]-[5-(2-hydroxyethoxy)-2-methoxy]benzyl]-[5-(2-hydroxyethoxy)-2-methoxy]benzyl]-[5-(2-hydroxyethoxy)-2-methoxy]benzyl]-3-ethyl-4-methoxyphenoxy]ethanol

The product from Example 15, Step 1 (0.134g) is dissolved in THF (1 ml), and lithium aluminum hydride (0.1g) is added. The mixture is stirred at room temperature for about 16 hours, then 0.1 ml water and 0.1 ml 30% aqueous sodium hydroxide are added sequentially, and the mixture diluted with THF, filtered, dried over magnesium sulfate, and concentrated in vacuo to give the desired product.

Using essentially the procedures as described above, the following compounds are prepared from the appropriate starting materials.

### Example 16

### 4-benzyloxy-5-[4-[4-[[2-carboxymethyloxy-5-hydroxy]benzyl]-[5-benzyloxy-2-carboxymethyloxy]benzyl]-[5-benzyloxy-2-carboxymethyloxy]benzyl]-2-hydroxymethylphenoxyacetic acid

### Example 17

### 1 ,2-bis-[[2-carboxymethyloxy-5-hydroxy-4-(5-hydroxy-2-carboxymethyloxy)benzyl]phenyl]ethane

### Example 18

### methyl-2-tert-butyldimethylsilyloxy-5-[4-[4-[4-[4-[4-[4-[4-[4-[4-[4-[4-[4-[4-[4-[[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyll-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-4-methoxyhydrocinnamate

### Example 19

### 4-benzyloxy-5-[4-[4-[4-[4-[[2-carboxymethyloxy-5-hydroxy]benzyl]-[5-benzyloxy-2-carboxymethyloxy]benzyl]-[5-benzyloxy-2-carboxymethyloxy]benzyl]-[5-benzyloxy-2-carboxymethyloxy]benzyl]-[5-benzyloxy-2-carboxymethyloxy]benzyl]-2-hydroxymethylphenoxyacetic acid

### Example 20

### 1,2-bis-[[2-carboxymethyloxy-5-hydroxy-4-(5-benzyloxy-2-carboxymethyloxy)benzyl]phenyl]ethane

### Example 21

### 2-[4-[4-[4-[4-[4-[4-[[5-(2-carboxyethyl)-4-hydroxymethyl-2-methoxy]phenoxymethyl]-[5-(2-carboxyethyl)-2-methoxy]phenoxymethyl]-[5-(2-carboxyethyl)-2-methoxy]phenoxymethyl]-[5-(2-carboxyethyl)-2-methoxy]phenoxymethyl]-[5-(2-carboxyethyl)-2-methoxy]phenoxymethyl]-[5-(2-carboxyethyl)-2-methoxy]phenoxymethyl]-[5-(2-carboxyethyl)-2-methoxy]phenoxymethyl]-4,5-dimethoxyhydrocinnamic acid

### Example 22

### 5-[3-[3-[[5-carboxymethyloxy-2-methoxy]benzyl]-[5-carboxymethyloxy-2-methoxy]benzyl]-[5-carboxymethyloxy-2-methoxy]benzyl]-3-ethyl-4-methoxyphenoxyacetic acid

### Example 23

### 5-[3-[3-[3-[3-[3-[3-[[5-carboxymethyloxy-2-methoxy]benzyl]-[5-carboxymethyloxy-2-methoxy]benzyl]-[5-carboxymethyloxy-2-methoxy]benzyl]-[5-carboxymethyloxy-2-methoxy]benzyl]-[5-carboxymethyloxy-2-methoxy]benzyl]-[5-carboxymethyloxy-2-methoxy]benzyl]-[5-carboxymethyloxy-2-methoxy]benzyl]-3-ethyl-4-methoxyphenoxyacetic acid

### Example 24

### 5-[3-[3-[[5-carbamoylmethyloxy-2-carboxymethyloxy]benzyl]-[5-carboxymethyloxy-2-methoxy]benzyl]-[5-carbamoylmethyloxy-2-methoxy]benzyl]-3-methyl-4-methoxyphenoxyacetic

Various of the above oligomers have been tested for their biological activity in processes normally associated with glycosaminoglycan-protein interaction. In these tests, the activity of the compounds tested indicates their ability to bind with bioactive proteins, as described earlier herein. The tests presented below are designed to measure various biological activities which are considered to correlate to pharmacological activity in human and animal patients.

### Inhibition of Heparanase Activity

Heparanase is an endoglucuronidase capable of degrading heparin sulfate (HS) at specific intrachain sites. Studies on degradation of sulfated proteoglycans in the subendothelial extracellular matrix (ECM) demonstrate a correlation between heparanase activity and the metastatic potential of various tumor cells. Heparanase activity is also suggested to play a role in the mobilization of normal circulating cells of the immune system during inflammatory processes.

The ability of compounds of the present invention to inhibit lymphomacell derived heparanase is tested in the assay system described by Vlodavsky et al., *Cancer Research* **43,** 2704-2711 (1983). 355 labeled ECM is incubated for 24 hours with ESb mouse lymphoma heparanase in the presence of various concentrations of the oligomers tested. Degradation of the HS is followed by gel filtration of the supematants. Heparanase activity is expressed as the total amount of labeled low-molecular-weight fragments released from the EMC substrate.

Compounds within the scope of the present invention have been determined to exhibit a marked activity in the heparanase assay, the results of which are presented in Table I below. The oligomeric compounds are identified in Table I by (1) their example number disclosed hereinabove, and (2) in brackets, the number of units making up the oligomer followed by the number of free carboxylic acids in each oligomer molecule.

**TABLE I-**

| **Results of Heparanase Assays** | |
|---|---|
| Compound Example No. [#-mer, #CO₂H] | % Inhibition of Heparanase Activity (5µg/ml) |
| Ex 4 | 2 |
| [8,8] | |
| Ex 5, Step 2 | 20 |
| [8,8] | |
| Ex 2, Step 26 | 68 |
| [16,16] | |
| Ex 9 | 52 |
| [16,16] | |
| Ex 8 | 70 |
| [16,16] | |

### Induction of Lipoprotein Lipase Release In-Vivo

The enzyme lipoprotein lipase (LPL) participates in the process of lipid transfer from the bloodstream to the tissues. LPL is bound to the external surface of endothelial cells via non-covalent association with cell-membrane glycosaminoglycans. Therefore, the injection of heparin results in a rapid release of LPL into the bloodstream.

In order to test for heparin-like activity, male albino rats (about 200 g) are injected with 10 mg/kg body weight of the oligomers tested in saline. Blood samples are taken from the animals immediately before the injection (time O) and 30 minutes afterwards. LPL activity is measured on duplicates of serum aliquot.

The assay system consists of 0.1 ml of serum sample and 0.1 ml of substrate containing labeled triolein, prepared according to the method of Nilsson-Ehle and Schotz, *J*. *Lipid Res*. **17**, 536-541 (1976). Incubations are carried out at 37°C for 45 minutes. The reaction is stopped by the addition of methanol/chloroform/heptane (1.4:1.25:1 v/v) and the extraction of fatty acids is performed according to the method of Belfrage et al, *J. Lipid Res.* **10,** 341-344 (1969), as modified by Nilsson-Ehle and Schotz. Enzyme activity is calculated according to the formula of Nilsson-Ehle and Schotz.

Biological activity determined by the foregoing tests for compounds within the scope of the present invention comprise useful data for determining utility in the treatment of cardiovascular diseases such as artherosclerosis.

### Anticoagulation Activity

The following test measures anticoagulant activity of the oligomers of the present invention and utilizes the Activated Partial Thromboplastin Time (APTT) test, with the following procedures.

To an assay cuvette is added 100 µl of normal pooled plasma (George King Biomedical Inc., Kansas) and 100 ml of a solution containing the test compound in aqueous 50 mM Tris hydrochloride at pH 7.5 (0.2 mg of sample in one ml buffer). The sample is placed in a MLA coagulation timer which automatically maintains the sample at 37°C for 2.5 minutes, 100 µl of actin activated cephaloplastin reagent is injected, kept 5 minutes, 100 µl of 35 mM CaCI is injected, and clot formation is determined photometrically and the clotting time recorded. Each example is examined at a variety of concentrations, generally from about .025 mg/ml to about 1 mg/ml, and the clotting times are graphed as a function of concentration. From the graphic results, the concentration required to double clotting time (IC_{DCT}) is calculated by linear interpolation.

### Inhibition of Ristocetin and Botrocetin-Induced Binding of vWF to Platelets

The binding of vWf (vonWillebrand Factor) to glycoprotein Ib is measured in one of two experimental systems, in the presence of either ristocetin or botrocetin as modulators of binding. The ristocetin modulated binding is described herein.

Formalin-fixed platelets, prepared according to the method of MacFarlane, D. et al., *Thromb. Diath. Haemorrh.* **34,** 306-308 (1975), were preincubated at room temperature for 15 minutes with specified dilutions of oligomers according to the present invention. Ristocetin, (Sigma, St. Louis, MO) diluted to a final concentration of 1.0 mg/ml, and ¹²⁵I-labelled multimeric vWF (isolated from human plasma cryoprecipitate according to the method of Fulcher, C.A. et al. *Proc. Natl. Acad. Sci. USA,* **79,** 1648-1652 (1982), and labelled according to the method of Fraker, P.J. et al. *Biochem. Biophys. Res. Commun.,* **80,** 849-857 (1978)) were then added to the incubation mixture, and the amount of ¹²⁵I- vWF bound to the platelets was determined. ¹²⁵I-vWF binding to the platelets was referenced against 100% binding which was defined as the amount of ¹²⁵I- vWF bound in the absence of added oligomer compound. The method for measuring vWF binding is reported in detail in Ruggeri et al., *J. Clin. Invest.,* **72**, 1-12, (1983), herein incorporated by reference.

### Platelet aggregation inhibition

The method for measuring platelet aggregation inhibition is essentially that of Ruggeri, et al., *Proc. Natl. Acad. Sci. USA,* **83,** 5708-5712 (1986), using formalin-fixed platelets as prepared by Plow, et al., *Proc. Natl. Acad. Sci. USA,* **79**, 3711-3715 (1982). A given amount of the compound of the present invention or other compound to be tested (10 to 100 µl of a 1 mg/ml solution) is incubated at 37°C with 400 µl of human fixed thrombin activated platelets for 2 minutes. 12.5 µl of human fibrinogen solution is added and aggregation is monitored using a platelet aggregation profiler (Model PAP-4). Controls consist of fibrinogen added to incubated platelets and the resulting aggregation is considered as 100%. Percent inhibition in the presence of a given concentration of test compound versus concentration of the compound is plotted and the concentration required to give 50% inhibition (IC₅₀) is determined.

Compounds within the scope of the present invention have been determined to exhibit a marked activity in the foregoing tests, the results of which are presented in Table II below. The oligomeric compounds are identified in Table II by (1) their example number disclosed hereinabove, and (2) in brackets, the number of units making up the oligomer followed by the number of free carboxylic acids in each oligomer molecule.

**TABLE II-**

| **Results of Anticoagulant and Antithrombotic Assays** | | | |
|---|---|---|---|
| Compound Example No [#-mer, #-CO₂H] | APTT IC_{DCT} (µg/ml) | Platelet Aggregation IC₅₀(µg/ml) | Ristocetin-Induced Inhibition of vWF Binding to Gp1b IC₅₀(µg/ml) |
| Ex 10, Step 13 | >250 | | |
| [4,0] | | | |
| Ex 1, Step 3 | >250 | | >100 |
| [4,4] | | | |
| Ex12, Step 11 | >250 | | >100 |
| [5,5] | | | |
| Ex 3 | 700 | | 21% at 100 µg/ml |
| [4,4] | | | |
| Ex 7 | 500 | | 17% at 100 µg/ml |
| [8,8] | | | |
| Ex 6 | 387 | >1 mg/ml | 42% at 100 µg/ml |
| [8,8] | | | |
| Ex 4 | 430 | >1 mg/ml | 50% at 100 µg/ml |
| [8,8] | | | |
| Ex 5, Step 2 | 37 | >1 mg/ml | |
| [8,8] | | | |
| Ex 9 | 105 | 319 | |
| [16,16] | | | |
| Ex2, Step 26 | 35 | 250 | |
| [16,16] | | | |
| Ex 8 | 35 | | |
| [16,16] | | | |
| Ex 16 | 360 | | |
| [4,4] | | | |
| Ex 17 | 580 | | |
| [4,4] | | | |
| Ex 19 | 345 | | |
| [6,6] | | | |
| Ex 20 | 555 | | |
| [4,4] | | | |
| Ex 22 | >315 | | |
| [4,4] | | | |
| Ex 23 | 300 | | |
| [8,8] | | | |
| (Polymer 1)¹ | 15 | 0.6 | |
| (Polymer 2)² | 17 | 0.38 | |

| | | | |
|---|---|---|---|
| ⁽¹⁾Compound of Example 13, International Publication Number WO 91/07183. | | | |
| ⁽²⁾Compound of Example 13, this case. | | | |

As the foregoing results show, oligomers within the scope of the present invention exhibit anticoagulant and/or antithrombotic activity, which is related to the number of monomeric groups comprising the oligomer chain, the nature of the linking groups and the number and nature of negatively charged substituents thereon.

### Human Granulocyte Elastase Inhibition Assay

The Human Granulocyte Elastase Inhibition assay is a standard test procedure and is essentially that of Kramps, et al., "L-Pyroglutamyl-L-prolyl-L-valine-p-nitroanilide, a highly specific substrate for granulocyte elastase", *Scand. J. clin. Lab. Invest.* **43,** 427-432 (1983).

A stock solution of 8.0 mM L-pyroglutamyl-L-prolyl-L-valine-p-nitroanilide (S-2484, obtained from Kabi) in dimethylsulfoxide is diluted to 2.0 mM in 0.03 M Tris buffer, pH 8.3, to give the working substrate solution. Human neutrophil elastase (obtained from ICN Biochemical) is diluted in the same buffer to 1 unit/ml to give the working enzyme solution.

A control assay is run in which a solution of 10 µl of buffer and 10µl of working enzyme solution is incubated at room temperature for 1 minute and 330 µl of buffer and 50 µl of working substrate solution is then added. The increase in absorbance (ΔOD/min) at 405 nm is measured.

In the experimental assay 10µl of a solution containing a compound of the present invention (in concentrations ranging from 1 mg/ml to 0.1 µg/ml), or other inhibitor to be tested, in buffer is substituted for the 10 µl of buffer with which the enzyme is incubated in the control assay and the procedure followed as for the control assay above. The ΔOD/min is measured and the result expressed as percentage inhibition for the given concentration of inhibitor as compared with the ΔOD/min for the control. Percent inhibition versus concentration of compound of the present invention or other inhibitor is plotted and the data extrapolated to give the concentration required for 50% inhibition of the enzyme (IC₅₀).

It has been unexpectedly found that compounds within the scope of the present invention markedly inhibit the activity of human nuetrophil elastase and are considered to be useful in the treatment of elastase-mediated disorders.

Table III below presents the results of the elastase inhibition assay in terms of IC₅₀ values of compounds within the scope of the present invention and polymers disclosed herein and/or claimed in PCT Intemational Publication Number WO 91/07183.

**TABLE III-**

| **Results of Human Granulocyte Elastase Inhibition Assay** | | |
|---|---|---|
| Compound of Example [#-mer, #-CO₂H] | IC₅₀(nM) | IC₅₀(ng/ml) |
| Ex 12, Step 11 | >1000 | >1000 |
| [5,5] | | |
| Ex 3 | >1000 | >1000 |
| [4,4] | | |
| Ex 7 | 22 | 34 |
| [8,8] | | |
| Ex 6 | 24 | 36 |
| [8,8] | | |
| Ex 4 | 20 | 30 |
| [8,8] | | |
| Ex2, Step 19, Second Product | >1000 | >1000 |
| [8,0] | | |
| Ex 5, Step 2 | 30 | 50 |
| [8,8] | | |
| Ex 2, Step 26 | 24 | 74 |
| [16,16] | | |
| Ex 9 | 14 | 42 |
| [16,16] | | |
| Ex 8 | 24 | 74 |
| [16,16] | | |
| Ex 16 | 330 | 330 |
| [4,4] | | |
| Ex 17 | 135 | 97 |
| [4,4] | | |
| Ex 19 | 38 | 57 |
| [6,6] | | |
| Ex 20 | 780 | 703 |
| [4,4] | | |
| Ex 21 | 41 | 70 |
| [8,8] | | |
| Ex 22 | >3mM | --- |
| [4,4] | | |
| Ex 15, Step 2 | 4,500 | 6,200 |
| [8,0] | | |
| Ex 23 | 25 | 39 |
| [8,8] | | |
| (Polymer 1)¹ | | 130 |
| (Polymer 2)² | | 40 |

| | | |
|---|---|---|
| ⁽¹⁾Compound of Example 13, International Publication Number WO 91/07183. | | |
| ⁽²⁾Compound of Example 13, this case. | | |

### Smooth Muscle Cell DNA Synthesis Assay

Vascular smooth muscle cells (VSMC) are isolated from swine coronaries obtained from swine hearts (Hatfield Meat Packing, Hatfield, PA) by enzyme dissociation as described by Gunther, et al., in J. Cell. Biol. **92**, 289 (1982). Hyaluronidase (0.5 mg/ml) is added to facilitate dispersal. Cells are maintained in Earle's modified minimal essential medium (EMEM) with D-valine substituted for L-valine, supplemented with Hepes (10 mM), fetal bovine serum (FBS) (10%), penicillin (100 U/ml) and streptomycin ( 100 µg/ml). At confluency these cells are bipolar and elongated, exhibit the characteristic "hill nd valley" morphology typical of VSMC and stained for α-actin stress fibers (BTI antimuscle Actin IgG according to manufacturer's (Biomedical Techniques, Inc., Stoughton, MA and Organon Technika Worp., West Chester, PA) instructions).

The VSMC (passage 2) are plated at a density of 3 x 10⁴ /cm2 (1x10⁴ cells/well of 96 well tray) and after 48 hours of normal growth are arrested by replacement of above media with serum-free media for 48 hours consisting of: EMEM: Hams F12 (1:1), insulin, 5 µg/ml; transferrin, 35 µg/ml; selenite, 5 ng/ml; antibiotics and buffer (as above). The oligomer of the present invention to be tested (.01-10 µM) is added 2 hours prior to addition of porcine platelet derived growth factor (Research and Diagonostics, Minneapolis, MN), or FBS,in fresh serum-free media. Twenty-four hours later the oligomers are again added preceding addition of growth factors by 2 hours. (Concentrated solutions of the oligomers (40 mM) are prepared in DMSO, diluted with DMSO to 10 mM and then to 10 µM and further dilution with culture media. DMSO at the highest concentration tested (0.2%) has no effect on DNA synthesis). Consecutive dosing of the VSMC with growth factors produces a reproducible mitogenic response of large magnitude.

The VSMC are pulsed (5 hours) with ³H-thymidine (Amersham, Arlington Heights, IL) (1 µCi/well of a 96 well plate; 42 Ci/mmole) added 15-18 hours after the last dose of growth factor. DNA synthesis is determined in NaOH extraction of ethanol/ether fixed cells as reported by Nemecek, et al., PNAS **83**, 674 (1986). The cells are washed with phosphate bufferred saline (200 µl/well of 96-well tray. The cells are incubated with trichloroacetic acid (TCA) (10%, 200 ul) for 5 minutes to remove cytoplasmic label. After a rapid wash with cold 10% TCA, cells are fixed with ethanol/ether (2:1, 200 µl/well 5 min) and dried. The labeled DNA is extracted with 0.5 N NaOH (200 µl/well 30 min incubation, room temperature). The extracted DNA is neutralized (0.5 N HCI) and counted by liquid scintillation.

Statistical evaluation is as reported by Bilder, et al., *Am. J. Physiol*. **260**: C271, (1991). IC₅₀ values are calculated by linear regression (RPL of RS1) from % inhibition of a minimum of 3 log fold dilutions of oligomers in the presence of PDGF (9 ng/ml) which give 70-80% of a maximal PDGF response. For comparison to other growth factors, equi-effective concentrations of fetal bovine serum (3%) are used. Percent inhibition is calculated by comparing the effect of PDGF alone (vehicle treated cultures + PDGF minus vehicle treated cultures without PDGF) to the effect of oligomers (oligomer + PDGF minus oligomer alone) and divided by the effect of PDGF times 100. i.e., = % inhibition = ((ΔVehicle + PDGF) - (Δoligomer + PDGF)/(Δvehicle + PDGF)) x 100.

Table IV below presents the results of the smooth muscle cell DNA synthesis assay in terms of the IC₅₀ values for inhibition of incorporation of 3H-thymidine in response to PDGF for compounds within the scope of the present invention.

| Compound Example No. [#-mer, #-CO_{2H}] | IC₅₀ (µM) |
|---|---|
| Ex 4 | 1.6 |
| [8,8] | |
| Ex 2, Step 26 | 0.04 |
| [16,16] | |
| Ex 23 | 0.3 |
| [8,8] | |
| Ex 19 | 0.3 |
| [6,6] | |

### Inhibition of DNA Binding to Anti-DNA Antibodies

In order to study the effect of the oligomeric compounds on nucleic acid-protein interaction, the binding of DNA to anti-DNA mouse antibodies (MoAb) is employed as a model.

The anti-DNA A52 hybridoma antibody (IgG 2b,k) was produced by fusion of a BALB/C myeloma cell line with spleen cells of unimmunized, female NZB/NZW FI mice as described previously (Eilat et al., *J*. *Immunol.* **133,** 489-494 (1984)). The nitrocellulose filter assay for the binding of radiolabeled DNA to the specific antibody was performed essentially as described in Eilat. Briefly, reaction mixtures contained 10 µl (50 5 ng, 4000 cpm) of E. coli ¹⁴C DNA (Amersham, Buckinghamshire, England), 10 µl of medium containing A52 mouse hybridoma IgG, 10 µl of the tested inhibitor (in saline) and 0.1 ml of 0.2M borate buffered saline pH 8. In experiments designed to measure how successfully the compunds of the present invention compete with DNA in binding to DNA-specific antibodies, different concentrations of the compounds tested are mixed with radioactive DNA before adding the antibodies. The binding mixtures are left for 15 minutes at 37°C, followed by 1 hour at 4°C, then filtered through 0.45 µm nitrocellulose filters (Millipore, Bedford, MA). The filters are washed twice with borate buffered saline (3 ml), then dried and counted in a toluene-based scintillation liquid.

The foregoing pharmacological test results indicate that the potency and activity of the oligomers of the present invention are related to the number and nature of the charged substituents thereon. It is believed that a component of the means by which the oligomers of the present invention exert their biological activity is electrostatic interaction at charged biological sites. Consequently, preferred compounds of the present invention have at least one substituent which bears a positive or negative charge at biological pH, or have at least one substituent which may be converted by metabolic or other biological processes to such a charged substituent, i.e. in the manner of a prodrug.

Compounds within the scope of the present invention are useful in the treatment of cardiovascular disorders such as thrombosis, bone metabolic disorders, hypolipaemic disorders, neuronal disorders, gastrointestinal disorders, disorders which may be treated by agents effective in binding DNA, and elastase-mediated connective tissue degradation disorders.

The dosage regimen in carrying out the methods of this invention is that which insures maximum therapeutic response until improvement is obtained and thereafter the minimum effective level which gives relief. In general, the oral dose may be between about 3 mg/kg and about 1000 mg/kg (preferably in the range of 10 to 300 mg/kg), and the i.v. dose about 0.1 mg/kg to about 10 mg/kg (preferably in the range of about 0.5 to about 5 mg/kg), bearing in mind, of course, that in selecting the appropriate dosage in any specific case, consideration must be given to the patient's weight, general health, age and other factors which may influence response to the drug. The drug may be administered as frequently as is necessary to achieve and sustain the desired therapeutic response. Some patients may respond quickly to a relatively large or small dose and require little or no maintenance dosage. On the other hand, other patients may require sustained dosing from about 1 to about 4 times a day depending on the physiological needs of the particular patient. Usually the drug may be administered orally 1 to 4 times per day. It is anticipated that many patients will require no more than about one to about two doses daily.

Pharmaceutical compositions according to the present invention useful in the treatment of one or more of the foregoing disorders comprise one or more oligomeric compounds as described herein in an amount effective by oral, parenteral or inhalation administration to inhibit the binding of bioactive macromolecules involved in the pathology of such disorder.

Compositions of this invention may be formulated for administration in any convenient way, and the invention includes within its scope orally, parenterally and inhalatally administrable pharmaceutical compositions containing at least one oligomeric compound as described hereinabove adapted for use in human or veterinary medicine. Such compositions may be formulated in a conventional manner using one or more pharmaceutically acceptable carriers or excipients. Suitable carriers include diluents or fillers, sterile aqueous media and various non-toxic organic solvents. The compositions may be formulated in the form of tablets, capsules, lozenges, troches, hard candies, powders, aqueous suspensions, or solutions, injectable solutions, elixirs, syrups and the like and may contain one or more agents selected from the group including sweetening agents, flavoring agents, coloring agents and preserving agents, in order to provide a pharmaceutically acceptable preparation.

The particular carrier and the ratio of therapeutically effective compound to carrier are determined by the solubility and chemical properties of the compounds, the particular mode of administration and standard pharmaceutical practice. For example, excipients such as lactose, sodium citrate, calcium carbonate and dicalcium phosphate and various disintegratants such as starch, alginic acid and certain complex silicates, together with lubricating agents such as magnesium stearate, sodium lauryl; sodium lauryl sulphate and talc, can be used in producing tablets. For a capsule form, lactose and high molecular weight polyethylene glycols are among the preferred pharmaceutically acceptable carriers. Where aqueous suspensions for oral use are formulated, the carrier can be emulsifying or suspending agents.

Diluents such as ethanol, propylene glycol, glycerin and chloroform and their combinations can be employed as well as other materials.

For parenteral administration such as intramuscular and subcutaneous injection, solutions or suspensions of the polymeric compounds in sesame or peanut oil or aqueous propylene glycol solutions, as well as sterile aqueous solutions can be employed. The aqueous solutions using pure distilled water are also useful for intravenous injection purposes, provided that their pH is properly adjusted, suitably buffered, made isotonic with sufficient saline or glucose and sterilized by heating or by microfiltration.

It is also anticipated that the present invention would be useful as an injectable dosage form which may be administered in an emergency to a patient suffering from stroke or heart attack. Such treatment may be followed by intravenous infusion of the active oligomeric compound and the amount of compound infused into such a patient should be effective to achieve and maintain the desired therapeutic response.

It will be evident to those skilled in the art that the invention is not limited to the details of the foregoing illustrative examples and that the present invention may be embodied in other specific forms without departing from the essential attributes thereof, and it is therefore desired that the present embodiments and examples be considered in all respects as illustrative and not restrictive, reference being made to the appended claims, rather than to the foregoing description, and all changes which come within the meaning and range of equivalency of the claims are therefore intended to be embraced therein.

## Claims

1. A pharmaceutical composition comprising in admixture with a pharmaceutically acceptable carrier, an aromatic oligomeric compounds comprising a single polymeric species of the formula I
Mᵢ-(Mₙ)ₙ-Mₜ I
wherein
n represents a sequence of whole numbers beginning at 2 and having an upper limit of 50;
Mᵢ is 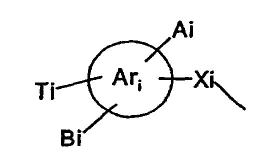
Mₙ is 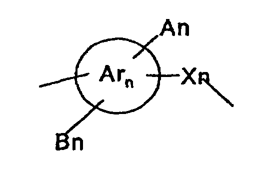
Mₜ is 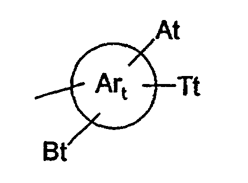 where 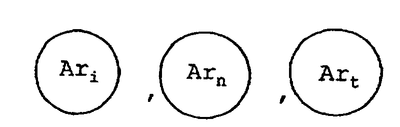 are independently benzene or naphthalene,
where 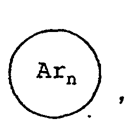 Aₙ , Bₙ, and Xₙ
may be the same or different for each of the (Mₙ) groups in the n sequence; and
Aᵢ, Bᵢ, Aₙ, Bₙ, Aₜ and Bₜ are independently hydrogen, (C₁-C₂₀)alkyl, benzyl, phenethyl, cyano, (C₁-C₄)alkoxy(C₁-C₄)alkoxy, or -(CH₂)ₐ-W-(CH₂)_{b}-Rₛ, where Rₛ is NRR', COOR, CONRR', NR(COR'), PO(OR)₂, COR, SO₂OR, OSO₂OR, halogen, OR, SO₂R, SOR, SR or CHO, and where a and b are independently 0 to 4, (a+b) is less than 5, W is -O-, -S- , -SO-, -SO₂-, -NR(COR')-, -NR-, or a bond, and R and R' are independently hydrogen, (C₁-C₂₀)alkyl, benzyl, or phenethyl,
Tᵢ and Tₜ are independently hydrogen, (C₁-C₂₀)alkyl, allyl, vinyl, halo, cyano, (C₁-C₄)alkoxy(C₁-C₄)alkoxy, halo(C₁-C₂₀)alkyl, hydroxy, *tert*-butyldimethylsilyloxy(C₁-C₂₀)alkyl, hydroxy(C₁-C₂₀)alkyl, (C₁-C₄)alkoxy, benzyloxy, phenethyloxy, phenoxy, 2-napthyloxy, (C₁-C₄) alkoxy(C₁-C₂₀)alkyl, benzyloxy(C₁-C₂₀)alkyl, phenethyloxy(C₁-C₂₀)alkyl, phenoxy(C₁-C₂₀)alkyl, 2-napthyloxy(C₁-C₂₀)alkyl, mercapto, mercapto(C₁-C₂₀)alkyl, (C₁-C₂₀)alkylthio, benzylthio, phenethylthio, (C₁-C₂₀)alkylthio(C₁-C₂₀)alkyl, benzylthio(C₁-C₂₀)alkyl, phenethylthio(C₁-C₂₀)alkyl, amino, (C₁-C₂₀)alkylamino, amino(C₁-C₂₀)alkyl, (C₁-C₂₀)alkylamino(C₁-C₂₀)alkyl, acylamino, acylamino (C₁-C₂₀)alkyl, carboxy, carboxy(C₁-C₂₀)alkyl, (C₁-C₄)alkoxycarbonyl, (C₁-C₄)alkoxycarbony(C₁-C₂₀)lalkyl, benzyloxycarbonyl, phenethyloxycarbonyl, benzyloxycarbonyl(C₁-C₂₀)alkyl, phenethyloxycarbonyl(C₁-C₂₀)alkyl, benzyloxycarbonyloxy, phenethyloxycarbonyloxy, formyl, formyl (C₁-C₂₀)alkyl, acyl, acyloxy, and acyl (C₁-C₂₀)alkyl,
Xᵢ and Xₙ are independently -(CR₁R₂)ₘ-Y-(CR₃R₄)ₚ-where R₁, R₂, R₃ and R₄ are independently hydrogen or (C₁-C₂₀)alkyl, m and p are independently 0 to 5, provided that (m+p) is 0 to 5, and Y is a bond, -O-, -S-, cis or trans -CR₆=CR₇-, -CR₆=CR₇-CR₈=CHR₉- where each of the double bonds may independently be cis or trans, -N(R₅)-, -N(R₅)CO-, -CONR₅-, carbonyl, carbonyloxy, or oxycarbonyl where R₅, R₆, R₇, R₈ and R₉ are independently hydrogen or (C₁-C₂₀)alkyl;
or a pharmaceutically acceptable salt thereof,
provided that when
n is 2;
Tᵢ and Tₜ are hydrogen;
Xᵢ and Xₙ are both carbonyl or methylene; 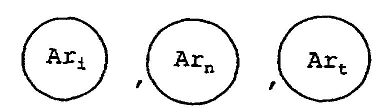 are all phenyl;
then Aᵢ, Aₙ and Aₜ are not all OH or OCOCH₃ and Bᵢ, Bₙ and Bₜ are not F nor Cl, or Aᵢ, Aₙ and Aₜ are not F nor Cl and Bᵢ, Bₙ and Bₜ are not all OH nor OCOCH₃;
and
provided that the oligomeric compound is not:
the tetrameric (di[5-methoxycarbonylethyl-3-(5-[2-methoxycarbonylethyl]-2-hydroxybenzyl)-2-hydroxyphenyl] methane) and di(5-hydroxycarbonyl-ethyl-3-[5-(2-hydroxycarbonylethyl)-2-hydroxybenzyl]-2-hydroxyphenyl) methane, provided further that at least one of Aᵢ, Bᵢ, Bₙ, Aₜ, Bₜ, Tᵢ and Tₜ is a substituent group chosen from the following list: amides, carboxyl, phosphate, phosphonate ester, sulfate, sulfonate, sulfate esters, sulfonate ester and thiol or a pro-substituent group chosen from the following list: amides, esters, alkylcarbonyl, aldehyde and alkylthiol.

2. A pharmaceutical composition according to claim 1 wherein
Mᵢ is 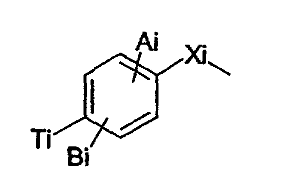
Mₙ is 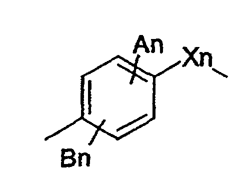
and Mₜ is

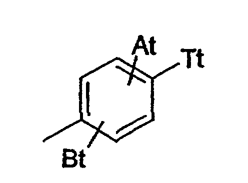

3. A pharmaceutical composition according to claim 1 wherein
Mᵢ is 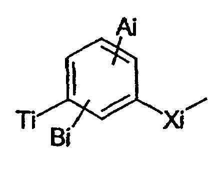
Mₙ is 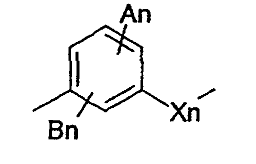
and Mₜ is

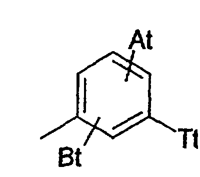

4. A pharmaceutical composition according to claim 1 wherein n is a sequence of whole numbers beginning at 2 and having an upper limit of 18 and wherein (Mₙ)ₙ represents from 2 to 18 monomeric units.

5. A pharmaceutical composition according to claim 1 wherein n is a sequence of whole numbers beginning at 4 and having an upper limit of 14 and wherein (Mₙ)ₙ represents from 4 to 14 monomeric units.

6. A pharmaceutical composition according to claim 1 wherein n is a sequence of whole numbers beginning at 6 and having an upper limit of 18 and wherein (Mₙ)ₙ represents from 6 to 18 monomeric units.

7. A pharmaceutical composition according to claim 1 wherein the composition comprises an oligomeric compound of the formula II 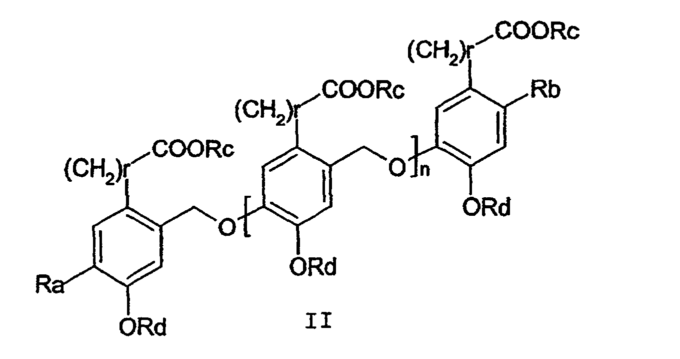 wherein
Ra is hydrogen, hydroxy or acyloxy
Rb is hydrogen, (C₁-C₂₀)alkyl or hydroxy(C₁-C₂₀)alkyl;
Rc is hydrogen or (C₁-C₂₀)alkyl;
Rd is hydrogen or (C₁-C₂₀)alkyl;
r is 1 to 4 and
n is 2 to 30;
or a pharmaceutically acceptable salt thereof.

8. A pharmaceutical composition according to claim 1 wherein the composition comprises an oligomeric compound of the formula III 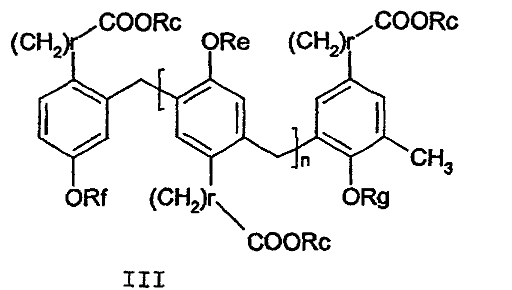 wherein
R_{c}, Rₑ, R_{f} and R_{g} are independently hydrogen or (C₁-C₂₀)alkyl;
r is 1 to 4 and
n is 1 to 30;
or a pharmaceutically acceptable salt thereof.

9. A pharmaceutical composition according to any claim chosen from claims 1 to 3 and 7 wherein n is a sequence of whole numbers having an upper limit of from 4 to 8.

10. A pharmaceutical composition according to claim 1 chosen from the group comprising:
- 2-[4- [4-[4-[[5-(2-carboxyethyl)-2-methoxy] phenoxymethyl]-[5-(2-carboxyethyl)-2-methoxy] phenoxymethyl]-[5-(2-carboxy-ethyl)-2-methoxy]phenoxymethyl]-[5-(2-carboxyethyl)-2-methoxy]phenoxymethyl]-4-methoxyhydro-cinnamic acid,
- 1,2-bis-[4-[4-[4-[[2-(2-carboxyethyl)-5-hydroxy] benzyl]-[2-(2-carboxyethyl-5-hydroxy]benzyl]-[2-(2-carboxyethyl)-5-hydroxy]benzyl]-[2-(2-carboxyethyl)-5-hydroxy]phenyl] ethane
- bis-[5-(2-carboxyethyl)-3-[5-(2-carboxyethyl)-2-hydroxybenzyl]-2-hydroxyphenyl],
- methyl-5-acetoxy-2-[4-[4-[[5-(2-methoxycarbonylethyl)-4-hydroxymethyl-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-4-methoxyhydrocinnamate,
- 1,2-bis-[2-(2-carboxyethyl)-4-[2-(2-carboxyethyl)-5-hydroxybenzyl]-5-hydroxyphenyl],
- 5-[4-[4-[4-[4-[4-[4-[[2-(2-carboxyethyl)-5-hydroxy] benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-4-hydroxy-2- methylhydrocinnamic acid,
- 2-(2-methoxycarbonylethyl)-4-[4-[4-[4-[4-[4-[4-[[2-(2-methoxycarbonylethyl)-5-trimethylacetyloxy]benzyl]-[2-(2-methoxycarbonylethyl)-5-methoxy]benzyl]-(2-(2-methoxycarbonylethyl)-5-methoxy]benzyl]-[2-(2-methoxycarbonylethyl)-5-methoxy]benzyl]-[2- (2-methoxycarbonyiethyl)-5-methoxy]benzyl]-[2-(2-methoxycarbonylethyl)-5-methoxy]benzyl]-[2-(2-methoxycarbonylethyl)-5-methoxy]benzyl]-5-methoxybenzyloxy-tert-butyldimethylsilane,
- 1,2-bis-[4-[4-[4-[[2-(2-methoxycarbonylethyl)-5-trimethylacetyloxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]phenyl]ethane,
- 5-[4-[4-[4-[4-[4-[4-[[2-(2-carboxyethyl)-5-hydroxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-4-methoxy-2-methylhydrocinnamic acid,
- 5-[4-[4-[4-[4-[4-[4-[4-[4-[4-[4-[4-[4-[4-[4-[[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-4-hydroxy-2-methylhydrocinnamic acid,
- 1,2-bis-[4-[4-[4-[4-[4-[4-[4-[[2-(2-carboxyethyl)-5-hydroxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]phenyl]ethane,
- 5-[4-[4-[4-[4-[4-[4-[4-[4-[4-[4-[4-[4-[4-[4-[[2-(2-carboxyethyl)-5-hydroxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl) -5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl) -5-methoxy]benzyl]-[2-(2-carboxyethyl)-5-methoxy]benzyl]-[2-(2-carboxyethyl)-5-methoxy]benzyl]-4-hydroxy-2-methylhydrocinnamic acid,
- methyl-5-acetoxy-2-[4-[4-[4-[4-[4-[4-[[5-(2-methoxycarbonylethyl)-4-hydroxymethyl-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl) -2-methoxy]phenoxymethyl]-4-methoxyhydrocinnamate,
- methyl-5-acetoxy-2-[4-[4-[4-[4-[4-[4-[4-[4-[4-[4-[4-[4-[4-[4-[[5-(2-methoxycarbonylethyl)-4-hydroxymethyl-2-methoxy]phenoxymethyl]-[5-(2-methoxycarbonylethyl)-2-methoxy]phenoxymethyl]-(5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-4-methoxyhydrocinnamate,
- 1,2-bis-[4-[4-[4-[[2-(2-carboxyethyl)-5-hydroxy] benzyl]-[2-(2-carboxyethyl)-5-methoxy]benzyl]-[2-(2-carboxyethyl)-5-methoxy]benzyl]-[2-(2-carboxyethyl)-5-methoxy]phenyl]ethane.
- methyl-5-acetoxy-2-[4-[4-[4-[4-[4-[4-[4-[4-[4-[4-[4-[4-[4-[4-[[5-(2-methoxycarbonylethyl) -4-hydroxymethyl-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl) -2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-4-methoxyhydrocinnamate, - 1,2-bis-[4-[4-[4-[[2-(2-carboxyethyl)-5-hydroxy]benzyl] -[2-(2-carboxyethyl)-5-methoxy]benzyl]-[2-(2-carboxyethyl)-5-methoxy]benzyl]-[2-(2-carboxyethyl)-5-methoxy]phenyl]ethane.

11. Use of a composition according to any claim from 1 to 10 for the manufacture of a medicament for treating an elastase-mediated connective tissue degradation.

12. Use of a composition according to any claim from 1 to 10 for the manufacture of a medicament for treating thrombosis, inhibiting smooth muscle cell proliferation, or use as anticoagulant.

13. An oligomeric compound comprising a single polymeric species of the formula I
Mᵢ-(Mₙ)ₙ-Mₜ I
wherein
n represents a sequence of whole numbers beginning at 2 and having an upper limit of 50;
Mᵢ is 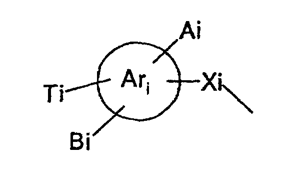
Mₙ is 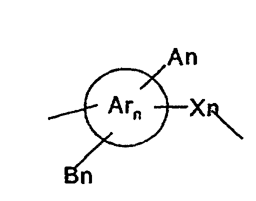
Mₜ is 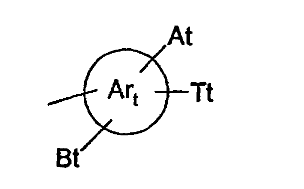 where 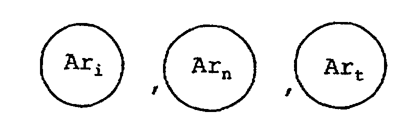 are independently benzene or naphthalene,
where 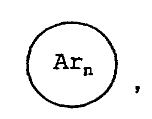 Aₙ , Bₙ, and Xₙ may be the same or different for each of the (Mₙ) groups in the n sequence; and
Aᵢ, Bᵢ, Aₙ, Bₙ, Aₜ and Bₜ are independently hydrogen, (C₁-C₂₀)alkyl, benzyl, phenethyl, cyano, (C₁-C₄)alkoxy(C₁-C₄)alkoxy, or-(CH₂)ₐ-W-(CH₂)_{b}-Rₛ, where Rₛ is NRR', COOR, CONRR', NR(COR'), PO(OR)₂, COR, SO₂OR, OSO₂OR, halogen, OR, SO₂R, SOR, SR or CHO, and where a and b are independently 0 to 4, (a+b) is less than 5, W is -O-, -S-, -SO-, -SO₂-, -NR(COR')-, -NR-, or a bond, and R and R' are independently hydrogen, (C₁-C₂₀)alkyl, benzyl, or phenethyl,
Tᵢ and Tₜ are independently hydrogen, (C₁-C₂₀)alkyl, allyl, vinyl, halo, cyano, (C₁-C₄)alkoxy(C₁-C₄)alkoxy, halo(C₁-C₂₀)alkyl, hydroxy, *tert*-butyldimethylsilyloxy(C₁-C₂₀)alkyl, hydroxy(C₁-C₂₀)alkyl, (C₁-C₄)alkoxy, benzyloxy, phenethyloxy, phenoxy, 2-napthyloxy, (C₁-C₄)alkoxy(C₁-C₂₀)alkyl, benzyloxy(C₁-C₂₀)alkyl, phenethyloxy(C₁-C₂₀)alkyl, phenoxy (C₁-C₂₀)alkyl, 2-napthyloxy(C₁-C₂₀)alkyl, mercapto, mercapto (C₁-C₂₀)alkyl, (C₁-C₂₀)alkylthio, benzylthio, phenethylthio, (C₁-C₂₀)alkylthio(C₁-C₂₀)alkyl, benzylthio (C₁-C₂₀)alkyl, phenethylthio(C₁-C₂₀)alkyl, amino, (C₁-C₂₀)alkylamino, amino(C₁-C₂₀)alkyl, (C₁-C₂₀)alkylamino (C₁-C₂₀)alkyl, acylamino, acylamino(C₁-C₂₀)alkyl, carboxy, carboxy (C₁-C₂₀)alkyl, (C₁-C₄)alkoxycarbonyl, (C₁-C₄)alkoxycarbony(C₁-C₂₀)lalkyl, benzyloxycarbonyl, phenethyloxycarbonyl, benzyloxycarbonyl (C₁-C₂₀)alkyl, phenethyloxycarbonyl (C₁-C₂₀)alkyl, benzyloxycarbonyloxy, phenethyloxycarbonyloxy, formyl, formyl(C₁-C₂₀)alkyl, acyl, acyloxy, and acyl(C₁-C₂₀)alkyl,
Xᵢ and Xₙ are independently -(CR₁R₂)ₘ-Y-(CR₃R₄)ₚ-where R₁, R₂, R₃ and R₄ are independently hydrogen or (C₁-C₂₀)alkyl, m and p are independently 0 to 5, provided that (m+p) is 0 to 5, and Y is a bond, -O-, -S-, cis or trans -CR₆=CR₇-, -CR₆=CR₇-CR₈=CHR₉- where each of the double bonds may independently be cis or trans, -N(R₅)-, -N(R₅)CO-, -CONR₅-, carbonyl, carbonyloxy, or oxycarbonyl where R₅, R₆, R₇, R₈ and R₉ are independently hydrogen or (C₁-C₂₀)alkyl;
or a pharmaceutically acceptable salt thereof,
provided that when
n is 2;
Tᵢ and Tₜ are hydrogen;
Xᵢ and Xₙ are both carbonyl or methylene; 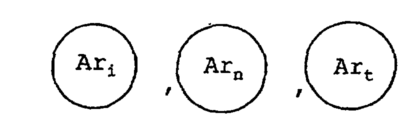 are all phenyl;
then Aᵢ, Aₙ and Aₜ are not all OH or OCOCH₃ and Bᵢ, Bₙ and Bₜ are not F nor Cl, or Aᵢ, Aₙ and Aₜ are not F nor Cl and Bᵢ, Bₙ and Bₜ are not all OH nor OCOCH₃;
and
provided that the oligomeric compound is not:
the tetrameric (di[5-methoxycarbonylethyl-3-(5-[2-methoxycarbonylethyl]-2-hydroxybenzyl)-2-hydroxyphenyl] methane) and di(5-hydroxycarbonyl-ethyl-3-[5-(2-hydroxycarbonylethyl)-2-hydroxybenzyl]-2-hydroxyphenyl) methane, provided further that at least one of Aᵢ, Bᵢ, Bₙ, Aₜ, Bₜ, Tᵢ and Tₜ is a substituent group chosen from the following list: amines, carboxyl, phosphate, phosphonate ester, sulfate, sulfonate, sulfate esters, sulfonate ester and thiol or a pro-substituent group chosen from the following list: amides, esters, alkylcarbonyl, aldehyde and alkylthiol.

## Patentansprüche

1. Pharmazeutische Zusammensetzung, umfassend in Anmischung mit einem pharmazeutisch verträglichen Träger eine aromatische oligomere Verbindung, umfassend eine einzige polymere Spezies der Formel I
Mᵢ-(Mₙ)ₙ-Mₜ I
worin
n eine Folge ganzer Zahlen beginnend mit 2 und mit einer oberen Grenze von 50 wiedergibt;
Mᵢ 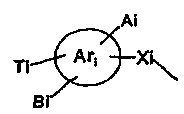 darstellt,
Mₙ 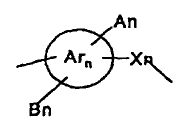 darstellt,
Mₜ 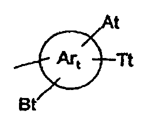 darstellt,
worin 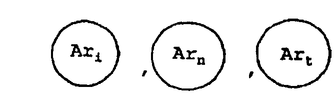 unabhängig voneinander Benzol oder Naphthalin bedeuten,
worin 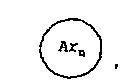 Aₙ, Bₙ, und Xₙ gleich oder verschieden für jede der Gruppen (Mₙ) in der Folge n sein können; und
Aᵢ, Bᵢ, Aₙ, Bₙ, Aₜ und Bₜ unabhängig voneinander Wasserstoff, (C₁-C₂₀)Alkyl, Benzyl, Phenethyl, Cyano, (C₁-C₄)Alkoxy(C₁-C₄)alkoxy, oder -(CH₂)ₐ-W-(CH₂)_{b}-Rₛ darstellen, worin Rₛ NRR', COOR, CONRR', NR(COR'), PO(OR)₂, COR, SO₂OR, OSO₂OR, Halogen, OR, SO₂R, SOR, SR oder CHO darstellt und worin a und b unabhängig voneinander 0 bis 4 sind, (a+b) weniger als 5 sind, W -O-, -S-, -SO-, -SO₂, -NR(COR')-, -NR- oder eine Bindung darstellt und R und R' unabhängig voneinander Wasserstoff, (C₁-C₂₀)Alkyl, Benzyl oder Phenethyl darstellen,
Tᵢ und Tₜ unabhängig voneinander Wasserstoff, (C₁-C₂₀)Alkyl, Allyl, Vinyl, Halogen, Cyano, (C₁-C₄)Alkoxy(C₁-C₄)alkoxy, Halogen(C₁-C₂₀)alkyl, Hydroxy, tert-Butyldimethylsilyloxy(C₁-C₂₀)alkyl, Hydroxy (C₁-C₂₀)alkyl, (C₁-C₄)Alkoxy, Benzyloxy, Phenethyloxy, Phenoxy, 2-Naphthyloxy, (C₁-C₄)-Alkoxy(C₁-C₂₀)alkyl, Benzyloxy(C₁-C₂₀)alkyl, Phenethyloxy(C₁-C₂₀)alkyl, Phenoxy(C₁-C₂₀)alkyl, 2-Naphthyloxy(C₁-C₂₀)alkyl, Mercapto, Mercapto (C₁-C₂₀)alkyl, (C₁-C₂₀)Alkylthio, Benzylthio, Phenethylthio, (C₁-C₂₀) Alkylthio (C₁-C₂₀)alkyl, Benzylthio(C₁-C₂₀)alkyl, Phenethylthio(C₁-C₂₀)alkyl, Amino, (C₁-C₂₀) Alkylamino, Amino (C₁-C₂₀)alkyl, (C₁-C₂₀)Alkylamino(C₁-C₂₀)alkyl, Acylamino, Acylamino (C₁-C₂₀)alkyl, Carboxy, Carboxy(C₁-C₂₀)alkyl, (C₁-C₄)Alkoxycarbonyl, (C₁-C₄)Alkoxycarbonyl(C₁-C₂₀)alkyl, Benzyloxycarbonyl, Phenethyloxycarbonyl, Benzyloxycarbonyl(C₁-C₂₀)alkyl, Phenethyloxycarbonyl(C₁-C₂₀)alkyl, Benzyloxycarbonyloxy, Phenethyloxycarbonyloxy, Formyl, Formyl(C₁-C₂₀)alkyl, Acyl, Acyloxy und Acyl (C₁-C₂₀)alkyl darstellen;
Xᵢ und Xₙ unabhängig voneinander -(CR₁R₂)ₘ-Y-(CR₃R₄)ₚ- darstellen, worin R₁, R₂, R₃ und R₄ unabhängig voneinander Wasserstoff oder (C₁-C₂₀)Alkyl darstellen, m und p unabhängig voneinander 0 bis 5 sind, mit der Maßgabe, dass (m+p) 0 bis 5 ist und Y eine Bindung, -O-, -S-, cis oder trans -CR₆=CR₇-, -CR₆=CR₇-CR₈=CR₉-, worin jede der Doppelbindungen unabhängig voneinander cis oder trans sein kann, -N(R₅)-, -N(R₅)CO-, -CONR₅-, Carbonyl, Carbonyloxy oder Oxycarbonyl darstellt, wobei R₅, R₆, R₇, R₈ und R₉ unabhängig voneinander Wasserstoff oder (C₁-C₂₀)Alkyl darstellen,
oder ein pharmazeutisch verträgliches Salz davon,
mit der Maßgabe, dass wenn
n 2 ist;
Tᵢ und Tₜ Wasserstoff darstellen;
Xᵢ und Xₙ beide Carbonyl oder Methylen darstellen; 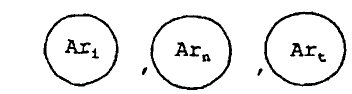 alle Phenyl darstellen; dann Aᵢ, Aₙ und Aₜ nicht alle OH oder OCOCH₃ bedeuten und Bᵢ, Bₙ und Bₜ weder F noch Cl sind oder Aᵢ, Aₙ und Aₜ weder F noch Cl sind und Bᵢ, Bₙ und Bₜ nicht alle OH noch OCOCH₃ bedeuten;
und mit der Maßgabe, dass die oligomere Verbindung nicht bedeutet:
tetrameres (Di[5-methoxycarbonylethyl-3-(5-[2-methoxycarbonylethyl]-2-hydroxybenzyl)-2-hydroxyphenyl]methan) und (Di[5-hydroxycarbonylethyl-3-[5-(2-hydroxycarbonylethyl)-2-hydroxybenzyl]-2-hydroxyphenyl]methan) mit der weiteren Maßgabe, dass mindestens ein Rest von Aᵢ, Bᵢ, Bₙ, Aₜ, Bₜ, Tᵢ und Tₜ eine aus der nachstehenden Liste: Amide, Carboxyl, Phosphat, Phosphonatester, Sulfat, Sulfonat, Sulfatester, Sulfonatester und Thiol ausgewählte Substituentengruppe oder eine aus der nachstenden Liste: Amide, Ester, Alkylcarbonyl, Aldehyd und Alkylthiol ausgewählte Prosubstituentengruppe ist.

2. Pharmazeutische Zusammensetzung nach Anspruch 1, worin
Mᵢ 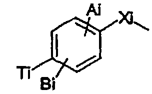 darstellt,
Mₙ 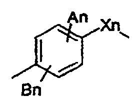 darstellt, und
Mₜ 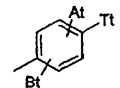 darstellt.

3. Pharmazeutische Zusammensetzung nach Anspruch 1, worin
Mᵢ 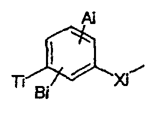 darstellt,
Mₙ 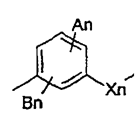 darstellt, und
Mₜ 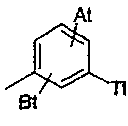 darstellt.

4. Pharmazeutische Zusammensetzung nach Anspruch 1, worin n eine Folge ganzer Zahlen beginnend mit 2 und mit einer oberen Grenze von 18 wiedergibt und worin (Mₙ)ₙ 2 bis 18 monomere Einheiten wiedergibt.

5. Pharmazeutische Zusammensetzung nach Anspruch 1, worin n eine Folge ganzer Zahlen beginnend mit 4 und mit einer oberen Grenze von 14 wiedergibt und worin (Mₙ)ₙ 4 bis 14 monomere Einheiten wiedergibt.

6. Pharmazeutische Zusammensetzung nach Anspruch 1, worin n eine Folge ganzer Zahlen beginnend mit 6 und mit einer oberen Grenze von 18 wiedergibt und worin (Mₙ)ₙ 6 bis 18 monomere Einheiten wiedergibt.

7. Pharmazeutische Zusammensetzung nach Anspruch 1, worin die Zusammensetzung eine oligomere Verbindung der Formel II 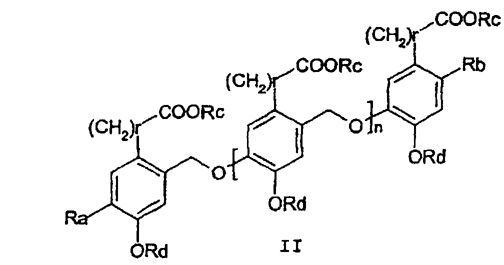 umfasst,
worin
Ra Wasserstoff, Hydroxy oder Acyloxy ist;
Rb Wasserstoff, (C₁-C₂₀)Alkyl oder Hydroxy(C₁-C₂₀)alkyl ist;
Rc Wasserstoff oder (C₁-C₂₀)Alkyl ist;
Rd Wasserstoff oder (C₁-C₂₀)Alkyl ist;
r 1 bis 4 ist und
n 2 bis 30 ist;
oder ein pharmazeutisch verträgliches Salz davon.

8. Pharmazeutische Zusammensetzung nach Anspruch 1, worin die Zusammensetzung eine oligomere Verbindung der Formel III 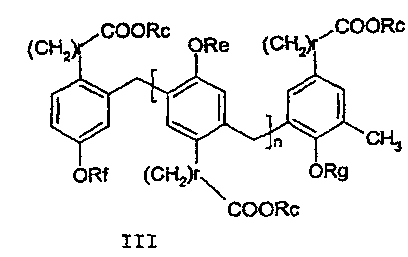 umfasst,
worin
R_{c}, Rₑ, R_{f} und R_{g} unabhängig voneinander Wasserstoff oder (C₁-C₂₀)Alkyl darstellen;
r 1 bis 4 ist und
n 1 bis 30 ist;
oder ein pharmazeutisch verträgliches Salz davon.

9. Pharmazeutische Zusammensetzung nach einem Anspruch, ausgewählt aus Ansprüchen 1 bis 3 und 7, worin n eine Folge ganzer Zahlen mit einer oberen Grenze von 4 bis 8 wiedergibt.

10. Pharmazeutische Zusammensetzung nach Anspruch 1, ausgewählt aus der Gruppe, umfassend:
- 2-[4-[4-[4[[5-(2-Carboxyethyl)-2-methoxy]phenoxymethyl]-[5-(2-carboxyethyl)-2-methoxy]phenoxymethyl]-[5-(2-carboxyethyl)-2-methoxy]phenoxymethyl]-[5-(2-carboxyethyl)-2-methoxy]phenoxymethyl]-4-methoxyhydrozimtsäure,
- 1,2-Bis[4-[4-[4[[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]phenyl]ethan,
- Bis-[5-(2-carboxyethyl)-3-[5-(2-carboxyethyl)-2-hydroxybenzyl]-2-hydroxyphenyl,
- Methyl-5-acetoxy-2-[4-[4[[5-(2-methoxycarbonylethyl)-4-hydroxymethyl-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-4-methoxyhydrocinnamat,
- 1,2-Bis-[2-(2-carboxyethyl)-4-[2-(2-carboxyethyl)-5-hydroxybenzyl]-5-hydroxyphenyl],
- 5-[4-[4-[4-[4-[4-[4-[[2-(2-Carboxyethyl)-5-hydroxy]-benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-4-hydroxy-2-methylhydrocinnamat,
- 2-(2-Methoxycarbonylethyl)-4-[4-[4-[4-[4-[4-[4-[[2-(2-methoxycarbonylethyl)-5-trimethylacetyloxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]benzyl]-5-methoxybenzyloxytert-butyldimethylsilan,
- 1,2-Bis-[4-[4-[4-[[2-(2-methoxycarbonylethyl)-5-trimethylacetyloxy]benzyl]-[2-(2-methoxycarbonylethyl)-5-methoxy]benzyl]-[2-(2-methoxycarbonylethyl)-5-methoxy]benzyl]-[2-(2-methoxycarbonylethyl)-5-methoxy]phenyl]ethan,
- 5-[4-[4-[4-[4-[4-[4-[[2-(2-Carboxyethyl)-5-hydroxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-4-methoxy-2-methylhydrozimtsäure,
- 5-[4-[4-[4-[4-[4-[4-[4-[4-[4-[4-[4-[4-[4-[4-[[2-(2-Carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl]-[2-(2-carboxyethyl)-5-hydroxy]benzyl] -4-hydroxy-2-methylhydrozimtsäure,
- 1,2-Bis-[4-[4-[4-[4-[4-[4-[4-[[2-(2-carboxyethyl)-5-hydroxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]phenyl]ethan,
- 5-[4-[4-[4-[4-[4-[4-[4-[4-[4-[4-[4-[4-[4-[4-[[2-(2-Carboxyethyl)-5-hydroxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-[2-(2-carboxyethyl)-5-methoxy]benzyl]-4-hydroxy-2-methylhydrozimtsäure,
- Methyl-5-acetoxy-2-[4-[4-[4-[4-[4-[4-[[5-(2-methoxycarbonylethyl)-4-hydroxymethyl-2-methoxy]phenoxymethyl]-[5-(2-methoxycarbonylethyl)-2-methoxy]phenoxymethyl]-[5-(2-methoxycarbonylethyl)-2-methoxy]-phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-4-methoxyhydrocinnamat,
- Methyl-5-acetoxy-2-[4-[4-[4-[4-[4-[4-[4-[4-[4-[4-[4-[4-[4-[4-[[5-(2-methoxycarbonylethyl)-4-hydroxymethyl-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]-phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-4-methoxyhydrocinnamat,
- 1,2-Bis-[4-[4-[4-[[2-(2-carboxyethyl)-5-hydroxy]benzyl]-[2-(2-carboxyethyl)-5-methoxy]benzyl]-[2-(2-carboxyethyl)-5-methoxy]benzyl]-[2-(2-carboxyethyl)-5-methoxy]phenyl]ethan,
- Methyl-5-acetoxy-2-[4-[4-[4-[4-[4-[4-[4-[4-[4-[4-[4-[4-[4-[4-[[5-(2-methoxycarbonylethyl)-4-hydroxymethyl-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]-phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-[5-(2-methoxycarbonylethyl)-2-methoxy]phenoxymethyl]-4-methoxyhydrocinnamat,
- 1,2-Bis-[4-[4-[4-[[2-(2-carboxyethyl)-5-hydroxy]benzyl]-[2-(2-carboxyethyl)-5-methoxy]benzyl]-[2-(2-carboxyethyl)-5-methoxy]benzyl]-[2-(2-carboxyethyl)-5-methoxy]phenyl]ethan.

11. Verwendung einer Zusammensetzung nach einem Anspruch von 1 bis 10 zur Herstellung eines Arzneimittels zur Behandlung von Elastase vermitteltem Bindegewebsabbau.

12. Verwendung einer Zusammensetzung nach einem Anspruch von 1 bis 10 zur Herstellung eines Arzneimittels zur Behandlung von Thrombose, Hemmung von Glattmuskelzellproliferation oder Verwendung als Gerinnungshemmer.

13. Oligomere Verbindung, umfassend eine einzige polymere Spezies der Formel I
Mᵢ-(Mₙ)ₙ-Mₜ I
worin
n eine Folge ganzer Zahlen beginnend mit 2 und mit einer oberen Grenze von 50 wiedergibt;
Mᵢ 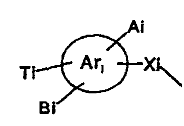 darstellt,
Mₙ 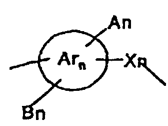 darstellt,
Mₜ 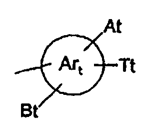 darstellt,
worin 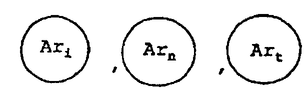 unabhängig voneinander Benzol oder Naphthalin bedeuten,
worin 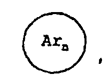 Aₙ, Bₙ, und Xₙ gleich oder verschieden für jede der Gruppen (Mₙ) in der Folge n sein können; und
Aᵢ, Bᵢ, Aₙ, Bₙ, Aₜ und Bₜ unabhängig voneinander Wasserstoff, (C₁-C₂₀)Alkyl, Benzyl, Phenethyl, Cyano, (C₁-C₄)Alkoxy(C₁-C₄) alkoxy, oder - (CH₂)ₐ-W-(CH₂)_{b}-Rₛ darstellen, worin Rₛ NRR', COOR, CONRR', NR(COR'), PO(OR)₂, COR, SO₂OR, OSO₂OR, Halogen, OR, SO₂R, SOR, SR oder CHO darstellt und worin a und b unabhängig voneinander 0 bis 4 sind, (a+b) weniger als 5 sind, W -O-, -S-, -SO-, -SO₂, -NR(COR')-, -NR- oder eine Bindung darstellt und R und R' unabhängig voneinander Wasserstoff, (C₁-C₂₀)Alkyl, Benzyl oder Phenethyl darstellen,
Tᵢ und Tₜ unabhängig voneinander Wasserstoff, (C₁-C₂₀)Alkyl, Allyl, Vinyl, Halogen, Cyano, (C₁-C₄)Alkoxy(C₁-C₄)alkoxy, Halogen(C₁-C₂₀)alkyl, Hydroxy, tert-Butyldimethylsilyloxy(C₁-C₂₀)alkyl, Hydroxy (C₁-C₂₀)alkyl, (C₁-C₄)Alkoxy, Benzyloxy, Phenethyloxy, Phenoxy, 2-Naphthyloxy, (C₁-C₄)-Alkoxy(C₁-C₂₀)alkyl, Benzyloxy(C₁-C₂₀)alkyl, Phenethyloxy(C₁-C₂₀)alkyl, Phenoxy(C₁-C₂₀)alkyl, 2-Naphthyloxy (C₁-C₂₀)alkyl, Mercapto, Mercapto (C₁-C₂₀)alkyl, (C₁-C₂₀)Alkylthio, Benzylthio, Phenethylthio, (C₁-C₂₀)Alkylthio(C₁-C₂₀)alkyl, Benzylthio(C₁-C₂₀)alkyl, Phenethylthio(C₁-C₂₀)alkyl, Amino, (C₁-C₂₀)Alkylamino, Amino (C₁-C₂₀)alkyl, (C₁-C₂₀)Alkylamino(C₁-C₂₀)alkyl, Acylamino, Acylamino(C₁-C₂₀)alkyl, Carboxy, Carboxy(C₁-C₂₀)alkyl, (C₁-C₄)Alkoxycarbonyl, (C₁-C₄)Alkoxycarbonyl(C₁-C₂₀)alkyl, Benzyloxycarbonyl, Phenethyloxycarbonyl, Benzyloxycarbonyl(C₁-C₂₀)alkyl, Phenethyloxycarbonyl(C₁-C₂₀)alkyl, Benzyloxycarbonyloxy, Phenethyloxycarbonyloxy, Formyl, Formyl(C₁-C₂₀)alkyl, Acyl, Acyloxy und Acyl(C₁-C₂₀)alkyl darstellen;
Xᵢ und Xₙ unabhängig voneinander -(CR₁R₂)ₘ-Y-(CR₃R₄)ₚ- darstellen, worin R₁, R₂, R₃ und R₄ unabhängig voneinander Wasserstoff oder (C₁-C₂₀)Alkyl darstellen, m und p unabhängig voneinander 0 bis 5 sind, mit der Maßgabe, dass (m+p) 0 bis 5 ist und Y eine Bindung, -O-, -S-, cis oder trans -CR₆=CR₇-, -CR₆=CR₇-CR₈=CR₉-, worin jede der Doppelbindungen unabhängig voneinander cis oder trans sein kann, -N(R₅)-, -N(R₅)CO-, -CONR₅-, Carbonyl, Carbonyloxy oder Oxycarbonyl darstellt, wobei R₅, R₆, R₇, R₈ und R₉ unabhängig voneinander Wasserstoff oder (C₁-C₂₀)Alkyl darstellen,
oder ein pharmazeutisch verträgliches Salz davon,
mit der Maßgabe, dass wenn
n 2 ist;
Tᵢ und Tₜ Wasserstoff darstellen;
Xᵢ und Xₙ beide Carbonyl oder Methylen darstellen; 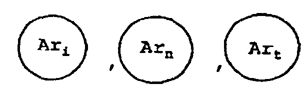 alle Phenyl darstellen;
dann Aᵢ, Aₙ und Aₜ nicht alle OH oder OCOCH₃ bedeuten und Bᵢ, Bₙ und Bₜ weder F noch Cl sind oder Aᵢ, Aₙ und Aₜ weder F noch Cl sind und Bᵢ, Bₙ und Bₜ nicht alle OH noch OCOCH₃ bedeuten;
und mit der Maßgabe, dass die oligomere Verbindung nicht bedeutet:
tetrameres (Di[5-methoxycarbonylethyl-3-(5-[2-methoxycarbonylethyl]-2-hydroxybenzyl)-2-hydroxyphenyl]methan) und (Di[5-hydroxycarbonylethyl-3-[5-(2-hydroxycarbonylethyl)-2-hydroxybenzyl]-2-hydroxyphenyl]methan) mit der weiteren Maßgabe, dass mindestens ein Rest von Aᵢ, Bᵢ, Bₙ, Aₜ, Bₜ, Tᵢ und Tₜ eine aus der nachstehenden Liste: Amine, Carboxyl, Phosphat, Phosphonatester, Sulfat, Sulfonat, Sulfatester, Sulfonatester und Thiol ausgewählte Substituentengruppe oder eine aus der nachstenden Liste: Amide, Ester, Alkylcarbonyl, Aldehyd und Alkylthiol ausgewählte Prosubstituentengruppe ist.

## Revendications

1. Composition pharmaceutique comprenant, en mélange avec un véhicule pharmaceutiquement acceptable, un composé aromatique oligomère comprenant une seule espèce polymère de formule I
Mᵢ-(Mₙ)-Mₜ I
dans laquelle
n représente une série de nombres entiers commençant à 2 et présentant une limite supérieure de 50;
Mᵢ est 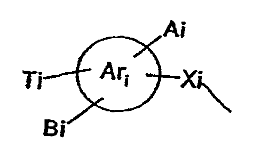
Mₙ est 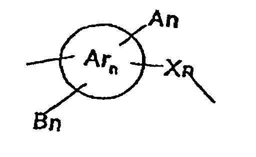
Mₜ est 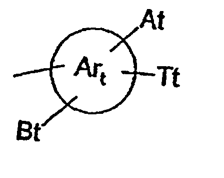 où 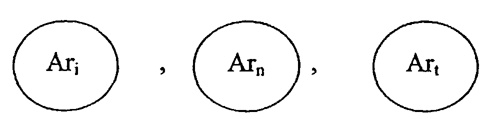 sont indépendamment des noyaux benzène ou naphtalène,
où 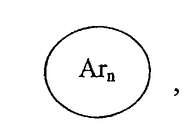 Aₙ, Bₙ et Xₙ
peuvent être identiques ou différents pour chacun des groupes (Mₙ) dans la série n; et
Aᵢ, Bᵢ, Aₙ, Bₙ, Aₜ et Bₜ sont indépendamment des atomes d'hydrogène ou des groupes alkyle en C₁-C₂₀, benzyle, phénéthyle, cyano, alcoxy(en C₁-C₄)alcoxy(en C₁-C₄), ou -(CH₂)ₐ-W-(CH₂)_{b}-Rₛ, où Rₛ est NRR', COOR, CONRR', NR(COR'), PO(OR)₂, COR, SO₂OR, OSO₂OR, halogéno, OR, SO₂R, SOR, SR ou CHO, et où a et b valent indépendamment 0 à 4, (a+b) est inférieur à 5, W est -O-, -SO-, -SO₂-, -NR(COR')-, -NR-, ou une liaison, et R et R' sont indépendamment des atomes d'hydrogène ou des groupes alkyle en C₁-C₂₀, benzyle ou phénéthyle,
Tᵢ et Tₜ sont indépendamment des atomes d'hydrogène ou des groupes alkyle en C₁-C₂₀, allyle, vinyle, halogéno, cyano, alcoxy(en C₁-C₄)-alcoxy(en C₁-C₄), halogénoalkyle en C₁-C₂₀, hydroxy, tert-butyldiméthylsilyloxy(alkyle en C₁-C₂₀), hydroxyalkyle en C₁-C₂₀, alcoxy en C₁-C₄, benzyloxy, phénéthyloxy, phénoxy, 2-naphtyloxy, alcoxy(en C₁-C₄)(alkyle en C₁-C₂₀), benzyloxy(alkyle en C₁-C₂₀), phénéthyloxy(alkyle en C₁-C₂₀), phénoxy(alkyle en C₁-C₂₀), 2-naphtyloxy(alkyle en C₁-C₂₀), mercapto, mercapto(alkyle en C₁-C₂₀), alkylthio en C₁-C₂₀, benzylthio, phénéthylthio, alkylthio(en C₁-C₂₀)(alkyle en C₁-C₂₀), benzylthio(alkyle en C₁-C₂₀), phénéthylthio(alkyle en C₁-C₂₀), amino, alkylamino en C₁-C₂₀, aminoalkyle en C₁-C₂₀, alkylamino(en C₁-C₂₀)(alkyle en C₁-C₂₀), acylamino, acylamino(alkyle en C₁-C₂₀), carboxy, carboxy(alkyle en C₁-C₂₀), alcoxy(en C₁-C₄)-carbonyle, alcoxy(en C₁-C₄)carbonyl(alkyle en C₁-C₂₀), benzyloxycarbonyle, phénéthyloxycarbonyle, benzyloxycarbonyl(alkyle en C₁-C₂₀), phénéthyloxycarbonyl(alkyle en C₁-C₂₀), benzyloxycarbonyloxy, phénéthyloxycarbonyloxy, formyle, formyl(alkyle en C₁-C₂₀), acyle, acyloxy et acyl(alkyle en C₁-C₂₀),
Xᵢ et Xₙ sont indépendamment -(CR₁R₂)ₘ-Y-(CR₃R₄)ₚ- où R₁, R₂, R₃ et R₄ sont indépendamment des atomes d'hydrogène ou des groupes alkyle en C₁-C₂₀, m et p valent indépendamment 0 à 5, à condition que (m+p) vaille de 0 à 5, et Y est une liaison, .-O-, -S-, -CR₆=CR₇- cis ou trans, -CR₆=CR₇-CR₈=CHR₉- où chacune des doubles liaisons peut être indépendamment en cis ou en trans, -N(R₅)-, -N(R₅)CO-, -CONR₅-, carbonyle, carbonyloxy ou oxycarbonyle, où R₅, R₆, R₇, R₈ et R₉ sont indépendamment des atomes d'hydrogène ou des groupes alkyle en C₁-C₂₀;
ou un de ses sels pharmaceutiquement acceptables,
à condition que, lorsque
n est 2;
Tᵢ et Tₜ sont des atomes d'hydrogène;
Xᵢ et Xₙ sont tous deux des groupes carbonyle ou méthylène; 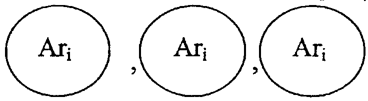 sont tous des groupes phényle;
alors Aᵢ, Aₙ et Aₜ ne soient pas tous des groupes OH ou OCOCH₃ et Bᵢ, Bₙ et Bₜ ne soient ni F ni Cl, ou Aᵢ, Aₙ et At ne soient ni F ni Cl et Bᵢ, Bₙ et Bₜ ne soient tous ni OH ni OCOCH₃;
et
à condition que le composé oligomère ne soit pas:
les composés tétramères di[5-méthoxycarbonyléthyl-3-(5-[2-méthoxycarbonyléthyl]-2-hydroxybenzyl)-2-hydroxyphényl]méthane et di(5-hydroxycarbonyléthyl-3-[5-(2-hydroxycarbonyléthyl)-2-hydroxybenzyl]-2-hydroxyphényl)méthane, à condition, par ailleurs, qu'au moins un des radicaux Aᵢ, Bᵢ, Bₙ, Aₜ, Bₜ, Tᵢ et Tₜ soit un groupe substituant choisi dans la liste suivante: amides, carboxyle, phosphate, ester phosphonate, sulfate, sulfonate, esters sulfate, ester sulfonate et thiol, ou un groupe précurseur de substituant choisi dans la liste suivante: amides, esters, alkylcarbonyle, aldéhyde et alkylthiol.

2. Composition pharmaceutique selon la revendication 1 dans laquelle
Mᵢ est 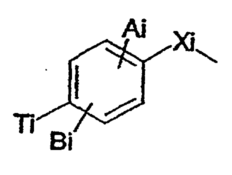
Mₙ est 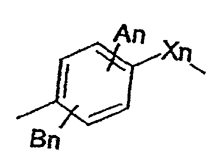
et Mₜ est

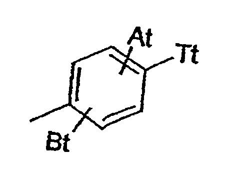

3. Composition pharmaceutique selon la revendication 1 dans laquelle
Mᵢ est 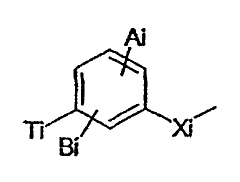
Mₙ est 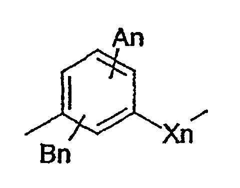
et Mₜ est

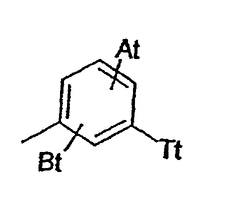

4. Composition pharmaceutique selon la revendication 1 dans laquelle n est une série de nombres entiers commençant à 2 et présentant une limite supérieure de 18 et dans laquelle (Mₙ)ₙ représente de 2 à 18 motifs monomères.

5. Composition pharmaceutique selon la revendication 1 dans laquelle n est une série de nombres entiers commençant à 4 et présentant une limite supérieure de 14 et dans laquelle (Mₙ)ₙ représente de 4 à 14 motifs monomères.

6. Composition pharmaceutique selon la revendication 1 dans laquelle n est une série de nombres entiers commençant à 6 et présentant une limite supérieure de 18 et dans laquelle (Mₙ)ₙ représente de 6 à 18 motifs monomères.

7. Composition pharmaceutique selon la revendication 1 dans laquelle la composition comprend un composé oligomère de formule II 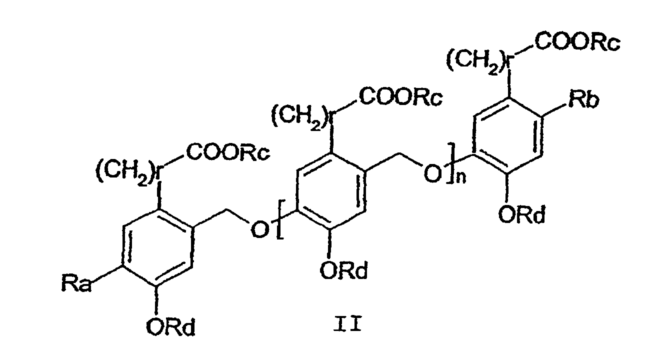 dans laquelle
Rₐ est un atome d'hydrogène ou un groupe hydroxy ou acyloxy,
R_{b} est un atome d'hydrogène ou un groupe alkyle en C₁-C₂₀ ou hydroxyalkyle en C₁-C₂₀;
R_{c} est un atome d'hydrogène ou un groupe alkyle en C₁-C₂₀;
R_{d} est un atome d'hydrogène ou un groupe alkyle en C₁-C₂₀;
r vaut 1 à 4 et
n vaut 2 à 30;
ou un de ses sels pharmaceutiquement acceptables.

8. Composition pharmaceutique selon la revendication 1 dans laquelle la composition comprend un composé oligomère de formule III 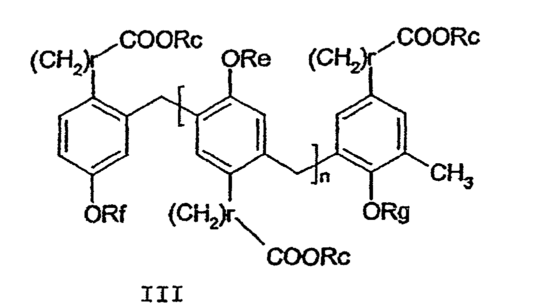 dans laquelle
R_{c}, Rₑ, R_{f} et R_{g} sont indépendamment des atomes d'hydrogène ou des groupes alkyle en C₁-C₂₀;
r vaut 1 à 4 et
n vaut 1 à 30;
ou un de ses sels pharmaceutiquement acceptables.

9. Composition pharmaceutique selon l'une quelconque des revendications 1 à 3 et 7 dans laquelle n est une série de nombres entiers présentant une limite supérieure de 4 à 8.

10. Composition pharmaceutique selon la revendication 1 choisie dans le groupe comprenant:
- l'acide 2-[4-[4-[4-[[5-(2-carboxyéthyl)-2-méthoxy]phénoxyméthyl]-[5-(2-carboxyéthyl)-2-méthoxy]phénoxyméthyl]-[5-(2-carboxyéthyl)-2-méthoxy]phénoxyméthyl]-[5-(2-carboxyéthyl)-2-méthoxy]phénoxyméthyl]-4-méthoxyhydrocinnamique,
- le 1,2-bis-[4-[4-[4-[[2-(2-carboxyéthyl)-5-hydroxy]benzyl]-[2-(2-carboxyéthyl-5-hydroxy)benzyl]-[2-(2-carboxyéthyl)-5-hydroxy]benzyl]-[2-(2-carboxyéthyl)-5-hydroxy]phényl]éthane,
- le bis-[5-(2-carboxyéthyl)-3-[5-(2-carboxyéthyl)-2-hydroxybenzyl]-2-hydroxyphényle],
- le 5-acétoxy-2-[4-[4-[[5-(2-méthoxycarbonyléthyl)-4-hydroxyméthyl-2-méthoxy]phénoxyméthyl]-[5-(2-méthoxycarbonyléthyl)-2-méthoxy]phénoxyméthyl]-[5-(2-méthoxycarbonyléthyl)-2-méthoxy]-phénoxyméthyl]-4-méthoxyhydrocinnamate de méthyle,
- le 1,2-bis-[2-(2-carboxyéthyl)-4-[2-(2-carboxyéthyl)-5-hydroxybenzyl]-5-hydroxyphényle],
- l'acide 5-[4-[4-[4-[4-[4-[4-[[2-(2-carboxyéthyl)-5-hydroxy]benzyl]-[2-(2-carboxyéthyl)-5-hydroxy]benzyl]-(2-(2-carboxyéthyl)-5-hydroxy]-benzyl]-[2-(2-carboxyéthyl)-5-hydroxy]benzyl]-[2-(2-carboxyéthyl)-5-hydroxy]benzyl]-[2-(2-carboxyéthyl)-5-hydroxy]benzyl]-(2-(2-carboxyéthyl)-5-hydroxy]benzyl]-4-hydroxy-2-méthylhydrocinnamique,
- le 2-(2-méthoxycarbonyléthyl)-4-[4-[4-[4-[4-[4-[4-[[2-(2-méthoxycarbonyléthyl)-5-triméthylacétyloxy]benzyl]-[2-(2-méthoxycarbonyléthyl)-5-méthoxy]benzyl]-[2-(2-méthoxycarbonyléthyl)-5-méthoxy]benzyl]-[2-(2-méthoxycarbonyléthyl)-5-méthoxy]benzyl]-(2-(2-méthoxycarbonyléthyl)-5-méthoxy]benzyl]-[2-(2-méthoxycarbonyléthyl)-5-méthoxy]benzyl]-[2-(2-méthoxycarbonyléthyl)-5-méthoxy]benzyl]-5-méthoxybenzyloxy-tert-butyldiméthylsilane,
- le 1,2-bis-[4-[4-[4-[[2-(2-méthoxycarbonyléthyl)-5-triméthylacétyloxy]benzyl]-[2-(2-méthoxycarbonyléthyl)-5-méthoxy]benzyl]-[2-(2-méthoxycarbonyléthyl)-5-méthoxy]benzyl]-[2-(2-méthoxycarbonyléthyl)-5-méthoxy]phényl]éthane,
- l'acide 5-[4-[4-[4-[4-[4-[4-[[2-(2-carboxyéthyl)-5-hydroxy]benzyl]-[2-(2-carboxyéthyl)-5-méthoxy]benzyl]-[2-(2-carboxyéthyl)-5-méthoxy]-benzyl]-[2-(2-carboxyéthyl)-5-méthoxy]benzyl]-[2-(2-carboxyéthyl)-5-méthoxy]benzyl]-[2-(2-carboxyéthyl)-5-méthoxy]benzyl]-[2-(2-carboxyéthyl)-5-méthoxy]benzyl]-4-méthoxy-2-méthylhydrocinnamique,
- l'acide 5-[4-[4-[4-[4-[4-[4-[4-[4-[4-[4-[4-[4-[4-[4-[[2-(2-carboxyéthyl)-5-hydroxy]benzyl]-[2-(2-carboxyéthyl)-5-hydroxy]benzyl)-[2-(2-carboxyéthyl)-5-hydroxy]benzyl]-[2-(2-carboxyéthyl)-5-hydroxy]benzyl]-[2-(2-carboxyéthyl)-5-hydroxy]benzyl]-[2-(2-carboxyéthyl)-5-hydroxy]-benzyl]-[2-(2-carboxyéthyl)-5-hydroxy]benzyl]-[2-(2-carboxyéthyl)-5-hydroxy]benzyl]-[2-(2-carboxyéthyl)-5-hydroxy]benzyl]-[2-(2-carboxyéthyl)-5-hydroxy]benzyl]-[2-(2-carboxyéthyl)-5-hydroxy]benzyl]-[2-(2-carboxyéthyl)-5-hydroxy]benzyl]-[2-(2-carboxyéthyl)-5-hydroxy]benzyl]-[2-(2-carboxyéthyl)-5-hydroxy]benzyl]-[2-(2-carboxyéthyl)-5-hydroxy]-benzyl]-4-hydroxy-2-méthylhydrocinnamique,
- le 1,2-bis-[4-[4-[4-[4-[4-[4-[4-[[2-(2-carboxyéthyl)-5-hydroxy]-benzyl]-[2-(2-carboxyéthyl)-5-méthoxy]benzyl]-[2-(2-carboxyéthyl)-5-méthoxy]benzyl]-[2-(2-carboxyéthyl)-5méthoxy]benzyl]- [2-(2-carboxyéthyl)-5-méthoxy]benzyl]-[2-(2-carboxyéthyl)-5-méthoxy]benzyl]-[2-(2-carboxyéthyl)-5-méthoxy]benzyl)-[2-(2-carboxyéthyl)-5-méthoxy]phényl]-éthane,
- l'acide 5-[4-[4-[4-[4-[4-[4-[4-[4-[4-[4-[4-[4-[4-[4-[2-(2-carboxyéthyl)-5-hydroxy]benzyl]-[2-(2-carboxyéthyl)-5-méthoxy]benzyl]- [2-(2-carboxyéthyl)-5-méthoxy]benzyl]-[2-(2-carboxyéthyl)-5-méthoxy]benzyl]-[2-(2-carboxyéthyl)-5-méthoxy]benzyl]-[2-(2-carboxyéthyl)-5-méthoxy]-benzyl]-[2-(2-carboxyéthyl)-5-méthoxy]benzyl]-[2-(2-carboxyéthyl)-5-méthoxy]benzyl]-[2-(2-carboxyéthyl)-5-méthoxy]benzyl]-[2-(2-carboxyéthyl)-5-méthoxy]benzyl]-[2-(2-carboxyéthyl)-5-méthoxy]benzyl]-[2-(2-carboxyéthyl)-5-méthoxy]benzyl]-[2-(2-carboxyéthyl)-5-méthoxy]benzyl]-[2-(2-carboxyéthyl)-5-méthoxy]benzyl]-[2-(2-carboxyéthyl)-5-méthoxy]-benzyl]-4-hydroxy-2-méthylhydrocinnamique,
- le 5-acétoxy-2-[4-[4-[4-[4-[4-[4-[[5-(2-méthoxycarbonyléthyl)-4-hydroxyméthyl-2-méthoxy]phénoxyméthyl]-[5-(2-méthoxycarbonyléthyl)-2-méthoxy]phénoxyméthyl]-[5-(2-méthoxycarbonyléthyl)-2-méthoxy]-phénoxyméthyl]-[5-(2-méthoxycarbonyléthyl)-2-méthoxy]phénoxyméthyl]-[5-(2-méthoxycarbonyléthyl)-2-méthoxy]phénoxyméthyl]-[5-(2-méthoxycarbonyléthyl)-2-méthoxy)phénoxyméthyl]-[5-(2-méthoxycarbonyléthyl)-2-méthoxy]phénoxyméthyl]-4-méthoxyhydrocinnamate de méthyle,
- le 5-acétoxy-2-[4-[4-[4-[4-[4-[4-[4-[4-[4-[4-[4-[4-[4-[4-[[5-(2-méthoxycarbonyléthyl)-4-hydroxyméthyl-2-méthoxy]phénoxyméthyl]-[5-(2-méthoxycarbonyléthyl)-2-méthoxy]phénoxyméthyl]-[5-(2-méthoxycarbonyléthyl)-2-méthoxy]phénoxyméthyl]-[5-(2-méthoxycarbonyléthyl)-2-méthoxy]phénoxyméthyl]-[5-(2-méthoxycarbonyléthyl)-2-méthoxy]-phénoxyméthyl]-[5-(2-méthoxycarbonyléthyl)-2-méthoxy]phénoxyméthyl]-[5-(2-méthoxycarbonyléthyl)-2-méthoxy]phénoxyméthyl]-[5-(2-méthoxycarbonyléthyl)-2-méthoxy]phénoxyméthyl]-[5-(2-méthoxycarbonyléthyl)-2-méthoxy]phénoxyméthyl]-[5-(2-méthoxycarbonyléthyl)-2-méthoxy]-phénoxyméthyl]-[5-(2-méthoxycarbonyléthyl)-2-méthoxy]phénoxyméthyl]-[5-(2-méthoxycarbonyléthyl)-2-méthoxy]phénoxyméthyl]-[5-(2-méthoxycarbonyléthyl)-2-méthoxy]phénoxyméthyl]-[5-(2-méthoxycarbonyléthyl)-2-méthoxy]phénoxyméthyl]-[5-(2-méthoxycarbonyléthyl )-2-méthoxy]-phénoxyméthyl]-4-méthoxyhydrocinnamate de méthyle,
- le 1,2-bis-[4-[4-[4-[[2-(2-carboxyéthyl)-5-hydroxy]benzyl]-[2-(2-carboxyéthyl)-5-méthoxy]benzyl]-[2-(2-carboxyéthyl)-5-méthoxy]benzyl]-[2-(2-carboxyéthyl)-5-méthoxy]phényl]éthane,
- le 5-acétoxy-2-[4-[4-[4-[4-[4-[4-[4-[4-[4-[4-[4-[4-[4-[4-[[5-(2-méthoxycarbonyléthyl)-4-hydroxyméthyl-2-méthoxy]phénoxyméthyl]-[5-(2-méthoxycarbonyléthyl)-2-méthoxy]phénoxyméthyl]-[5-(2-méthoxycarbonyléthyl)-2-méthoxy]phénoxyméthyl]-[5-(2-méthoxycarbonyléthyl)-2-méthoxy]phénoxyméthyl]-[5-(2-méthoxycarbonyléthyl)-2-méthoxy]-phénoxyméthyl]-[5-(2-méthoxycarbonyléthyl)-2-méthoxy]phénoxyméthyl]-[5-(2-méthoxycarbonyléthyl)-2-méthoxy]phénoxyméthyl]-[5-(2-méthoxycarbonyléthyl)-2-méthoxy]phénoxyméthyl]-[5-(2-méthoxycarbonyléthyl)-2-méthoxy]phénoxyméthyl]-[5-(2-méthoxycarbonyléthyl)-2-méthoxy]-phénoxyméthyl]-[5-(2-méthoxycarbonyléthyl)-2-méthoxy]phénoxyméthyl]-[5-(2-méthoxycarbonyléthyl)-2-méthoxy]phénoxyméthyl]-[5-(2-méthoxycarbonyléthyl)-2-méthoxy]phénoxyméthyl]-[5-(2-méthoxycarbonyléthyl)-2-méthoxy]phénoxyméthyl]-[5-(2-méthoxycarbonyléthyl)-2-méthoxy]-phénoxyméthyl]4-méthoxyhydrocinnamate de méthyle,
- le 1,2-bis-[4-[4-[4-[[2-(2-carboxyéthyl)-5-hydroxy]benzyl]-[2-(2-carboxyéthyl)-5-méthoxy]benzyl]-[2-(2-carboxyéthyl)-5-méthoxy]benzyl]-[2-(2-carboxyéthyl)-5-méthoxy]phényl]éthane.

11. Utilisation d'une composition selon l'une quelconque des revendications 1 à 10 pour la fabrication d'un médicament destiné à traiter une dégradation des tissus conjonctifs ayant pour médiateur l'élastase.

12. Utilisation d'une composition selon l'une quelconque des revendications 1 à 10 pour la fabrication d'un médicament destiné à traiter la thrombose, à inhiber la prolifération cellulaire des muscles lisses, ou à être utilisé comme anticoagulant.

13. Composé oligomère comprenant une seule espèce polymère de formule I
Mᵢ-(Mₙ)ₙ-Mₜ I
dans laquelle
n représente une série de nombres entiers commençant à 2 et présentant une limite supérieure de 50;
Mᵢ est 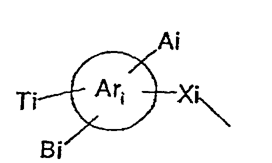
Mₙ est 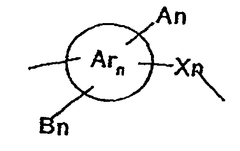
Mₜ est 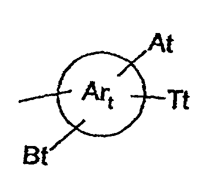 où 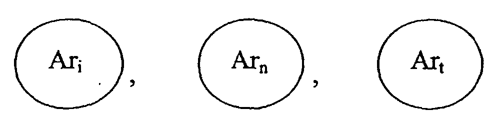 sont indépendamment des noyaux benzène ou naphtalène,
où 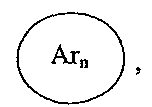 Aₙ, Bₙ et Xₙ
peuvent être identiques ou différents pour chacun des groupes (Mₙ) dans la série n; et
Aᵢ, Bᵢ, Aₙ, Bₙ, Aₜ et Bₜ sont indépendamment des atomes d'hydrogène ou des groupes alkyle en C₁-C₂₀, benzyle, phénéthyle, cyano, alcoxy(en C₁-C₄)alcoxy(en C₁-C₄), ou -(CH₂)ₐ-W-(CH₂)_{b}-Rₛ, où Rₛ est NRR', COOR, CONRR', NR(COR'), PO(OR)₂, COR, SO₂OR, OSO₂OR, halogéno, OR, SO₂R, SOR, SR ou CHO, et où a et b valent indépendamment 0 à 4, (a+b) est inférieur à 5, W est -O-, -SO-, -SO₂-, -NR(COR')-, -NR-, ou une liaison, et R et R' sont indépendamment des atomes d'hydrogène ou des groupes alkyle en C₁-C₂₀, benzyle ou phénéthyle,
Tᵢ et Tₜ sont indépendamment des atomes d'hydrogène ou des groupes alkyle en C₁-C₂₀, allyle, vinyle, halogéno, cyano, alcoxy(en C₁-C₄)-alcoxy(en C₁-C₄), halogénoalkyle en C₁-C₂₀, hydroxy, tert-butyldiméthylsilyloxy(alkyle en C₁-C₂₀), hydroxyalkyle en C₁-C₂₀, alcoxy en C₁-C₄, benzyloxy, phénéthyloxy, phénoxy, 2-naphtyloxy, alcoxy(en C₁-C₄)(alkyle en C₁-C₂₀), benzyloxy(alkyle en C₁-C₂₀), phénéthyloxy(alkyle en C₁-C₂₀), phénoxy(alkyle en C₁-C₂₀), 2-naphtyloxy(alkyle en C₁-C₂₀), mercapto, mercapto(alkyle en C₁-C₂₀), alkylthio en C₁-C₂₀, benzylthio, phénéthylthio, alkylthio(en C₁-C₂₀)(alkyle en C₁-C₂₀), benzylthio(alkyle en C₁-C₂₀), phénéthylthio(alkyle en C₁-C₂₀), amino, alkylamino en C₁-C₂₀, aminoalkyle en C₁-C₂₀, alkylamino(en C₁-C₂₀)(alkyle en C₁-C₂₀), acylamino, acylamino(alkyle en C₁-C₂₀), carboxy, carboxy(alkyle en C₁-C₂₀), alcoxy(en C₁-C₄)-carbonyle, alcoxy(en C₁-C₄)carbonyl(alkyle en C₁-C₂₀), benzyloxycarbonyle, phénéthyloxycarbonyle, benzyloxycarbonyl(alkyle en C₁-C₂₀), phénéthyloxycarbonyl(alkyle en C₁-C₂₀), benzyloxycarbonyloxy, phénéthyloxycarbonyloxy, formyle, formyl(alkyle en C₁-C₂₀), acyle, acyloxy et acyl(alkyle en C₁-C₂₀),
Xᵢ et Xₙ sont indépendamment -(CR₁R₂)ₘ-Y-(CR₃R₄)ₚ- où R₁, R₂, R₃ et R₄ sont indépendamment des atomes d'hydrogène ou des groupes alkyle en C₁-C₂₀, m et p valent indépendamment 0 à 5, à condition que (m+p) vaille de 0 à 5, et Y est une liaison, -O-, -S-, -CR₆=CR₇- cis ou trans, -CR₆=CR₇-CR₈=CHR₉- où chacune des doubles liaisons peut être indépendamment en cis ou en trans, -N(R₅)-, -N(R₅)CO-, -CONR₅-, carbonyle, carbonyloxy ou oxycarbonyle, où R₅, R₆, R₇, R₈ et R₉ sont indépendamment des atomes d'hydrogène ou des groupes alkyle en C₁-C₂₀; ou un de ses sels pharmaceutiquement acceptables,
à condition que, lorsque
n est 2;
Tᵢ et Tₜ sont des atomes d'hydrogène;
Xᵢ et Xₙ sont tous deux des groupes carbonyle ou méthylène; 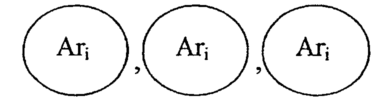 sont tous des groupes phényle; alors Aᵢ, Aₙ et Aₜ ne soient pas tous des groupes OH ou OCOCH₃ et Bᵢ, Bₙ et ne soient ni F ni Cl, ou Aᵢ, Aₙ et Aₜ ne soient ni F ni Cl et Bᵢ, Bₙ et Bₜ ne soient tous ni OH ni OCOCH₃;
et
à condition que le composé oligomère ne soit pas:
les composés tétramères di[5-méthoxycarbonyléthyl-3-(5-[2-méthoxycarbonyléthyl]-2-hydroxybenzyl)-2-hydroxyphényl]méthane et di(5-hydroxycarbonyléthyl-3 -[5-(2-hydroxycarbonyléthyl)-2-hydroxybenzyl]-2-hydroxyphényl)méthane, à condition, par ailleurs, qu'au moins un des radicaux Aᵢ, Bᵢ, Bₙ, Aₜ, Bₜ, Tᵢ et Tₜ soit un groupe substituant choisi dans la liste suivante: amides, carboxyle, phosphate, ester phosphonate, sulfate, sulfonate, esters sulfate, ester sulfonate et thiol, ou un groupe précurseur de substituant choisi dans la liste suivante: amides, esters, alkylcarbonyle, aldéhyde et alkylthiol.
